# EUROPÄISCHE PATENTSCHRIFT

(11) **EP 2 380 945 B1**
(45) Veröffentlichungstag und Bekanntmachung des Hinweises auf die Patenterteilung: **14.05.2014**
(21) Anmeldenummer: 11002667.1
(22) Anmeldetag: 31.03.2011
(51) Int. Cl.: C09K 19/20, C09K 19/32, C09K 19/46, C09K 19/34, C09K 19/02, C09K 19/42, C09K 19/04, C09K 19/54

(54) **Polymerisierbare Verbindungen und ihre Verwendung in Flüssigkristallmedien und Flüssigkristallanzeigen**
Polymerisable compounds and use of same in liquid crystal media and liquid crystal displays
Composés polymérisables et leur utilisation dans des milieux à base de cristaux liquides et écrans à base de cristaux liquides

(30) Priorität: 26.04.2010 DE 102010018188
(43) Veröffentlichungstag der Anmeldung: 26.10.2011
(73) Patentinhaber: Merck Patent GmbH, 64293 Darmstadt (DE)
(72) Erfinder: Jansen, Axel, Dr., 64293 Darmstadt (DE); Taugerbeck, Andreas, Dr., 64285 Darmstadt (DE); Haensel, Helmut, Dr., 64367 Muehltal (DE); Goetz, Achim, 64665 Alsbach-Haehnlein (DE)

(56) Entgegenhaltungen:
- EP-A1- 1 378 557
- EP-A1- 1 900 792
- DE-A1-102008 024 866
- DE-A1-102008 036 248
- US-A1- 2009 109 392

## Beschreibung

Die vorliegende Erfindung betrifft polymerisierbare Verbindungen, Verfahren und Zwischenprodukte zu ihrer Herstellung, und ihre Verwendung für optische, elektrooptische und elektronische Zwecke, insbesondere in Flüssigkristall (FK)-Medien und FK-Anzeigen mit polymerstabilisierter blauer Phase, sowie in FK-Medien für FK-Anzeigen des PS- oder PSA-Typs ("polymer sustained" bzw. "polymer sustained alignment"), sowie diese Verbindungen enthaltende FK-Medien und FK-Anzeigen.

Im Stand der Technik sind Medien für Anzeigenelemente bekannt, die im Betrieb in der flüssigkristallinen blauen Phase (kurz: blaue Phase) arbeiten. Solche Anzeigen sind beispielsweise in WO 2004/046805 A1 und WO 2008/061606 A1 beschrieben.

Die blaue Phase wird in der Regel am Übergang vom nematischen zum optisch isotropen Zustand beobachtet. Das Medium in der flüssigkristallinen blauen Phase kann blau sein, wie der Name andeutet, aber auch farblos. Ziel bisheriger Anstrengungen war es, den Temperaturbereich der blauen Phase von weniger als einem Grad auf einen praktisch nutzbaren Bereich auszudehnen (vgl. H. Kikuchi et al., Nature Materials (2002), 1(1), 64-68; Kikuchi, H. et al., Polymeric Materials Science and Engineering, (2003), 89, 90-91).

Zu diesem Zweck wird im Stand der Technik vorgeschlagen, dem FK-Medium eine polymerisierbare Verbindung beizufügen, welche dann im FK-Medium in situ polymerisiert wird. Das dabei gebildete Polymer oder Polymernetzwerk soll die blaue Phase stabilisieren.

Die bisher im Stand der Technik beschriebenen polymerstabilisierten blauen Phasen verwenden beispielsweise als Monomere ein monoreaktives nicht-mesogenes Monomer zusammen mit einem direaktiven mesogenen Monomer.

Die WO 2005/080529 A1 beschreibt beispielsweise polymerstabilisierte blaue Phasen mit mono- und multireaktiven Monomeren.

Der vorliegenden Erfindung lag als eine Aufgabe zugrunde, geeignete Monomere und entsprechende Polymere für die Stabilisierung von blauen Phasen zu finden. Das Polymer soll insbesondere folgende Effekte auf die Eigenschaften der stabilisierten FK-Phase haben:
- breiter Temperaturbereich der blauen Phase,
- schnelle Schaltzeit,
- geringe Klärpunktsdifferenz beim Polymerisieren,
- geringe Betriebsspannung (Vₒₚ),
- geringe Variation der Betriebsspannung mit der Temperatur,
- geringe Hysterese der Transmission einer Zelle bei Änderung der Betriebsspannung zum Erzielen definierter Graustufen.

Es werden außerdem Monomere benötigt, die eine gute 'Voltage holding ratio' (VHR) aufweisen, hohe Klärpunkte besitzen, und stabil gegenüber Belastungen durch Licht und Temperatur sind. Weiterhin ist eine gute Löslichkeit in LC-Materialien bzw. eine gute Mischbarkeit mit dem FK-Medium notwendig, um eine gute Verteilung im FK-Medium zu erreichen.

Der vorliegenden Erfindung liegt somit die Aufgabe zugrunde, verbesserte polymerisierbare Verbindungen, sowie solche Verbindungen enthaltende FK-Medien, zur Verfügung zu stellen, insbesondere für die Verwendung in FK-Anzeigen mit einer polymerstabilisierten blauen Phase. Die erfindungsgemäßen polymerisierbaren Verbindungen sollen die blaue Phase stabilisieren. Die erfindungsgemäßen FK-Medien sollen eine oder mehrere verbesserte Eigenschaften, insbesondere ausgewählt aus den oben genannten Eigenschaften, aufweisen. Insbesondere sollen die FK-Medien eine breite blaue Phase aufweisen, ein schnelles Schalten ermöglichen, eine gute Voltage holding ratio (VHR) aufweisen, geringe Spannungen (Vₒₚ) für den Schaltprozess benötigen und eine geringe Hysterese (ΔV) zeigen und einen geringen Memory Effekt (ME) aufweisen. Die FK-Medien sollen stabil gegenüber Belastungen durch Licht- und Temperatur sein.

Weiterhin sind im Stand der Technik sogenannte PS- bzw. PSA-Anzeigen bekannt ("Polymer Sustained" bzw. "Polymer Sustained Alignment"), für die auch gelegentlich der Begriff "Polymer Stabilized" verwendet wird. In diesen Anzeigen wird dem FK-Medium eine geringe Menge (zum Beispiel 0.3 Gew.%, typischerweise <1 Gew.%) einer oder mehrerer polymerisierbarer Verbindung(en) zugesetzt, welche nach Einfüllen in die FK-Zelle mit oder ohne angelegte elektrische Spannung zwischen den Elektroden in situ polymerisiert bzw. vernetzt wird, üblicherweise durch UV-Photopolymerisation. Als besonders geeignet hat sich der Zusatz von polymerisierbaren mesogenen oder flüssigkristallinen Verbindungen, auch als reaktive Mesogene oder "RM"s bezeichnet, zur FK-Mischung erwiesen.

In diesem Zusammenhang offenbart US 2009-0109392 A1 eine polymerstabilisierte flüssigkristalline Mischung nach einem Ausrichtungsprozeß enthaltend eine Komponente von Flüssigkristallverbindungen ohne Doppelbindung, eine weiter Komponente bestehend aus Flüssigkristalliverbindungen mit Doppelbindung, und wenigstens ein oder mehrere reaktive Monomeren mit Methacrylatgruppe.

DE 10 2008 036248 offenbart eine Flüssigkristall (FK)-Anzeige des PS-(polymer stabilized) oder PSA- (polymer sustained alignment) Typs, sowie polymerisierbare Verbindungen und FK-Medien zur Verwendung in PS-(polymer stabilized) und PSA-Anzeigen.

Nachfolgend wird der Begriff "PSA", falls nicht anders angegeben, stellvertretend für PS-Anzeigen und PSA-Anzeigen verwendet.

Mittlerweile wird das PS(A)-Prinzip in diversen klassischen FK-Anzeigen angewendet. So sind beispielsweise PSA-VA-, PSA-OCB-, PSA-IPS-, PSA-FFS- und PSA-TN-Anzeigen bekannt. Die Polymerisation der polymerisierbaren Verbindung(en) erfolgt bei PSA-VA- und PSA-OCB-Anzeigen vorzugsweise bei angelegter elektrischer Spannung, bei PSA-IPS-Anzeigen mit oder ohne angelegte elektrische Spannung. Wie man in Testzellen nachweisen kann, führt das PS(A)-Verfahren zu einem "pretilt" (Anstellwinkel) in der Zelle. Bei PSA-OCB-Anzeigen beispielsweise kann man erreichen, dass die Bend-Struktur stabilisiert wird, so dass man ohne OffsetSpannung auskommt oder diese reduzieren kann. Im Falle von PSA-VA-Anzeigen wirkt sich der "pretilt" positiv auf die Schaltzeiten aus. Für PSA-VA-Anzeigen kann ein Standard-MVA- bzw. -PVA Pixel- und Elektroden-Layout verwendet werden. Darüber hinaus kann man aber beispielsweise auch mit nur einer strukturierten Elektrodenseite und ohne Protrusions auskommen, was die Herstellung wesentlich vereinfacht und gleichzeitig zu einem sehr guten Kontrast bei sehr guter Lichtdurchlässigkeit führt.

PSA-VA-Anzeigen sind beispielsweise in JP 10-036847 A, EP 1 170 626 A2, US 6,861,107, US 7,169,449, US 2004/0191428 A1, US 2006/0066793 A1 und US 2006/0103804 A1 beschrieben. PSA-OCB-Anzeigen sind beispielsweise in T.-J- Chen et al., Jpn. J. Appl. Phys. 45, 2006, 2702-2704 und S. H. Kim, L.-C- Chien, Jpn. J. Appl. Phys. 43, 2004, 7643-7647 beschrieben. PSA-IPS-Anzeigen sind zum Beispiel in US 6,177,972 und Appl. Phys. Lett. 1999, 75(21), 3264 beschrieben. PSA-TN-Anzeigen sind zum Beispiel in Optics Express 2004, 12(7), 1221 beschrieben.

PSA-Anzeigen können ebenso wie die oben beschriebenen konventionellen FK-Anzeigen als Aktivmatrix- oder Passivmatrix-Anzeigen betrieben werden. Bei Aktivmatrix-Anzeigen erfolgt die Ansteuerung einzelner Bildpunkte üblicherweise durch integrierte, nicht-lineare aktive Elemente wie beispielsweise Transistoren (z.B. Dünnfilmtransistoren, engl. "thin film transistor" bzw. "TFT"), bei Passivmatrix-Anzeigen üblicherweise nach dem Multiplex-Verfahren, wobei beide Verfahren aus dem Stand der Technik bekannt sind.

Es sind jedoch nicht alle Kombinationen bestehend aus FK-Mischung und polymerisierbarer Komponente für PSA-Anzeigen geeignet, weil sich zum Beispiel kein oder kein ausreichender Tilt einstellt, oder weil zum Beispiel die sogenannte "Voltage Holding Ratio" (VHR oder HR) für TFT-Displayanwendungen unzureichend ist. Zudem hat sich gezeigt, dass bei Verwendung in PSA-Anzeigen die aus dem Stand der Technik bekannten FK-Mischungen und RMs noch einige Nachteile aufweisen. So eignet sich nicht jedes bekannte, in FK-Mischungen lösliche RM zur Verwendung in PSA-Anzeigen.

Darüber hinaus sollte die gewählte Kombination FK-Hostmischung/RM eine möglichst geringe Rotationsviskosität sowie möglichst gute elektrische Eigenschaften aufweisen, insbesondere sollte sie eine möglichst hohe VHR besitzen. Bei PSA-Anzeigen ist vor allem eine hohe VHR nach Bestrahlung mit UV-Licht erforderlich, da die UV-Belichtung ein notwendiger Teil des Herstellungsprozesses der Anzeige ist, aber auch als normale Belastung im Betrieb der fertigen Anzeige auftritt.

Insbesondere wäre es wünschenswert, neue Materialien für PSA-Anzeigen zur Verfügung zu haben, die einen besonders kleinen "pretilt"-Winkel erzeugen. Hierbei sind Materialien bevorzugt, die während der Polymerisation bei gleicher Belichtungszeit einen niedrigeren "pretilt"-Winkel erzeugen als die bisher bekannten Materialien, und/oder durch deren Verwendung der mit den bekannten Materialien erzielbare (höhere) "pretilt"-Winkel bereits nach kürzerer Belichtungszeit erreicht werden kann. Dadurch könnten die Produktionszeit (engl. "tact time") der Anzeige verkürzt und die Kosten des Produktionsprozesses verringert werden.

Ein weiteres Problem bei der Herstellung von PSA-Anzeigen ist das Vorhandensein bzw. die Entfernung von Restmengen unpolymerisierter RMs insbesondere nach dem Polymerisationsschritt zur Erzeugung des "pretilt"-Winkels in der Anzeige. Beispielsweise können solche nicht abreagierten RMs die Eigenschaften der Anzeige nachteilig beeinflussen, indem sie z.B. nach Fertigstellung der Anzeige während des Betriebes unkontrolliert polymerisieren.

So zeigen die aus dem Stand der Technik bekannten PSA-Anzeigen oft den unerwünschten Effekt des sogenannten "image sticking" oder "image burn", d.h. dass das in der FK-Anzeige durch vorübergehende Ansteuerung einzelner Bildpunkte (pixel) erzeugte Bild auch nach Abschalten des elektrischen Feldes in diesen Bildpunkten, oder nach Ansteuerung anderer Bildpunkte, noch sichtbar bleibt.

Es ist deshalb wünschenswert, dass die Polymerisation der RMs bei der Herstellung der PSA-Anzeige möglichst vollständig abläuft und die Anwesenheit von unpolymerisierten RMs in der Anzeige möglichst ausgeschlossen oder auf ein Minimum reduziert wird. Hierzu werden Materialien benötigt, die eine möglichst effektive und vollständige Polymerisation ermöglichen.

Es besteht somit immer noch ein großer Bedarf an PSA-Anzeigen sowie FK-Medien und polymerisierbaren Verbindungen zur Verwendung in solchen Anzeigen, welche die oben beschriebenen Nachteile nicht oder nur in geringem Maße zeigen und verbesserte Eigenschaften besitzen. Zudem besteht ein großer Bedarf nach PSA-Anzeigen, sowie Materialien zur Verwendung in PSA-Anzeigen, die vorteilhafte Eigenschaften aufweisen, insbesondere einen hohen spezifischen Widerstand bei gleichzeitig großem Arbeitstemperaturbereich, kurze Schaltzeiten auch bei tiefen Temperaturen und niedriger Schwellenspannung, einen niedrigen "pretilt"-Wnkel, eine Vielzahl von Graustufen, einen hohen Kontrast und einen weiten Blickwinkel ermöglichen, sowie hohe Werte der "voltage holding ratio" (VHR) nach UV-Belastung und der Tieftemperaturstabilität, auch als "LTS" (low temperature stability) bezeichnet, d.h. der Stabilität der FK-Mischung gegen spontane Auskristallisation einzelner Komponenten.

Der Erfindung liegt somit die weitere Aufgabe zugrunde, neue geeignete Materialien, insbesondere RMs und diese enthaltende FK-Medien, für die Verwendung in PSA-Anzeigen bereitzustellen, die die oben angegebenen Nachteile nicht oder in geringerem Maße aufweisen, möglichst schnell und vollständig polymerisieren, eine möglichst schnelle Einstellung eines niedrigen "pretilt"-Winkels ermöglichen, das Auftreten von "image sticking" in der Anzeige verringern oder vermeiden, und vorzugsweise gleichzeitig sehr hohe spezifische Widerstände, niedrige Schwellenspannungen und niedrige Schaltzeiten ermöglichen. Zudem sollten die FK-Medien günstige FK-Phaseneigenschaften sowie hohe VHR- und LTS-Werte aufweisen.

Die oben beschriebenen Aufgaben wurden erfindungsgemäß gelöst durch die Bereitstellung von Materialien, Verfahren und FK-Anzeigen wie in der vorliegenden Anmeldung beschrieben. Insbesondere wurde überraschend gefunden, dass die oben beschriebenen Aufgaben teilweise oder vollständig gelöst werden können, indem man zur Herstellung solcher FK-Anzeigen FK-Medien verwendet, welche eine oder mehrere erfindungsgemäße polymerisierbare Verbindungen wie nachstehend beschrieben enthalten, bzw. indem man FK-Anzeigen mit blauer Phase bzw. PSA-Anzeigen bereitstellt, die eine oder mehrere erfindungsgemäße Verbindungen in polymerisierter Form enthalten.

Die erfindungsgemäßen polymerisierbaren Verbindungen enthalten eine zentrale mesogene Gruppe und mindestens zwei polymerisierbare Gruppen, welche mit der mesogenen Gruppe direkt oder über Abstandsgruppen (engl. "spacer") verknüpft sind, wobei die zentrale mesogene Gruppe aus drei cyclischen Resten besteht, welche durch zwei Difluormethylenoxybrücken (CF₂O bzw. OCF₂) miteinander verknüpft sind.

Die Verwendung der erfindungsgemäßen polymerisierbaren Verbindungen in erfindungsgemäßen FK-Medien für FK-Anzeigen mit polymerstabilisierter blauer Phase führt zu einer deutlichen Stabilisierung der blauen Phase. Zudem hat sich überraschend gezeigt, dass bei der Verwendung der erfindungsgemäßen polymerisierbaren Verbindungen in FK-Medien mit polymerstabilisierter blauer Phase eine deutliche Verringerung der Hysterese (ΔV₅₀) und eine Erhöhung des Kontrastes erzielt wird, im Vergleich zu polymerisierbaren Verbindungen und FK-Medien wie im Stand der Technik beschrieben.

In PSA-Anzeigen führt die Verwendung der erfindungsgemäßen polymerisierbaren Verbindungen in erfindungsgemäßen FK-Medien zu einem besonders schnellen Erreichen des gewünschten "pretilts" und zu deutlich verkürzten Zeiten bei der Herstellung der Anzeige.

Im Stand der Technik, wie beispielsweise in der US 7,440,160 (WO 2004/046805 A1) und den darin zitierten Dokumenten, werden FK-Medien für FK-Anzeigenelemente beschrieben, die im Betrieb in der flüssigkristallinen blauen Phase (kurz: blaue Phase) arbeiten. Die WO 2005/080529 A1 beschreibt polymerstabilisierte blaue Phasen mit mono- und multireaktiven Monomeren. Die US 2009/0267025 A1 (WO 2006/063662 A1) US 2009/051855 A1, US 2009/0059132 A1, US 2009/0059157 A1 und WO 2008/061606 A1 beschreiben die Polymerstabilisierung blauer Phasen mit flüssigkristallinen reaktiven Komponenten (auch als reaktive Mesogene, kurz "RM"s, bezeichnet). In den vorgenannten Veröffentlichungen werden jedoch vorzugsweise RMs mit entweder direkt oder über Estergruppen verknüpften Phenylresten verwendet, wie beispielsweise die folgenden beiden RMs: 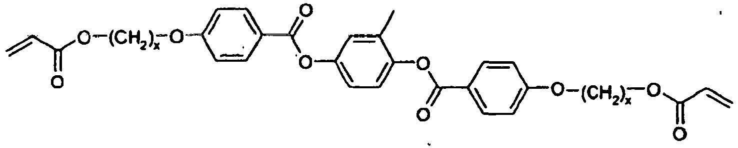 worin x entweder beide 3 oder 6 bedeuten.

Die Verwendung von reaktiven Komponenten, die vorzugsweise aus erfindungsgemäßen polymerisierbaren Verbindungen bestehen, für polymerstabilisierte blaue Phasen oder in PSA-Anzeigen werden im Stand der Technik jedoch weder beschrieben noch nahegelegt.

In der US 7,070,838 werden polymerisierbare Verbindungen enthaltend einen 2-Di- oder Trifluormethyl-1,4-phenylring, sowie deren Verwendung in polymerisierbaren Mischungen, FK-Polymeren und FK-Anzeigen mit cholesterischer Phase sowie in optischen Filmen beschrieben. Darin werden auch konkrete Verbindungen einer Formel 1a-2-19 mit folgender Struktur offenbart:

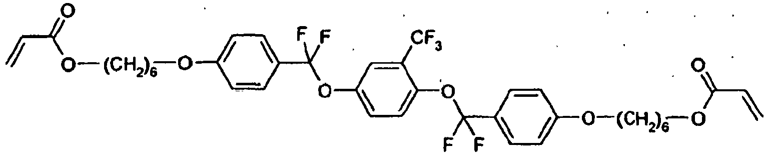

Jedoch werden keine Eigenschaften dieser Verbindung bei Verwendung in einer FK-Anzeige offenbart. Zudem wird die Verwendung solcher Verbindungen zur Stabilisierung von blauen Phasen oder in PSA-Anzeigen durch die US 7,070,838 weder beschrieben noch nahegelegt.

In der JP 2005-015473 A werden polymerisierbare Verbindungen mit ungesättigten Spacergruppen (Alkinylen oder Alkenylen) offenbart. Darin werden auch konkrete Verbindungen der Formeln 1-13-77 bis 1-13-84, 1-13-134, 1-13-135, 1-56-9, 1-56-10, 1-56-23, 1-56-24 offenbart, die über CF₂O-Brücken verknüpfte Phenylringe enthalten, sowie deren Verwendung zur Herstellung von optisch anisotropen Filmen und in ferroelektrischen FK-Medien. Darin werden auch konkrete Verbindungen beispielsweise mit folgenden Strukturen offenbart.

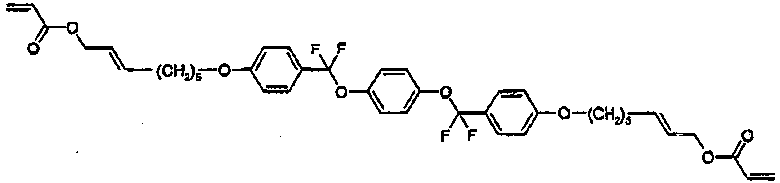

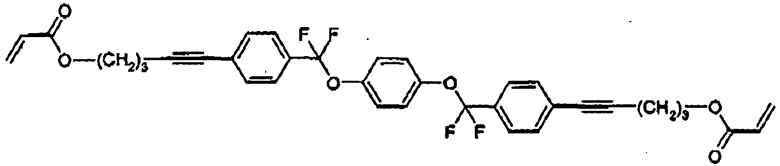

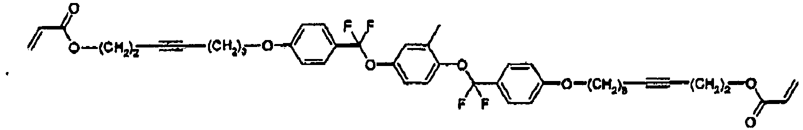

Jedoch wird die Verwendung solcher Verbindungen zur Stabilisierung von blauen Phasen oder in PSA-Anzeigen durch die JP 2005-015473 A weder beschrieben noch nahegelegt

Strukturverwandte, aber nicht polymeriserbare, flüssigkristalline Verbindungen werden zudem in EP 1 900 792 A1 und DE 10 2008 024866 offenbart.

Gegenstand der Erfindung ist die Verwendung von Verbindungen der Formel I

P^{a}-(Sp^{a})s₁-A²-Q¹-A¹-Q²-A³-(Sp^{b})ₛ₂-P^{b} I

worin die einzelnen Reste folgende Bedeutung besitzen
- P^{a}, P^{b}: jeweils unabhängig voneinander eine polymerisierbare Gruppe,
- Sp^{a}, Sp^{b}: jeweils unabhängig voneinander eine Abstandsgruppe,
- s1, s2: jeweils unabhängig voneinander 0 oder 1,
- Q¹, Q²: jeweils unabhängig voneinander -CF₂O- oder -OCF₂-,
- A¹, A², A³: jeweils unabhängig voneinander einen Rest ausgewählt aus folgenden Gruppen
a) der Gruppe bestehend aus trans-1,4-Cyclohexylen, 1.4-Cyclohexenylen und 1,4'-Bicyclohexylen, worin auch eine oder mehrere nicht benachbarte CH₂-Gruppen durch -O- und/oder -S- ersetzt sein können und worin auch ein oder mehrere H-Atome durch F ersetzt sein können,
b) der Gruppe bestehend aus 1,4-Phenylen und 1,3-Phenylen, worin auch eine oder zwei CH-Gruppen durch N ersetzt sein können und worin auch ein oder mehrere H-Atome durch L ersetzt sein können,
c) der Gruppe bestehend aus Tetrahydropyran-2,5-diyl, 1,3-Dioxan-2,5-diyl, Tetrahydrofuran-2,5-diyl, Cylcobut-1,3-diyl, Piperidin-1,4-diyl, Thiophen-2,5-diyl und Selenophen-2,5-diyl, welche auch ein oder mehrfach durch L substituiert sein können,
d) der Gruppe bestehend aus gesättigten, teilweise ungesättigten oder vollständig ungesättigten, und optional substituierten, polycyclischen Resten mit 5 bis 20 cyclischen C-Atomen, von denen auch eines oder mehrere durch Heteroatome ersetzt sein können, vorzugsweise ausgewählt aus der Gruppe bestehend aus Bicyclo[1.1.1]pentan-1,3-diyl, Bicyclo[2.2.2]octan-1,4-diyl, Spiro[3.3]heptan-2,6-diyl, 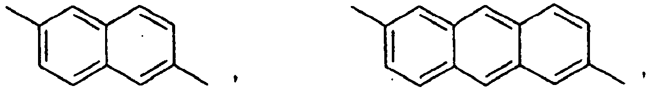 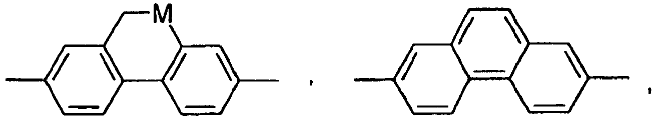 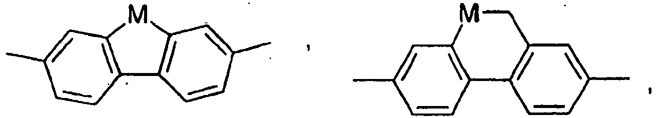 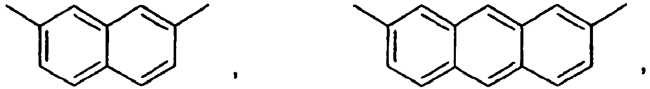 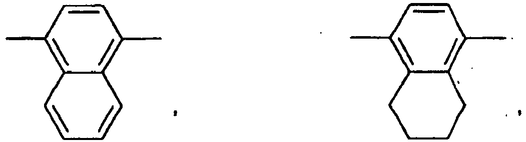 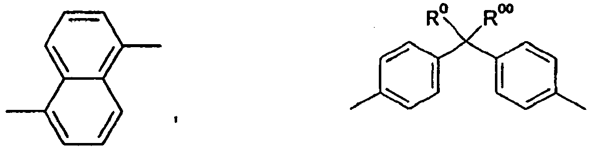 wobei in diesen Resten auch ein oder mehrere H- Atome durch L ersetzt sein können, und/oder eine oder mehrere Doppelbindungen durch Einfachbindungen ersetzt sein können, und/oder ein oder mehrere CH-Gruppen durch N ersetzt sein können,
- L: bei jedem Auftreten gleich oder verschieden F, CI, CN, SCN, SF₅ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen,
- R⁰, R⁰⁰: jeweils unabhängig voneinander H, F oder geradkettiges oder verzweigtes Alkyl mit 1 bis 12 C-Atomen, worin auch ein oder mehrere H-Atome durch F ersetzt sein können,
- M: -O-, -S-, -CH₂-, -CHY¹- oder -CY¹Y²-,
- Y¹, und Y²: jeweils unabhängig voneinander eine der oben für R⁰ angegebenen Bedeutungen, OCF₃, Cl oder CN, und vorzugsweise H, F, Cl, CN oder CF₃ und, alternativ, einer von Y¹ und Y² auch OCF₃,
oder die Verwendung eines Polymers erhältlich durch Polymerisation einer oder mehrerer Verbindungen der Formel I, in FK-Anzeigen mit blauer Phase.

Ein weiterer Gegenstand der Erfindung ist ein FK-Medium enthaltend eine oder mehrere Verbindungen der Formel I sowie gegebenenfalls zusätzlich eine oder mehrere polymerisierbare Verbindungen.

Ein weiterer Gegenstand der Erfindung ist ein FK-Medium enthaltend eine oder mehrere Verbindungen der Formel I sowie eine oder mehrere zusätzliche Verbindungen welche auch mesogen, flüssigkristallin und/oder polymerisierbar sein können.

Ein weiterer Gegenstand der Erfindung ist ein FK-Medium enthaltend ein Polymer erhältlich durch Polymerisation einer oder mehrerer Verbindungen der Formel I, sowie optional enthaltend eine oder mehrere zusätzliche Verbindungen, welche auch mesogen, flüssigkristallin und/oder polymerisierbar sein können.

Ein weiterer Gegenstand der Erfindung ist ein FK-Medium enthaltend
- eine polymerisierbare Komponente enthaltend eine oder mehrere polymerisierbare Verbindungen der Formel I, oder die polymerisierte Form dieser polymerisierbaren Komponente, sowie
- eine flüssigkristalline Komponente, im Folgenden auch als "FK-Hostmischung" bezeichnet, enthaltend eine oder mehrere, vorzugsweise zwei oder mehr niedermolekulare (d.h. monomere und unpolymerisierbare) Verbindungen wie vor- und nachstehend beschrieben, welche vorzugsweise mesogen oder flüssigkristallin sind.

Ein weiterer Gegenstand der Erfindung ist Verwendung von FK-Medien enthaltend eine oder mehrere Verbindungen der Formel I in FK-Anzeigen mit blauer Phase.

Ein weiterer Gegenstand der Erfindung ist ein Verfahren zur Herstellung eines FK-Mediums wie vor- und nachstehend beschrieben, indem man eine oder mehrere niedermolekulare flüssigkristalline Verbindungen, oder eine FK-Hostmischung wie vor- und nachstehend beschrieben, mit einer oder mehreren Verbindungen der Formel I und gegebenenfalls mit weiteren flüssigkristallinen Verbindungen und/oder Additiven, mischt.

Ein weiterer Gegenstand der Erfindung ist die Verwendung von Verbindungen der Formel I und diese enthaltende erfindungsgemäße FK-Medien in FK-Anzeigen zur Stabilisierung der blauen Phase, Insbesondere über einen möglichst großen Temperaturbereich.

Die Verbindungen der Formel I und diese enthaltende erfindungsgemäße FK-Medien eignen sich zur Verwendung in PS- und PSA-Anzeigen zur Erzeugung eines Tiltwinkels im FK-Medium durch in situ-Polymerisation der Verbindung(en) der Formel I in der PSA-Anzeige, vorzugsweise unter Anlegen eines elektrischen oder magnetischen Feldes.

Ein weiterer Gegenstand der Erfindung ist eine FK-Anzeige enthaltend eine oder mehrere Verbindungen der Formel I oder ein erfindungsgemäßes FK-Medium, insbesondere eine Anzeige mit blauer Phase.

Möglich, aber nicht erfindungsgemäß, ist auch die Realisierung einer FK-Anzeige des PS- oder PSA-Typs, enthaltend eine FK-Zelle mit zwei Substraten und zwei Elektroden, wobei mindestens ein Substrat lichtdurchlässig ist und mindestens ein Substrat eine oder zwei Elektroden aufweist, sowie einer zwischen den Substraten befindlichen Schicht eines FK-Mediums enthaltend eine polymerisierte Komponente und eine niedermolekulare Komponente, wobei die polymerisierte Komponente erhältlich ist durch

Polymerisation einer oder mehrerer polymerisierbarer Verbindungen zwischen den Substraten der FK-Zelle im FK-Medium, vorzugsweise unter Anlegen einer elektrischen Spannung an die Elektroden, wobei mindestens eine der polymerisierbaren Verbindungen aus Formel I ausgewählt ist.

Ein weiterer Gegenstand der Erfindung ist ein Verfahren zur Herstellung einer FK-Anzeige wie vor- und nachstehend beschrieben, indem man ein FK-Medium, enthaltend eine oder mehrere niedermolekulare flüssigkristalline Verbindungen oder eine FK-Hostmischung wie vor- und nachstehend beschrieben sowie eine oder mehrere polymerisierbare Verbindungen wovon mindestens eine aus Formel I ausgewählt ist, in eine FK-Zelle mit zwei Substraten und zwei Elektroden wie vor- und nachstehend beschrieben füllt, und die polymerisierbaren Verbindungen, polymerisiert.

Die PS- und PSA-Anzeigen weisen zwei Elektroden, vorzugsweise in Form von transparenten Schichten, auf, wobei diese auf einem oder beiden der Substrate aufgebracht sind, die die FK-Zelle bilden. Dabei ist entweder jeweils eine Elektrode auf je einem der beiden Substrate aufgebracht, wie zum Beispiel in PSA-VA-, PSA-OCB- oder PSA-TN-Anzeigen, oder beide Elektroden sind auf nur einem der beiden Substrate aufgebracht, während das andere Substrat keine Elektrode aufweist, wie zum Beispiel in PSA-IPS- oder PSA-FFS-Anzeigen.

Weiterer Gegenstand der Erfindung sind neue Verbindungen der Formel I, Verfahren zu ihrer Herstellung, sowie in diesen Verfahren verwendete oder daraus erhaltene neue Zwischenprodukte, insbesondere Verbindungen der Formel I, sowie deren Unterformeln wie vor- und nachstehend definiert, worin einer oder mehrere der Reste A¹, A² und A³ ausgewählt ist aus der Gruppe d) wie in Formel I definiert, bestehend aus optional substituierten, gesättigten oder teilweise oder vollständig ungesättigten, polycyclischen Resten mit 5 bis 20 cyclischen C-Atomen, von denen auch ein oder mehrere durch Heteroatome ersetzt sein können.

Besonders bevorzugt ist ein FK-Medium, eine FK-Anzeige, ein Verfahren oder ein Verwendung wie vor- und nachstehend beschrieben, worin das FK-Medium bzw. die darin enthaltene polymerisierbare oder polymerisierte Komponente keine Verbindungen der folgenden Formel enthalten: 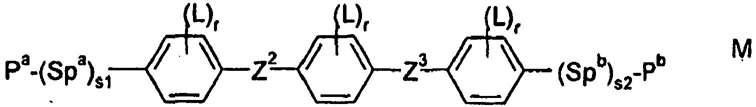 worin P^{a}, P^{b}, Sp^{a}, Sp^{b}, s1, s2 und L r die vor- und nachstehend angegebene Bedeutung besitzen, r 0, 1, 2, 3 oder 4 bedeutet, und Z² und Z³ jeweils unabhängig voneinander -COO- oder -OCO- bedeuten.

Vor- und nachstehend gelten folgende Bedeutungen:
Der Begriff "cyclisches C-Atom" bedeutet ein C-Atom, welches mit anderen C-Atomen und/oder Heteroatomen einen carbo- oder heterocyclischen Rest bildet.

Die Begriffe "Tilt" und "Tiltwinkel" beziehen sich auf eine gekippte oder geneigte Orientierung der FK-Moleküle eines FK-Mediums relativ zu den Oberflächen der Zelle in einer FK-Anzeige (hier vorzugsweise einer PS- oder PSA-Anzeige). Der Tiltwinkel bezeichnet dabei den durchschnittlichen Winkel (<90°) zwischen den Moleküllängsachsen der FK-Moleküle (FK-Direktor) und der Oberfläche der planparallelen Trägerplatten, welche die FK-Zelle bilden. Ein niedriger Wert des Tiltwinkels (d.h. eine große Abweichung vom 90°-Winkel) entspricht dabei einem großen Tilt. Eine geeignete Methode zur Messung des Tiltwinkels findet sich in den Beispielen. Soweit nicht anders angegeben, beziehen sich vor- und nachstehend offenbarte Werte des Tiltwinkels auf diese Messmethode.

Der Begriff "mesogene Gruppe" ist dem Fachmann bekannt und in der Literatur beschrieben, und bedeutet eine Gruppe, die durch die Anisotropie ihrer anziehenden und abstoßenden Wechselwirkungen wesentlich dazu beiträgt, in niedermolekularen oder polymeren Substanzen eine Flüssigkristall(FK-)Phase hervorzurufen. Verbindungen enthaltend mesogene Gruppen (mesogene Verbindungen) müssen nicht unbedingt selbst eine FK-Phase aufweisen. Es ist auch möglich, dass mesogene Verbindungen FK-Phasenverhalten nur nach Vermischung mit anderen Verbindungen und/oder nach Polymerisation zeigen. Typische mesogene Gruppen sind beispielsweise starre stäbchen- oder scheibchenförmige Einheiten. Ein Überblick über die im Zusammenhang mit mesogenen bzw. FK-Verbindungen verwendeten Begriffe und Definitionen findet sich in Pure Appl. Chem. 73(5), 888 (2001) und C. Tschierske, G. Pelzl, S. Diele, Angew. Chem. 2004, 116, 6340-6368.

Der Begriff "Abstandsgruppe" (engl. "spacer" oder "spacer group"), vor- und nachstehend auch als "Sp" bezeichnet, ist dem Fachmann bekannt und in der Literatur beschrieben, siehe beispielsweise Pure Appl. Chem. 73(5), 888 (2001) und C. Tschierske, G. Pelzl, S. Diele, Angew. Chem. 2004, 116, 6340-6368. Falls nicht anders angegeben, bezeichnet der Begriff "Abstandsgruppe" bzw. "Spacer" vor- und nachstehend eine flexible Gruppe, die in einer polymerisierbaren mesogenen Verbindung die mesogene Gruppe und die polymerisierbare(n) Gruppe(n) miteinander verbindet.

Der Begriff "reaktives Mesogen" oder "RM" bezeichnet eine Verbindung enthaltend eine mesogene Gruppe und eine oder mehrere funktionelle Gruppen, die zur Polymerisation geeignet sind (auch als polymerisierbare Gruppe oder Gruppe P bezeichnet).

Die Begriffe "niedermolekulare Verbindung" und "unpolymerisierbare Verbindung" bezeichnen, üblicherweise monomere, Verbindungen, die keine funktionelle Gruppe aufweisen, welche zur Polymerisation unter den üblichen dem Fachmann bekannten Bedingungen, insbesondere unter den zur Polymerisation der RMs verwendeten Bedingungen, geeignet ist.

"Halogen" bedeutet F, CI, Br oder I.

Definitionen wie "Alkyl, Alkoxy, Alkylcarbonyl, Alkoxycarbonyl, Alkylcarbonyloxy oder Alkoxycarbonyloxy mit 1 bis 12 C-Atomen" etc., bedeuten, dass die Reste enthaltend eine Carbonylgruppe (CO) sowie die ungesättigten Reste wie Alkenyl und Alkinyl mindestens zwei C-Atome, und die verzweigten Reste mindestens drei C-Atome aufweisen.

Die polymerisierbare Gruppe P^{a,b} ist eine Gruppe, die für eine Polymerisationsreaktion, wie beispielsweise die radikalische oder ionische Kettenpolymerisation, Polyaddition oder Polykondensation, oder für eine polymeranaloge Umsetzung, beispielsweise die Addition oder Kondensation an eine Polymerhauptkette, geeignet ist. Besonders bevorzugt sind Gruppen für die Kettenpolymerisation, insbesondere solche enthaltend eine C=C-Doppelbindung oder -C≡C-Dreifachbindung, sowie zur Polymerisation unter Ringöffnung geeignete Gruppen wie beispielsweise Oxetan- oder Epoxygruppen

Bevorzugte Gruppen P^{a,b} sind ausgewählt aus der Gruppe bestehend aus CH₂=CW¹-CO-O-, CH₂=CW¹-CO-, 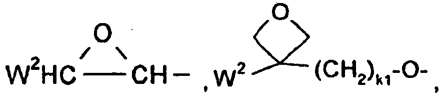 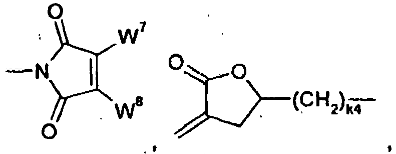 CH₂=CW²-(O)ₖ₃-, CW¹=CH-CO-(O)ₖ₃-, CW¹=CH-CO-NH-, CH₂=CW¹-CO-NH-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH-, HOOC-, OCN-, und W⁴W⁵W⁶Si-, worin W¹ H, F, CI, CN, CF₃, Phenyl oder Alkyl mit 1 bis 5 C-Atomen, insbesondere H, F, CI oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, CI oder Alkyl mit 1 bis 5 C-Atomen bedeuten, Phe 1,4-Phenylen bedeutet, welches optional mit einem oder mehreren, von P-Sp- verschiedenen Resten L wie oben definiert substituiert ist, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Besonders bevorzugte Gruppen P^{a,b} sind ausgewählt aus der Gruppe bestehend aus CH₂=CW¹-CO-O-, CH₂=CW¹-CO-, 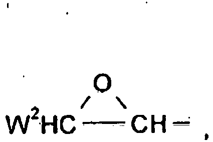 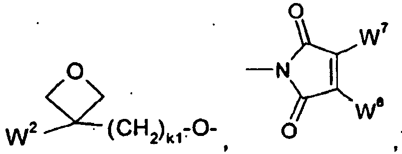 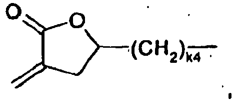 CH₂=CW²-O-, CW¹=CH-CO-(O)ₖ₃-, CW¹=CH-CO-NH-, CH₂=CW¹-CO-NH-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, CH₂=CW¹-CO-NH-, CH₂=CH-(COO)ₖ₋₁-Phe-(O)ₖ₂-, CH₂=CH-(CO)ₖ₁-Phe-(O)ₖ₂-, Phe-CH=CH- und W⁴W⁵W⁶Si-, worin W¹ H, F, Cl, CN, CF₃, Phenyl oder Alkyl mit 1 bis 5 C-Atomen, insbesondere H, F, CI oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander CI, Oxaalkyl oder Oxacarbonylalkyl mit 1 bis 5 C-Atomen bedeuten, W⁷ und W⁸ jeweils unabhängig voneinander H, CI oder Alkyl mit 1 bis 5 C-Atomen bedeuten, Phe 1,4-Phenylen bedeutet, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Ganz besonders bevorzugte Gruppen P^{a,b} sind ausgewählt aus der Gruppe bestehend aus CH₂=CW¹-CO-O-, insbesondere CH₂=CH-CO-O-, CH₂=C(CH₃)-CO-O- und CH₂=CF-CO-O-, ferner CH₂=CH-O-, (CH₂=CH)₂CH-O-CO-, (CH₂=CH)₂CH-O-, 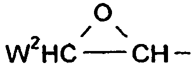 und

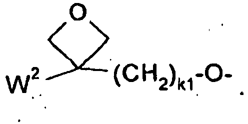

Weitere ganz besonders bevorzugte Gruppen P^{a,b} sind ausgewählt aus der Gruppe bestehend aus Vinyloxy-, Acrylat-, Methacrylat-, Fluoracrylat-, Chloracrylat-, Oxetan- und Epoxygruppen, und bedeuten besonders bevorzugt eine Acrylat- oder Methacrylatgruppe.

Bevorzugte Abstandsgruppen Sp^{a,b} sind ausgewählt aus der Formel Sp"-X", so dass der Rest P^{a/b}-Sp^{a/b}- der Formel P^{a/b}-Sp"-X"- entspricht, wobei
- Sp": Alkylen mit 1 bis 20, vorzugsweise 1 bis 12 C-Atomen bedeutet, welches optional durch F, Cl, Br, I oder CN ein- oder mehrfach substituiert ist, und worin auch eine oder mehrere nicht benachbarte CH₂-Gruppen jeweils unabhängig voneinander so durch -O-, -S-, -NH-, -N(R⁰)-, -Si(R⁰⁰R⁰⁰⁰)-, -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- oder -C≡C- ersetzt sein können, dass O- und/oder S-Atome nicht direkt miteinander verknüpft sind,
- X": -O-, -S-, -CO-, -CO-O-, -O-CO-, -O-CO-O-, -CO-N(R⁰⁰)-, -N(R⁰⁰)-CO-, -N(R⁰⁰)-CO-N(R⁰⁰)-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-CO-O-, -O-CO-CH=CH- oder eine Einfachbindung bedeutet,
- R⁰⁰ und R⁰⁰⁰: jeweils unabhängig voneinander H oder Alkyl mit 1 bis 12 C-Atomen bedeuten, und
- Y² und Y³: jeweils unabhängig voneinander H, F, CI oder CN bedeuten.
- X': ist vorzugsweise -O-, -S -CO-, -COO-, -OCO-, -O-COO-, -CO-NR⁰-,-NR⁰-CO-, -NR⁰-CO-NR⁰- oder eine Einfachbindung.

Typische Abstandsgruppen Sp" sind beispielsweise -(CH₂)ₚ₁-, -(CH₂CH₂O)_{q1} - CH₂CH₂-, -CH₂CH₂-S-CH₂CH₂-, -CH₂CH₂-NH-CH₂CH₂- oder -(SiR⁰⁰R⁰⁰⁰-O)ₚ₁- , worin p1 eine ganze Zahl von 1 bis 12 ist, q1 eine ganze Zahl von 1 bis 3 ist, und R⁰⁰ und R⁰⁰⁰ die oben angegebenen Bedeutungen besitzen.

Besonders bevorzugte Gruppen -Sp"-X"- sind -(CH₂)p₁-, -(CH₂)ₚ₁-O-, -(CH₂)ₚ₁-O-CO-, -(CH₂)ₚ₁-O-CO-O-, worin p1 und q1 die oben angegebene Bedeutung haben.

Besonders bevorzugte Gruppen Sp" sind beispielsweise jeweils geradkettiges Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyl-iminoethylen, 1-Methylalkylen, Ethenylen, Propenylen und Butenylen.

In einer weiteren bevorzugten Ausführungsform der Erfindung bedeuten P^{a} und/oder P^{b} in Formel I einen Rest mit zwei oder mehreren polymerisierbaren Gruppen (multifunktionelle polymerisierbare Reste). Geeignete Reste dieses Typs, sowie diese enthaltende polymerisierbare Verbindungen und ihre Herstellung sind beispielsweise in US 7,060,200 B1 oder US 2006/0172090 A1 beschrieben. Besonders bevorzugt sind multifunktionelle polymerisierbare Reste ausgewählt aus folgenden Formeln

-X-alkyl-CHP¹-CH₂-CH₂P² I*a

-X-alkyl-C(CH₂P¹)(CH₂P²)-CH₂P³ I*b

-X-alkyl-CHP¹CHP²-CH₂P³ I*c

-X-alkyl-C(CH₂P¹)(CH₂P²)-CₐₐH₂ₐₐ₊₁ I*d

-X-alkyl-CHP¹-CH₂P² I*e

-X-alkyl-CHP¹P² I*f

-X-alkyl-CP¹P²-CₐₐH₂ₐₐ₊₁ I*g

-X-alkyl-C(CH₂P¹)(CH₂P²)-CH₂OCH₂-C(CH₂P³)(CH₂P⁴)CH₂P⁵ I*h

-X-alkyl-CH((CH₂)ₐₐP¹)((CH₂)_{bb}P²) I*i

-X-alkyl-CHP¹CHP²-CₐₐH₂ₐₐ₊₁ I*k

-X'-alkyl-C(CH₃)(CH₂P¹)(CH₂P²) I*m

worin
- alkyl: eine Einfachbindung oder geradkettiges oder verzweigtes Alkylen mit 1 bis 12 C-Atomen bedeutet, worin eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, - CO-, -CO-O-, -O-CO-, -O-CO-O- so ersetzt sein können, dass O- und/oder S-Atome nicht direkt miteinander verknüpft sind, und worin auch ein oder mehrere H-Atome durch F, CI oder CN ersetzt sein können, wobei R⁰⁰ und R⁰⁰⁰ die oben angegebene Bedeutung haben,
- aa und bb: jeweils unabhängig voneinander 0, 1, 2, 3, 4, 5 oder 6 bedeuten,
- X: eine der für X' angegebenen Bedeutungen besitzt, und
- P¹⁻⁵: jeweils unabhängig voneinander eine der für P^{a} angegebenen Bedeutungen besitzen..

Vorzugsweise bedeutet A¹ in Formel I einen Rest ausgewählt aus der Gruppe bestehend aus folgenden Formeln: 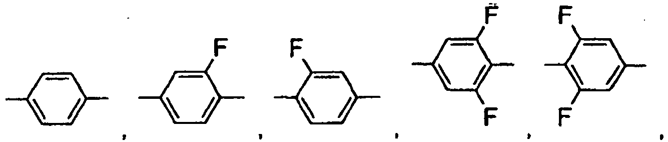 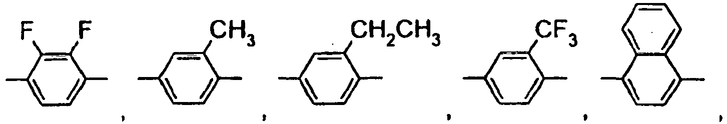 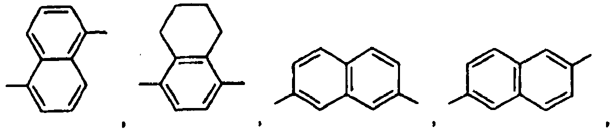 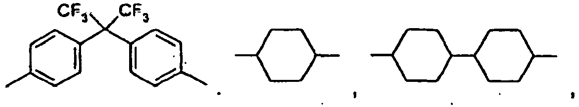 worin die einzelnen Ringe auch zusätzlich ein- oder mehrfach durch L vor- und nachstehend beschrieben substituiert sein können.

Besonders bevorzugt bedeutet A¹ in Formel I einen Rest ausgewählt aus der Gruppe bestehend aus folgenden Formeln

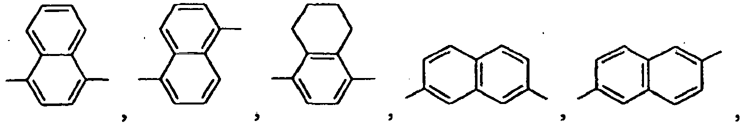

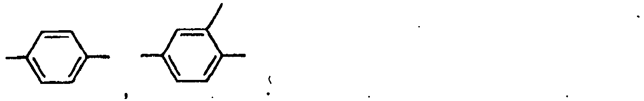

Vorzugsweise bedeuten A² und A³ in Formel I jeweils unabhängig voneinander einen Rest ausgewählt aus der Gruppe bestehend aus folgenden Formeln: 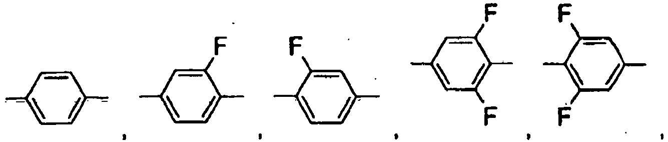 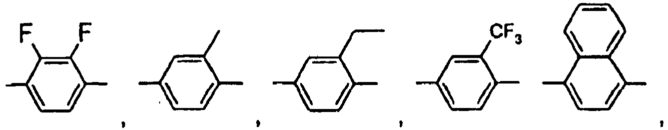 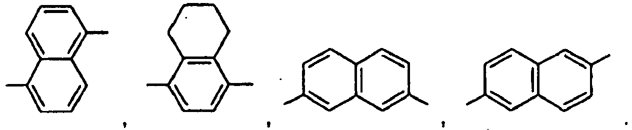 worin die einzelnen Ringe auch zusätzlich ein- oder mehrfach durch L wie vor- und nachstehend beschrieben substituiert sein können.

Besonders bevorzugt bedeuten A² und A³ in Formel I jeweils unabhängig voneinander einen Rest ausgewählt aus der Gruppe bestehend aus folgenden Formeln: 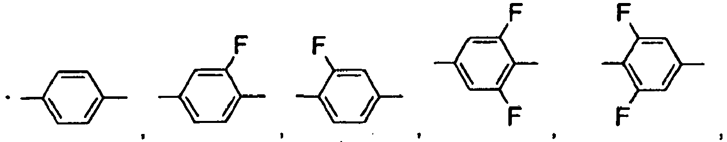 ganz besonders bevorzugt

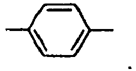

Weiterhin bevorzugt bedeuten A² und A³ in Formel I jeweils unabhängig voneinander einen Rest ausgewählt aus der Gruppe bestehend aus folgenden Formeln:

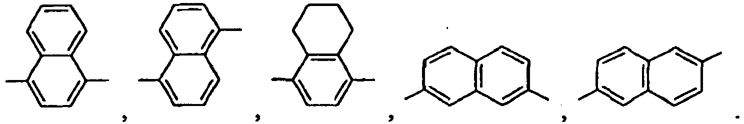

Weitere besonders bevorzugte Verbindungen der Formel I sowie deren vor- und nachstehend angegebenen Unterformeln sind solche worin
- Q¹ -CF₂O- und Q² -OCF₂- bedeutet,
- Q¹ -OCF₂- und Q² -CF₂O- bedeutet,
- Q¹ und Q² -CF₂O- bedeuten,
- Q¹ und Q² -OCF₂- bedeuten,
- s1 und s2 jeweils 0 bedeuten
- s1 und s2 jeweils 1 bedeuten,
- s1 1 und s2 0 bedeutet oder s1 0 und s2 1 bedeutet,
- A² und A³ die gleich Bedeutung besitzen

Ganz besonders bevorzugte Verbindungen der Formel I sowie deren vor- und nachstehend angegebenen Unterformeln sind solche worin Q¹ -CF₂O- und Q² -OCF₂- bedeutet.

Ganz besonders bevorzugte Verbindungen der Formel I sind ausgewählt aus der Gruppe bestehend aus den folgenden Formeln: 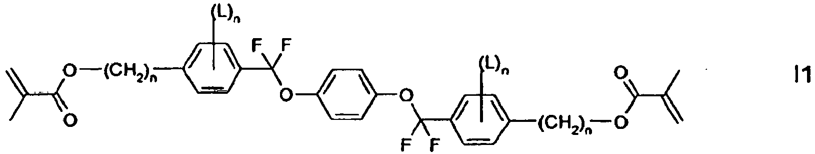 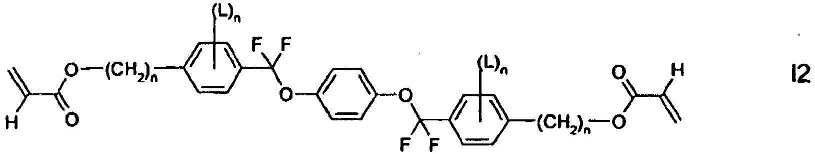 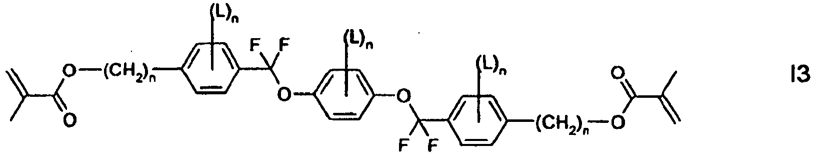 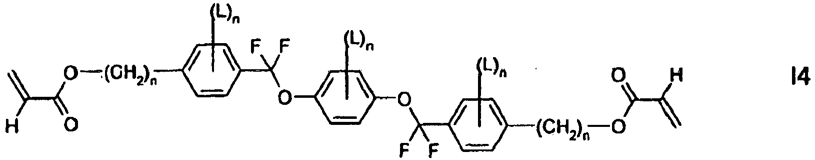 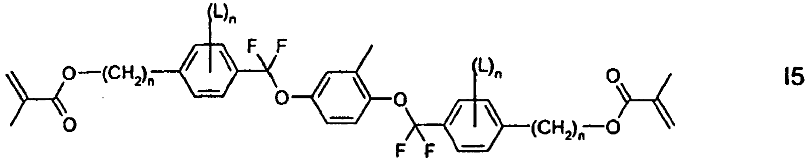 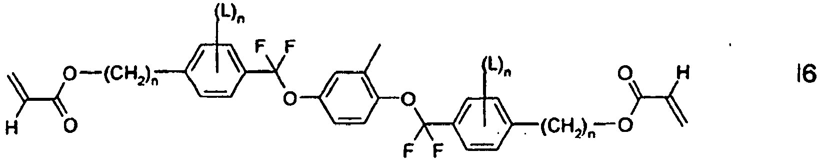 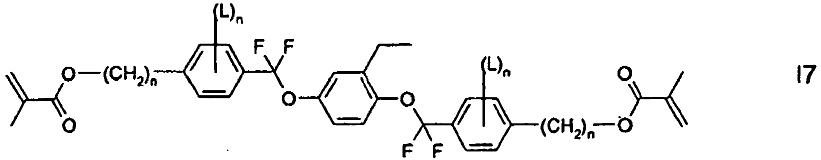 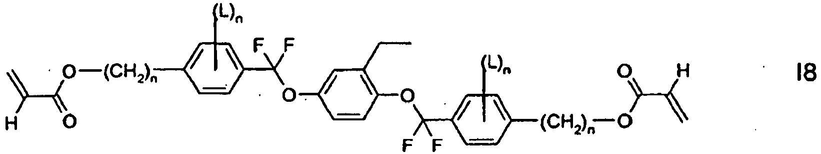 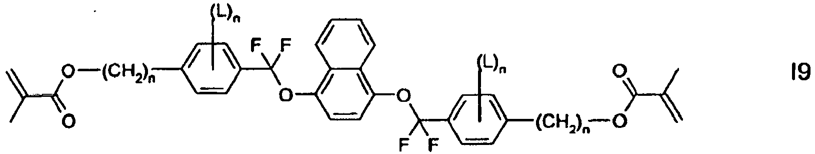 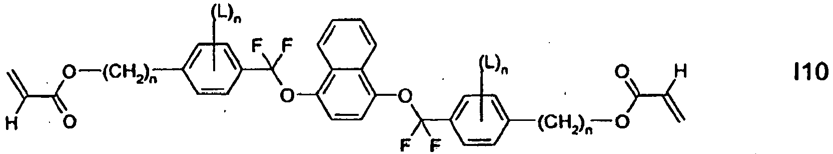 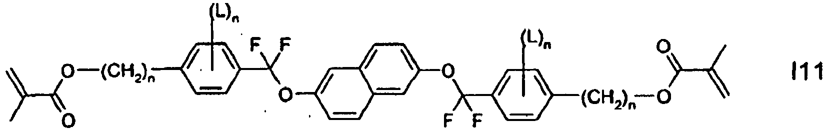 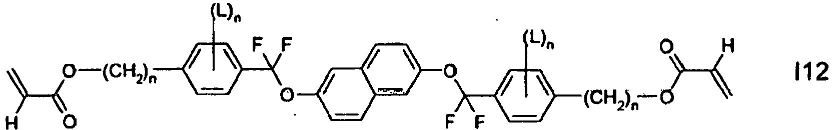 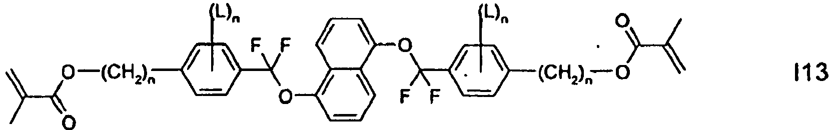 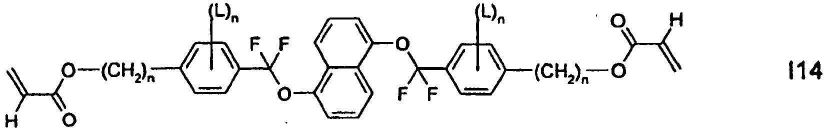 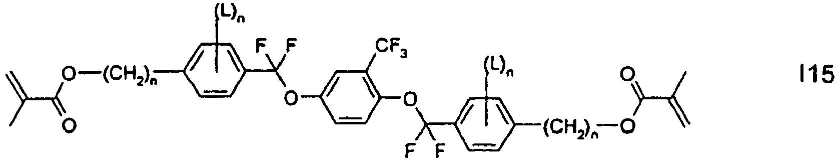 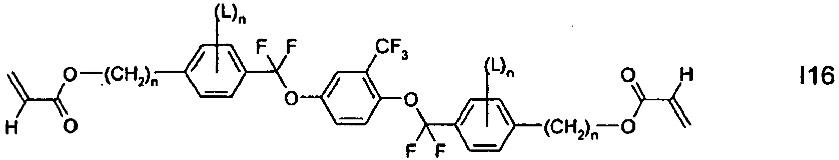 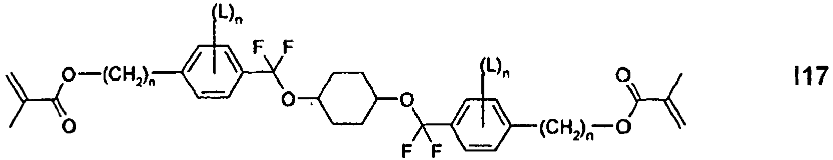 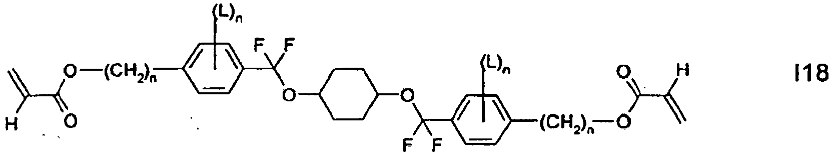 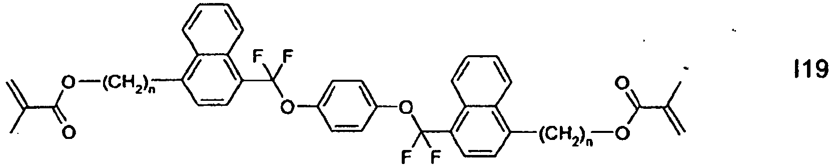 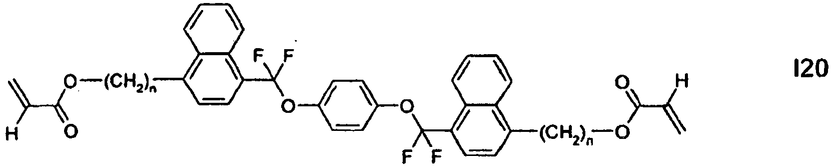 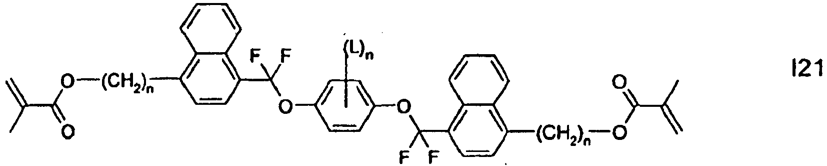 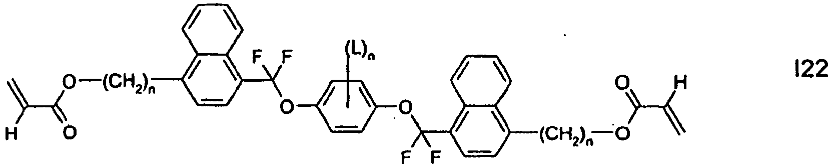 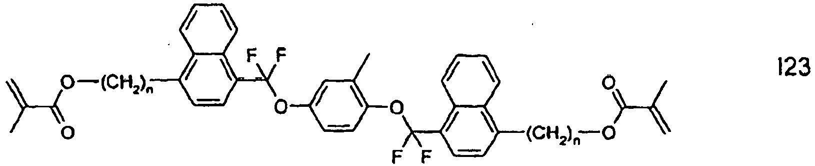 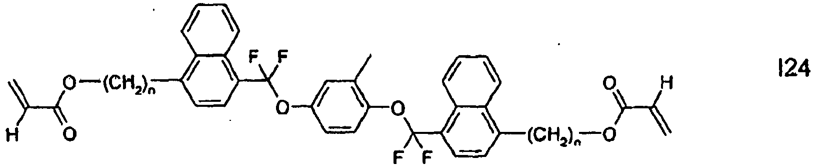 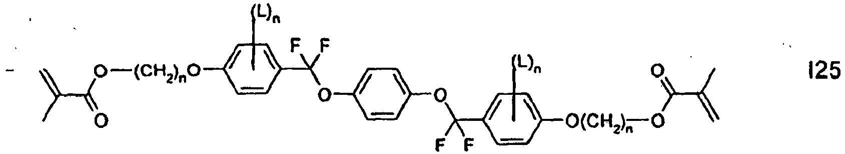 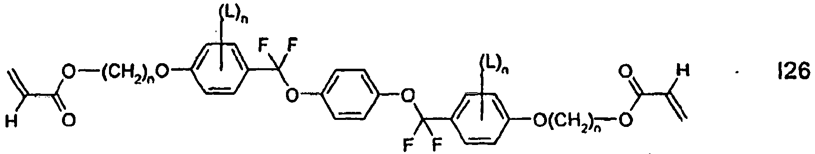 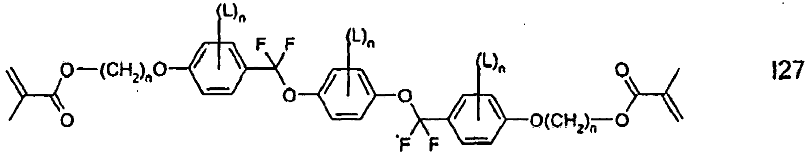 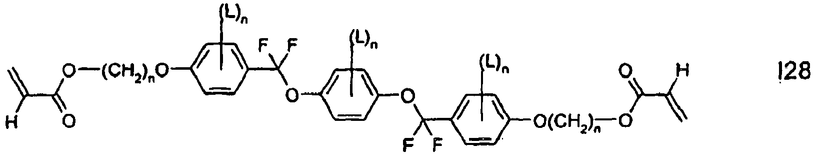 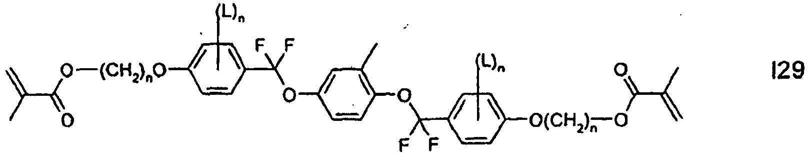 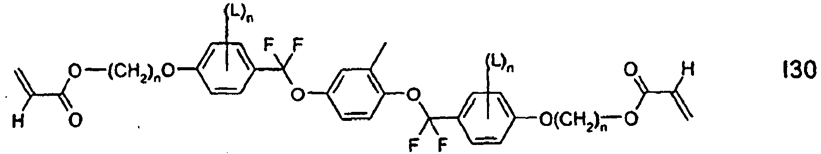 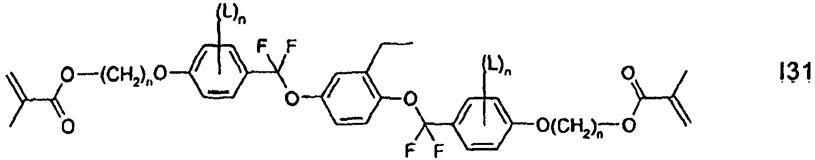 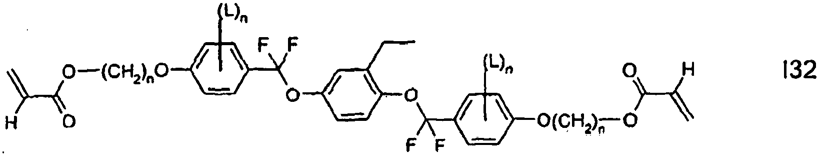 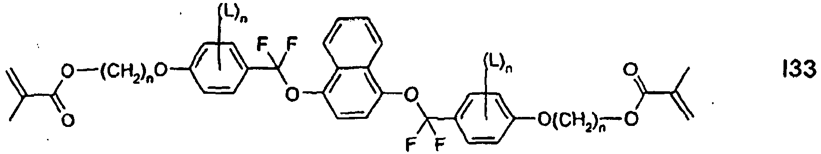 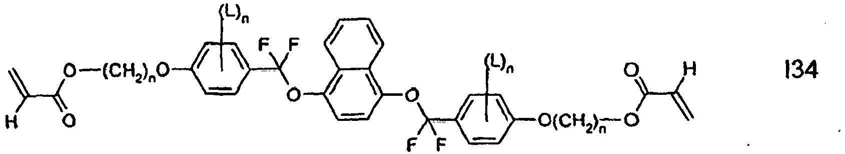 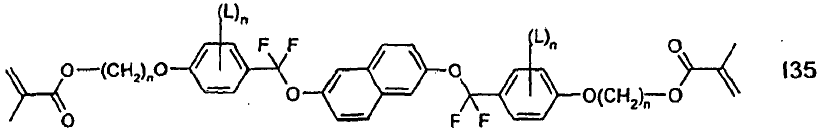 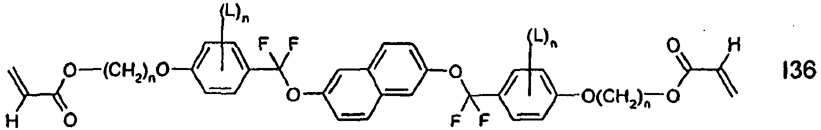 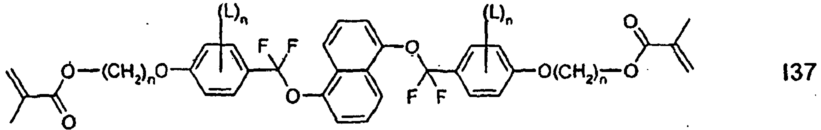 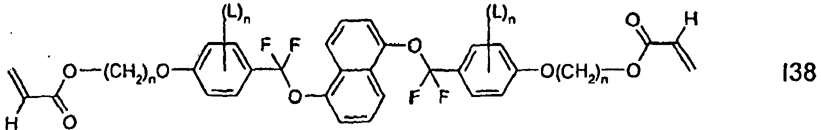 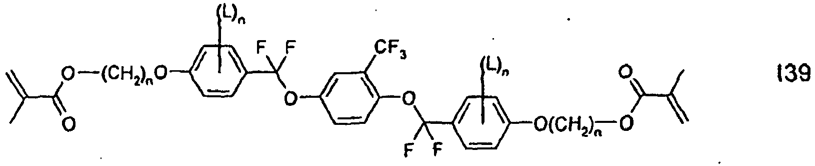 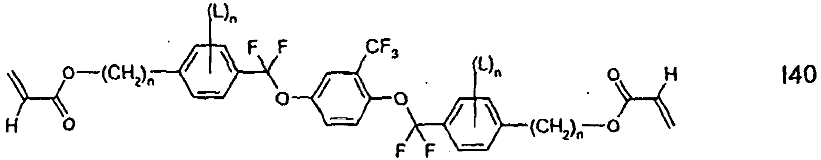 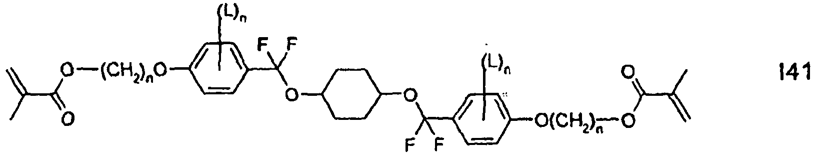 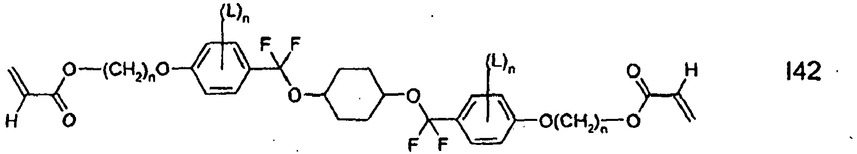 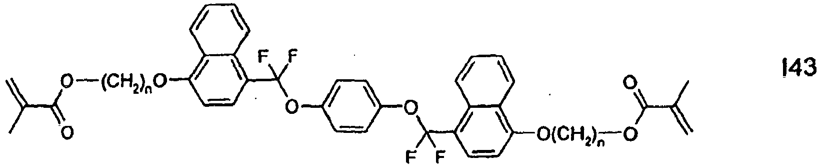 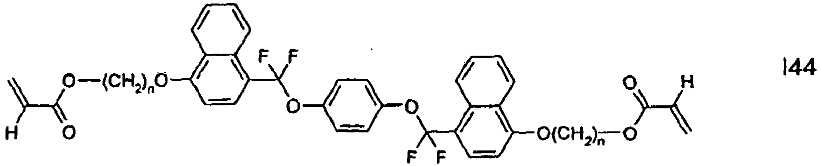 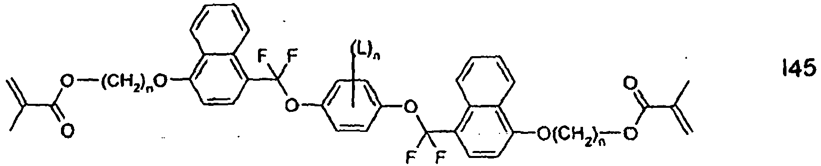 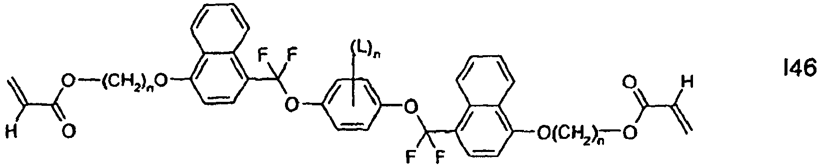 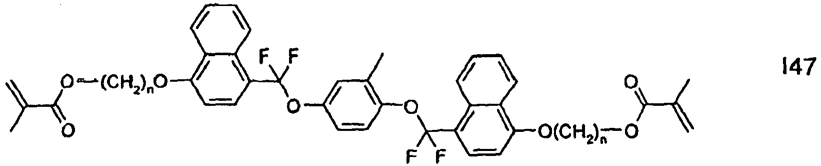 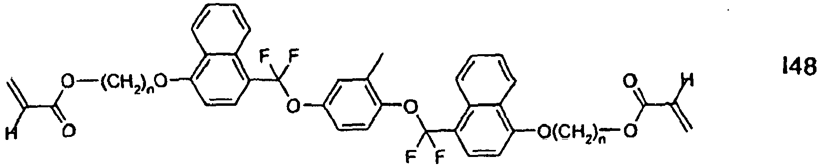 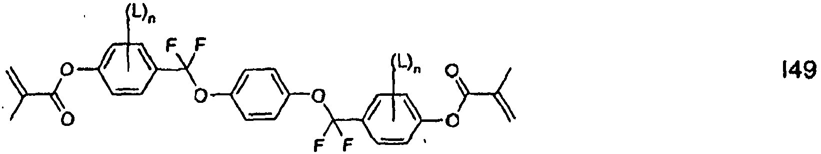 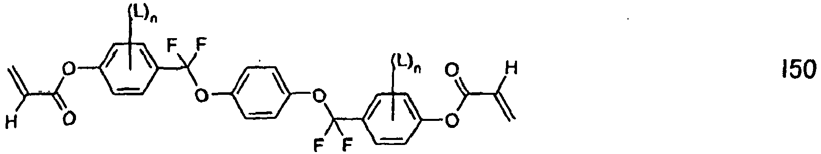 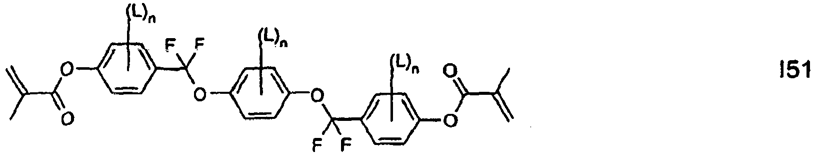 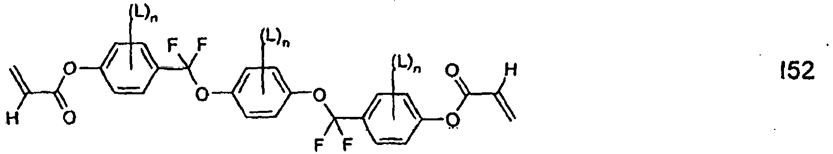 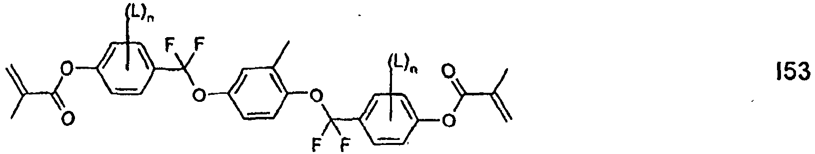 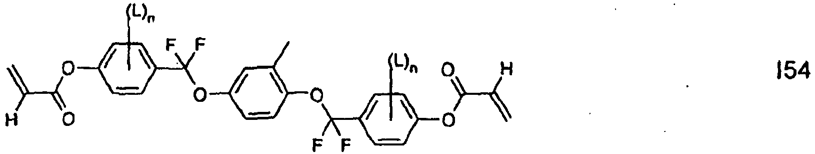 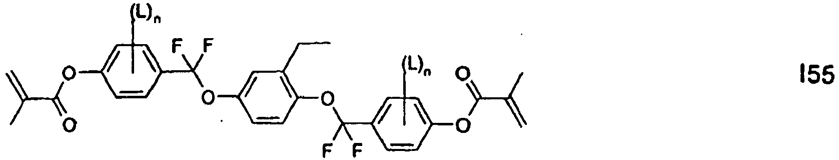 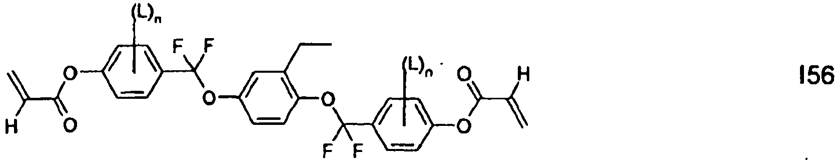 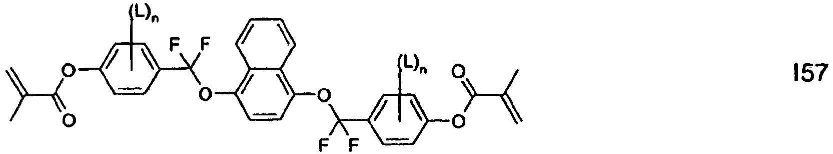 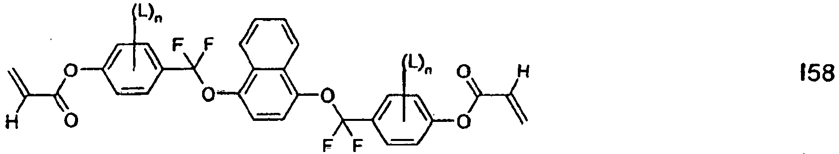 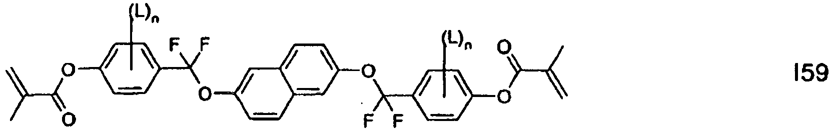 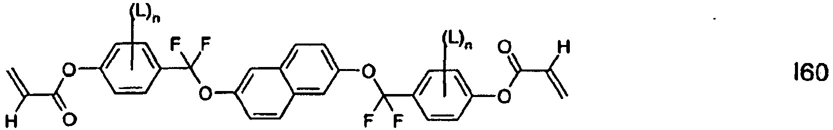 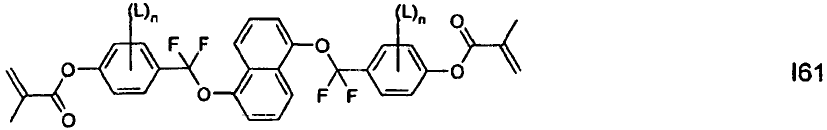 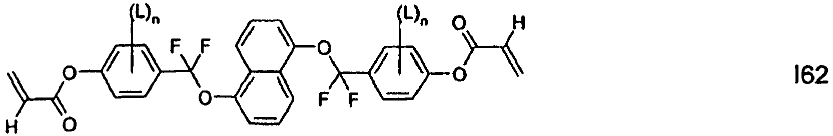 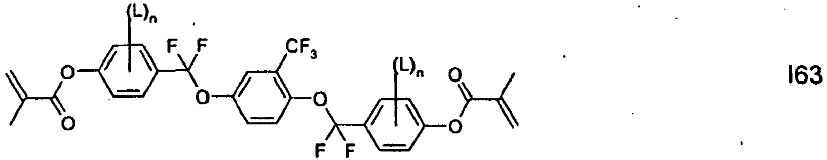 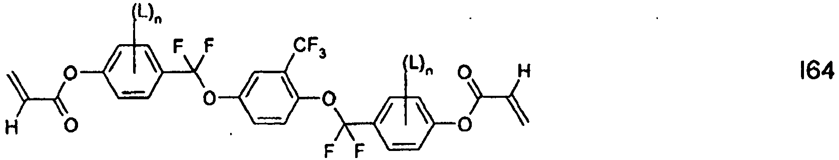 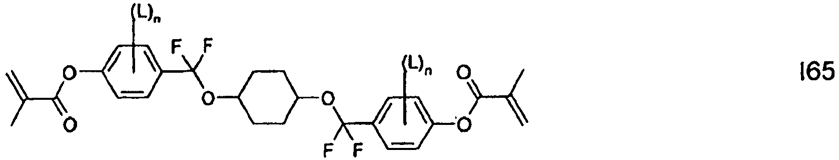 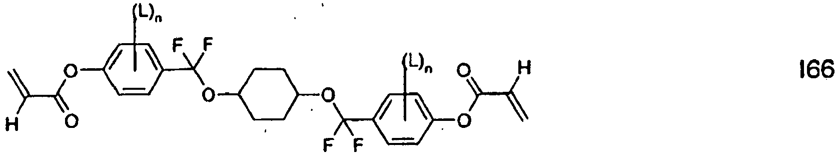 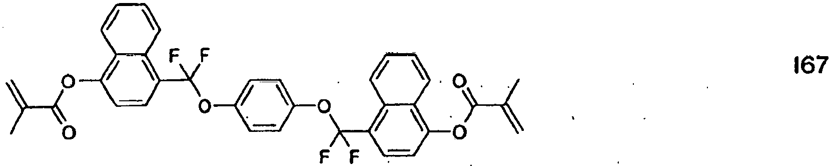 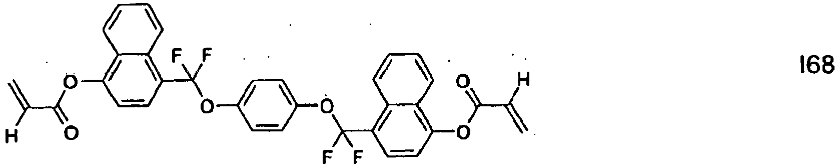 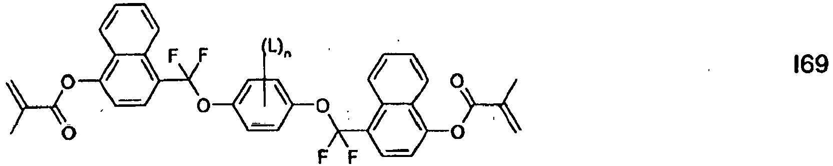 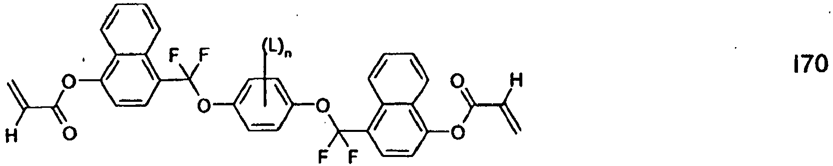 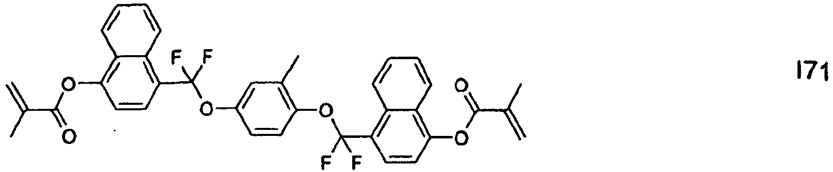 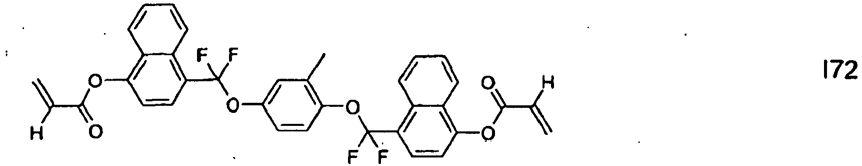 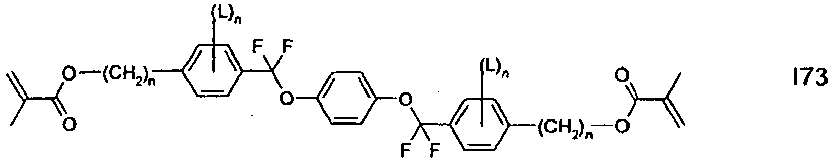 worin L bei jedem Auftreten gleich oder verschieden eine der vor- und nachstehend angegebenen Bedeutungen besitzt, r 0, 1, 2, 3 oder 4 bedeutet, und n eine ganze Zahl zwischen 1 und 24, bevorzugt zwischen 1 und 12, ganz besonders bevorzugt zwischen 2 und 8 bedeutet, und worin, falls ein Rest am Ende einer Einfach- bzw. Doppelbindung nicht genannt ist, es sich um eine endständige CH₃- bzw. CH₂-Gruppe handelt.

In den Formeln I1-I73 bedeutet 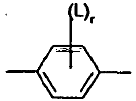 vorzugsweise eine Gruppe ausgewählt aus der Gruppe bestehend aus folgenden Formeln 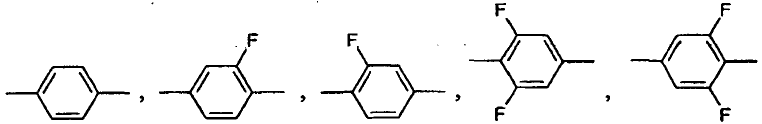 und

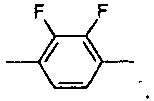

P^{a} und P^{b} bedeuten in den Verbindungen der Formel I sowie deren Unterformeln vorzugsweise Acrylat oder Methacrylat, ferner Fluoracrylat. Sp^{a} und Sp^{b} bedeuten in den Verbindungen der Formel I und deren Unterformeln vorzugsweise eine Rest ausgewählt aus der Gruppe bestehend aus -(CH₂)ₚ₁-, -(CH₂)ₚ₁-O-, -(CH₂)ₚ₁-O-CO- oder -(CH₂)ₚ₁-O-CO-O- und deren Spiegelbildern, worin p1 eine ganze Zahl von 1 bis 12, vorzugsweise von 1 bis 6, besonders bevorzugt 1, 2 oder 3 bedeutet, wobei diese Gruppen so mit P^{a} oder P^{b} verknüpft sind, dass O-Atome nicht direkt benachbart sind.

Ein weiterer Gegenstand der Erfindung sind neue Verbindungen der Formel I

P^{a}-(Sp^{a})ₛ₁-A²-Q¹-A¹-Q²-A³-(Sp^{b})ₛ₂-P^{b} I

worin P^{a}, P^{b}, Sp^{a}, Sp^{b}, s1, s2, Q¹, Q², A¹, A² und A³ die vor- und nachstehend angegebenen Bedeutungen besitzen, und worin einer oder mehrere der Reste A¹, A² und A³ ausgewählt sind aus der Gruppe b1) bestehend aus 1,3-Phenylen, worin auch eine oder zwei CH-Gruppen durch N ersetzt sein können und worin auch ein oder mehrere H-Atome durch L ersetzt sein können, oder der Gruppe d) bestehend aus gesättigten, teilweise gesättigten oder vollständig ungesättigten, und optional substituierten, polycyclischen Resten mit 5 bis 20 cyclischen C-Atomen, von denen auch eines oder mehrere durch Heteroatome ersetzt sein können, vorzugsweise worin einer oder mehrere der Reste A¹, A² und A³ ausgewählt sind aus der Gruppe bestehend aus 1,3-Phenylen, Bicyclo[1.1.1]pentan-1,3-diyl, Bicyclo[2.2.2]octan-1,4-diyl, Spiro[3.3]heptan-2,6-diyl, 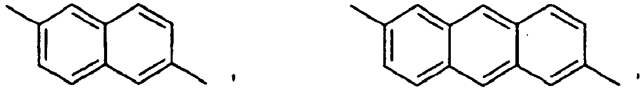 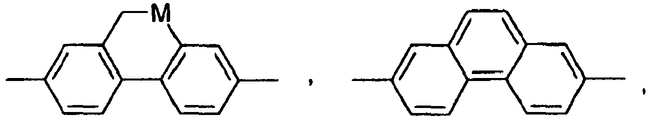 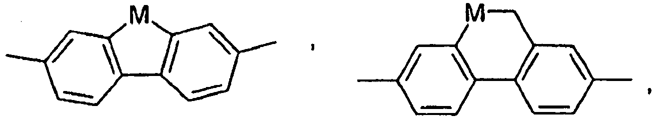 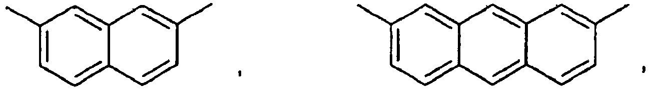 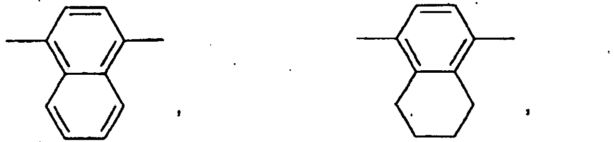 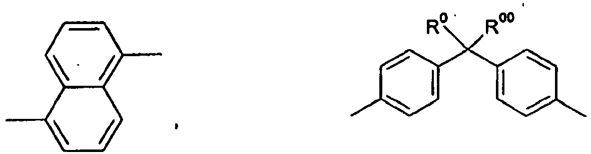 wobei in diesen Resten auch ein oder mehrere H-Atome durch L ersetzt sein können, und /oder eine oder mehrere Doppelbindungen durch Einfachbindungen ersetzt sein können, und/oder ein oder mehrere CH-Gruppen durch N ersetzt sein können, wobei L, R⁰, R⁰⁰, M, Y¹ und Y² die in Formel I angegebene Bedeutung besitzen.

Von diesen neuen Verbindungen sind solche besonders bevorzugt, worin
- A¹ einen Rest ausgewählt aus der Gruppe b1) oder d) bedeutet,
- A² und/oder A³ einen Rest ausgewählt aus der Gruppe b1) oder d) bedeutet,
- A¹ einen Rest ausgewählt aus der Gruppe b1) oder d) bedeutet, und A² und A³ keinen Rest ausgewählt aus der Gruppe d) bedeuten,
- A² und/oder A³ einen Rest ausgewählt aus der Gruppe b1) oder d) bedeuten, und A¹ keinen Rest ausgewählt aus der Gruppe b1) oder d) bedeutet,
- A¹ und/oder A² und/oder A³ einen Rest ausgewählt aus der Gruppe d) bedeuten,
- die Reste der Gruppe d) ausgewählt sind aus Gruppe d1) bestehend aus 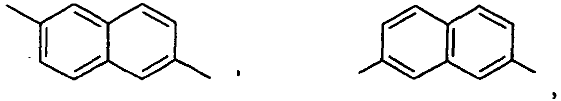 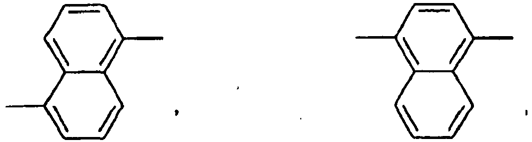 und 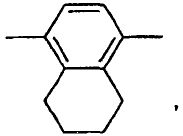 wobei in diesen Resten auch ein oder mehrere H-Atome durch L wie in Formel I definiert ersetzt sein können, wobei L besonders bevorzugt F, CN, SCN, SF₅, CH₂F, CHF₂, CF₃, OCH₂F, OCHF₂ oder OCF₃ bedeutet,
- die Reste der Gruppe d) ausgewählt sind aus Gruppe d2) bestehend aus 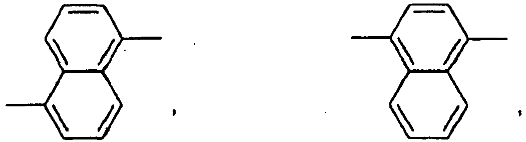 und 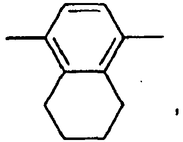 wobei in diesen Resten auch ein oder mehrere H-Atome durch L wie in Formel I definiert ersetzt sein können, wobei L besonders bevorzugt F, CN, SCN, SF₅, CH₂F, CHF₂, CF₃, OCH₂F, OCHF₂ oder OCF₃ bedeutet,
- die Reste P^{a} und P^{b} ausgewählt sind aus der Gruppe bestehend aus Vinyloxy-, Acrylat-, Methacrylat-, Fluoracrylat-, Chloracrylat-, Oxetan- und Epoxygruppen, besonders bevorzugt Acrylat- oder Methacrylatgruppen,
- die Reste Sp^{a} und Sp^{b} ausgewählt sind aus der Gruppe bestehend aus - (CH₂)ₚ₁-, -(CH₂)ₚ₁-O-, -(CH₂)ₚ₁-O-CO- und -(CH₂)ₚ₁-O-CO-O- und deren Spiegelbildern, worin p1 eine ganze Zahl von 1 bis 12, vorzugsweise von 1 bis 6, besonders bevorzugt 1, 2 oder 3 bedeutet, und wobei diese Reste so mit P^{a} oder P^{b} verknüpft sind, dass O-Atome nicht direkt benachbart sind,
- Q¹ -CF₂O- und Q² -OCF₂- bedeutet,
- Q¹ -OCF₂- und Q² -CF₂O- bedeutet,
- Q¹ und Q² -CF₂O- bedeuten,
- Q¹ und Q² -OCF₂- bedeuten,
- s1 und s2 jeweils 0 bedeuten,
- s1 und s2 jeweils 1 bedeuten,
- s1 1 und s2 0 bedeutet oder s1 0 und s2 1 bedeutet.

Von diesen neuen Verbindungen ganz besonders bevorzugt sind solche worin Q¹ -CF₂O- und Q² -OCF₂- bedeutet.

Von diesen neuen Verbindungen ganz besonders bevorzugt sind ferner solche ausgewählt aus einer oder mehreren der folgenden Gruppen:
der Gruppe bestehend aus den Formeln I9-I14,
der Gruppe bestehend aus den Formeln I19-I24,
der Gruppe bestehend aus den Formeln I33-I38,
der Gruppe bestehend aus den Formeln I43-I48,
der Gruppe bestehend aus den Formeln I57-I62 und
der Gruppe bestehend aus den Formeln I67-I72,
worin L eine der vor- und nachstehend angegebenen Bedeutungen besitzt und n eine ganze Zahl zwischen 1 und 24, bevorzugt zwischen 1 und 12, ganz besonders bevorzugt zwischen 2 und 8 ist.

Ein weiterer Gegenstand der Erfindung sind neue Zwischenprodukte zur Herstellung von Verbindungen der Formel I, ausgewählt aus Formel IA

G-O-(Sp^{a})ₛ₁-A²-Q¹-A¹-Q²-A³-(Sp^{b})ₛ₂-O-G' IA

worin Q¹, Q², A¹, A², A³, Sp^{a}, Sp^{b}, s1 und s2 die in Formel I oder deren Unterformeln angegebenen Bedeutungen, oder eine der vor- und nachstehend angegebenen bevorzugten Bedeutungen, besitzen, und G und G' jeweils unabhängig voneinander ein H-Atom oder eine Schutzgruppe bedeuten.

Geeignete Schutzgruppen G sind dem Fachmann bekannt. Bevorzugte Schutzgruppen sind Alkyl, Acryl und Alkylsilyl- oder Arylsilylgruppen, 2-Tetrahydropyranyl oder Methoxymethyl.

Die Verbindungen und Zwischenprodukte der Formel I und IA sowie deren Unterformeln können in Analogie zu dem Fachmann bekannten und in Standardwerken der organischen Chemie beschriebenen Verfahren, wie beispielsweise in Houben-Weyl, Methoden der organischen Chemie, Thieme-Verlag, Stuttgart, hergestellt werden.

Besonders geeignete und bevorzugte Verfahren zur Herstellung von Verbindungen und Zwischenprodukten der Formel I und IA sowie deren Unterformeln sind in folgenden Schemata beispielhaft dargestellt und enthalten vorzugsweise einen oder mehrere der nachfolgend beschriebenen Schritte.

Der Fachmann kann die Synthese in geeigneter Weise modifizieren und so zu weiteren erfindungsgemäßen Verbindungen gelangen. Die besonders bevorzugten Verbindungen mit einem Alkoxyspacer oder direkt an den Ring gebundenen Acrylaten werden beispielsweise durch Umsetzung von Dihydrochinonderivaten wie z.B. Verbindung **2** mit den Dithianyliumsalzen **3** erhalten. Die dabei zunächst gebildeten Verbindungen **4** werden zu den Verbindungen **5** umgesetzt. Anschließend können die Hydroxygruppen geeignet funktionalisiert werden, z.B. durch Veresterung mit Methacrylsäure (vgl. **Schema 1**).

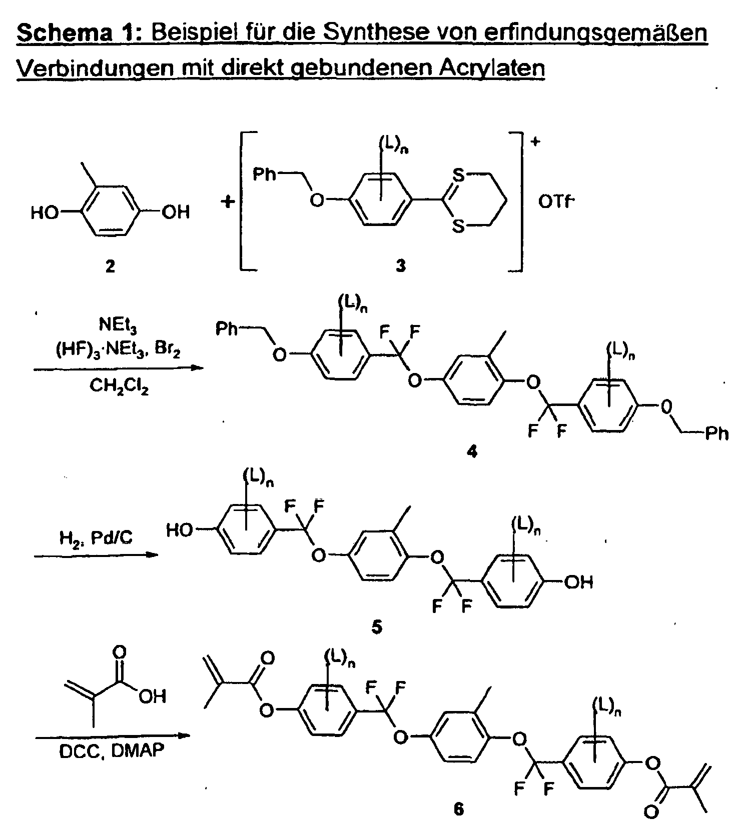

Sollen erfindungsgemäße Verbindungen mit Alkoxyspacern erhalten werden (z.B. Verbindung **8** in **Schema 2**), dann werden die Verbindungen **5** mit Bromalkanolen **6** in Gegenwart einer geeigneten Base umgesetzt. Anschließend kann dann wieder mit Acrylsäuren verestert werden.

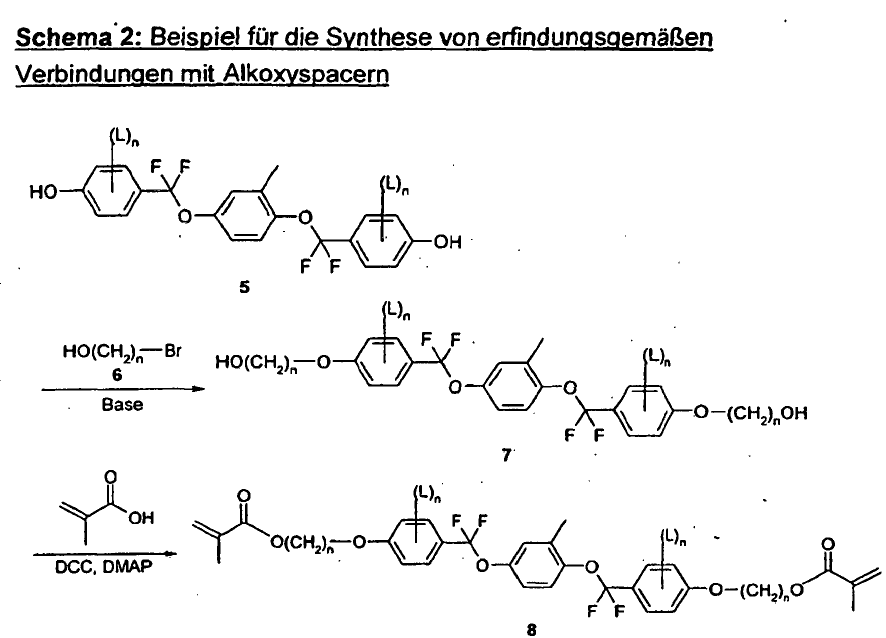

Alternativ kann die Einführung des Spacer auch im Sinne einer Mitsunobu-Reaktion erfolgen (vgl. **Schema 3**).

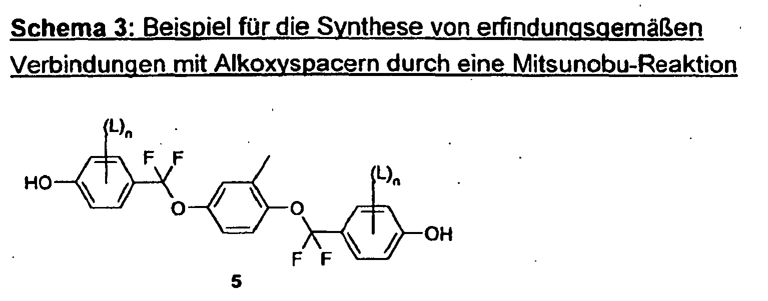

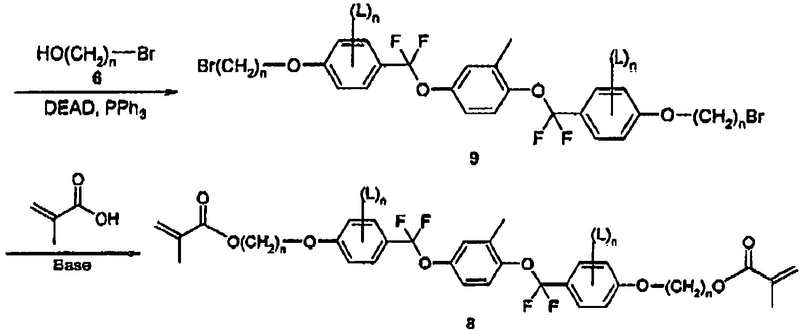

Zur Herstellung von PSA-Anzeigen werden die polymerisierbaren Verbindungen im FK-Medium zwischen den Substraten der FK-Anzeige unter Anlegen einer Spannung durch in-situ-Polymerisation polymerisiert oder vernetzt (falls eine Verbindung zwei oder mehr polymerisierbare Gruppen enthält). Die Polymerisation kann in einem Schritt durchgeführt werden. Es ist auch möglich, zunächst in einem ersten Schritt die Polymerisation unter Anlegen einer Spannung durchzuführen, um einen "pretilt"-Winkel zu erzeugen, und anschließend in einem zweiten Polymerisationsschritt ohne anliegende Spannung die im ersten Schritt nicht abreagierten Verbindungen zu polymerisieren bzw. zu vernetzen ("end curing").

Geeignete und bevorzugte Polymerisationsmethoden sind beispielsweise die thermische oder Photopolymerisation, vorzugsweise Photopolymerisation, insbesondere UV-Photopolymerisation. Dabei können gegebenenfalls auch ein oder mehrere Initiatoren zugesetzt werden. Geeignete Bedingungen für die Polymerisation, sowie geeignete Arten und Mengen der Initiatoren, sind dem Fachmann bekannt und in der Literatur beschrieben. Für die radikalische Polymerisation eignen sich zum Beispiel die kommerziell erhältlichen Photoinitiatoren Irgacure651®, Irgacure184®, Irgacure907®, Irgacure369®, oder Darocure1173® (Ciba AG). Falls ein Initiator eingesetzt wird, beträgt dessen Anteil vorzugsweise 0,001 bis 5 Gew.-%, besonders bevorzugt 0,001 bis 1 Gew.-%.

Die erfindungsgemäßen polymerisierbaren Verbindungen eignen sich auch für die Polymerisation ohne Initiator, was erhebliche Vorteile mit sich bringt, wie beispielsweise geringere Materialkosten und insbesondere eine geringere Verunreinigung des FK-Mediums durch mögliche Restmengen des Initiators oder dessen Abbauprodukte. Die Polymerisation kann somit auch ohne Zusatz eines Initiators erfolgen. Somit enthält das FK-Medium in einer bevorzugten Ausführungsform keinen Polymerisationsinitiator.

Die polymerisierbare Komponente oder das FK-Medium können auch einen oder mehrere Stabilisatoren enthalten, um eine unerwünschte spontane Polymerisation der RMs, beispielsweise während der Lagerung oder des Transports, zu verhindern. Geeignete Arten und Mengen der Stabilisatoren sind dem Fachmann bekannt und in der Literatur beschrieben. Besonders geeignet sind zum Beispiel die kommerziell erhältlichen Stabilisatoren der Serie Irganox® (Ciba AG), wie beispielsweise Irganox® 1076. Falls Stabilisatoren eingesetzt werden, beträgt deren Anteil, bezogen auf die Gesamtmenge der RMs beziehungsweise der polymerisierbaren Komponente, vorzugsweise 10 - 10,000 ppm, besonders bevorzugt 50 - 500 ppm.

Die erfindungsgemäßen polymerisierbaren Verbindungen können einzeln polymerisiert werden, es können aber auch Mischungen polymerisiert werden, welche zwei oder mehr erfindungsgemäße polymerisierbare Verbindungen enthalten, oder Mischungen enthaltend eine oder mehrere erfindungsgemäße polymerisierbare Verbindungen und eine oder mehrere weitere polymerisierbare Verbindungen (Comonomere), welche vorzugsweise mesogen oder flüssigkristallin sind. Bei Polymerisation solcher Mischungen entstehen Copolymere. Die vor- und nachstehend genannten polymerisierbaren Mischungen sind ein weiterer Gegenstand der Erfindung. Die polymerisierbaren Verbindungen und Comonomere sind mesogen oder nicht-mesogen, vorzugsweise mesogen oder flüssigkristallin.

Geeignete und bevorzugte Comonomere für die Verwendung in erfindungsgemäßen Anzeigen sind beispielsweise ausgewählt aus den folgenden Formeln: 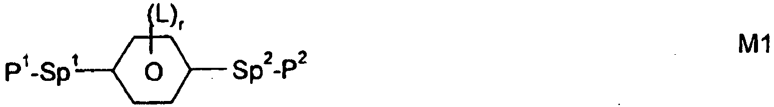 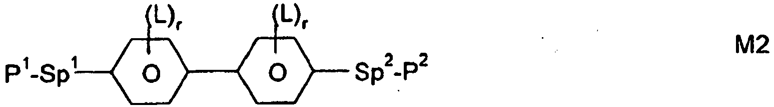 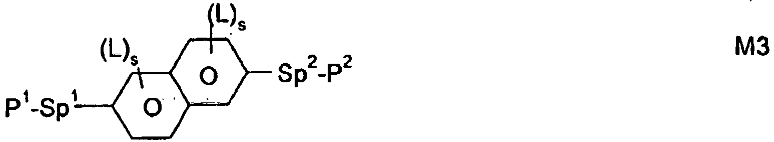 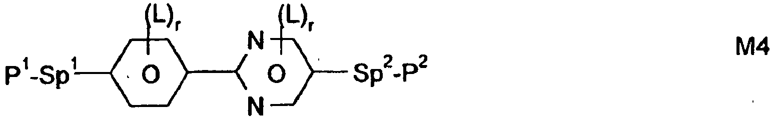 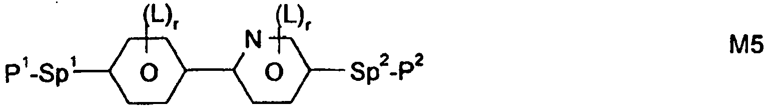 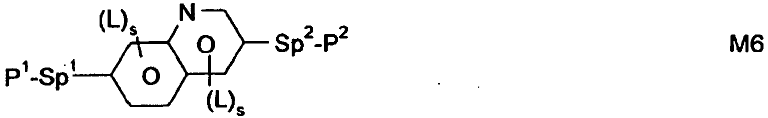 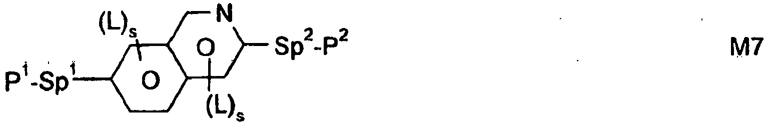 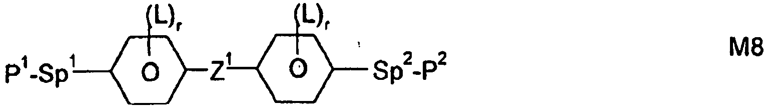 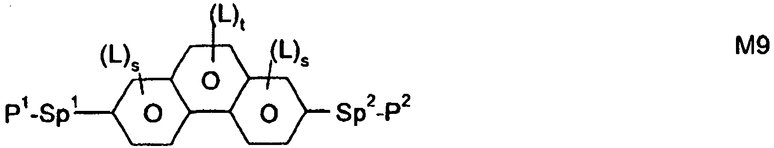 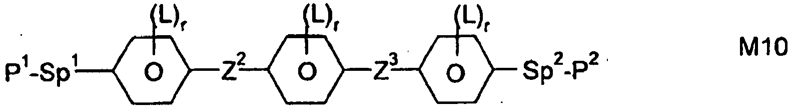 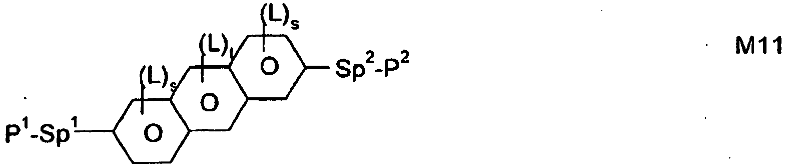 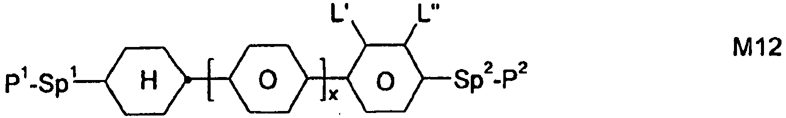 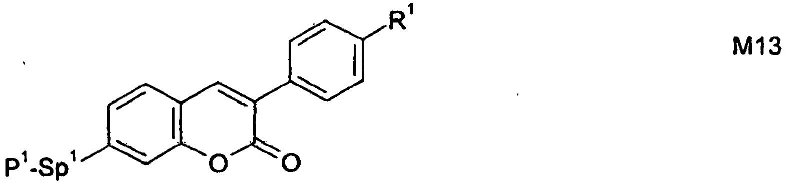 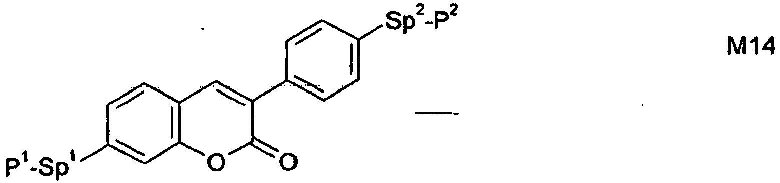 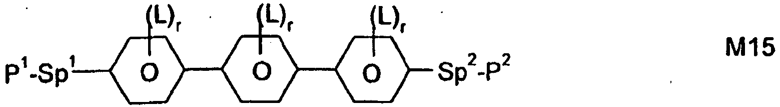 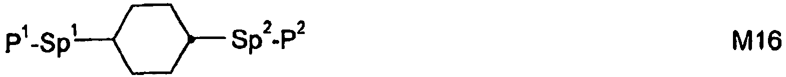 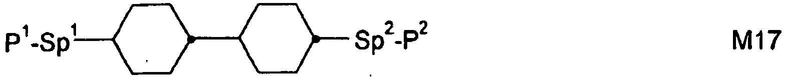 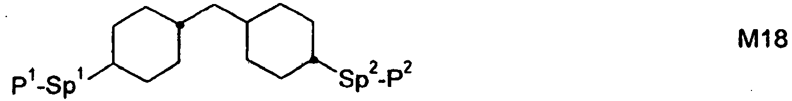 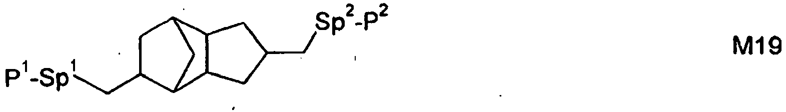 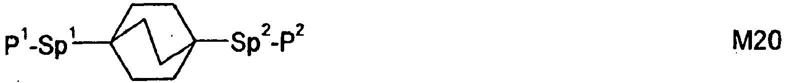 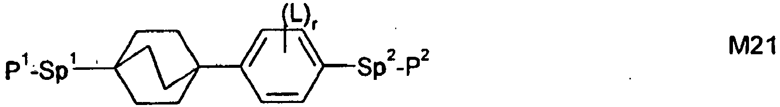 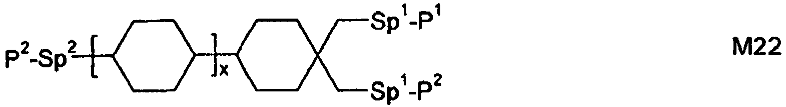 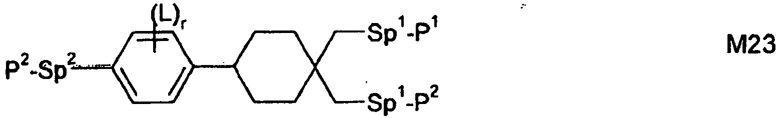 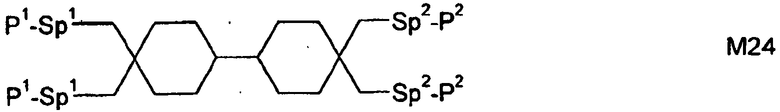 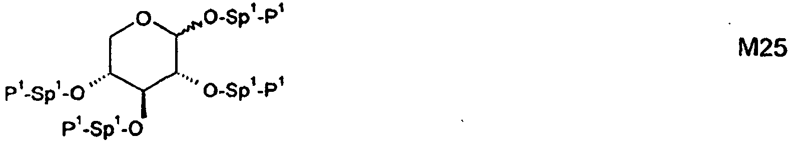 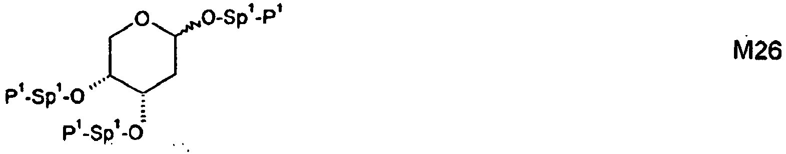 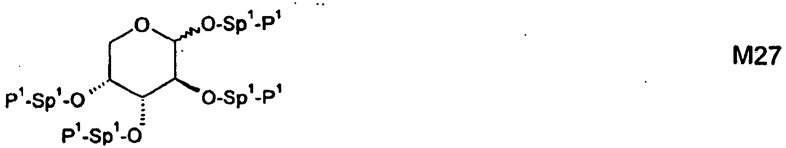 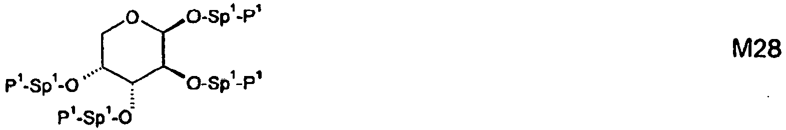 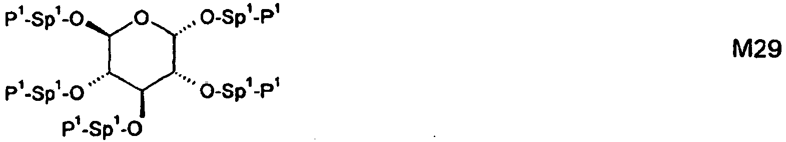 worin die einzelnen Reste folgende Bedeutung besitzen:
- P¹ und P²: jeweils unabhängig voneinander eine polymerisierbare Gruppe, vorzugsweise mit einer der vor- und nachstehend für P angegebenen Bedeutungen, besonders bevorzugt eine Acrylat-, Methacrylat-, Fluoracrylat-, Oxetan-, Vinyloxy- oder Epoxygruppe,
- Sp¹ und Sp²: jeweils unabhängig voneinander eine Einfachbindung oder eine Abstandsgruppe, vorzugsweise mit einer der vor- und nachstehend für Sp^{a} angegebenen Bedeutungen, und besonders bevorzugt -(CH₂)ₚ₁-, -(CH₂)ₚ₁-O-, -(CH₂)ₚ₁-CO-O- oder -(CH₂)ₚ₁-O-CO-O-, worin p1 eine ganze Zahl von 1 bis 12 ist, und wobei in den letztgenannten Gruppen die Verknüpfung zur benachbarten Ring über das O-Atom erfolgt,
wobei auch einer oder mehrere der Reste P¹-Sp¹- und P²-Sp²- einen Rest R^{aa} bedeuten können, mit der Maßgabe dass mindestens einer der vorhandenen Reste P¹-Sp¹- und P²-Sp²- nicht R^{aa} bedeutet,
- R^{aa}: H, F, CI, CN oder geradkettiges oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -N(R⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, CI, CN oder P¹-Sp¹- ersetzt sein können, besonders bevorzugt geradkettiges oder verzweigtes, optional ein- oder mehrfach fluoriertes, Alkyl, Alkoxy, Alkenyl, Alkinyl, Alkylcarbonyl, Alkoxycarbonyl, oder Alkylcarbonyloxy mit 1 bis 12 C-Atomen (wobei die Alkenyl- und Alkinylreste mindestens zwei und die verzweigten Reste mindestens drei C-Atome aufweisen),
- R⁰, R⁰⁰: jeweils unabhängig voneinander und bei jedem Auftreten gleich oder verschieden H oder Alkyl mit 1 bis 12 C-Atomen,
- R^{y} und R^{z}: jeweils unabhängig voneinander H, F, CH₃ oder CF₃,
- Z¹: -O-, -CO-, -C(R^{y}R^{z})-,oder -CF₂CF₂-,
- Z² und Z³: jeweils unabhängig voneinander -CO-O-, -O-CO-, -CH₂O-, -OCH₂-, -CF₂O-, -OCF₂-, oder -(CH₂)ₙ-, wobei n 2, 3 oder 4 ist,
- L: bei jedem Auftreten gleich oder verschieden F, CI, CN, SCN, SF₅ oder geradkettiges oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen vorzugsweise F,
- L' und L": jeweils unabhängig voneinander H, F oder CI,
- r: 0, 1, 2, 3 oder 4,
- s: 0, 1, 2 oder 3,
- t: 0, 1 oder 2,
- x: 0 oder 1.

Vorzugsweise enthält das FK-Medium oder die polymerisierbare Komponente zusätzlich zu den Verbindungen der Formel 1 oder IA eine oder mehrere Verbindungen ausgewählt aus der Gruppe bestehend aus den Formeln M16-M29, besonders bevorzugt bestehend aus den Formeln M16-M21, ganz besonders bevorzugt bestehend aus den Formeln M16, M17 und M18.

Vorzugsweise enthält das FK-Medium oder die polymerisierbare Komponente keine Verbindungen der Formel M10, worin Z² und Z³ -COO- oder -OCO- bedeuten.

Die FK-Medien zur Verwendung in den erfindungsgemäßen FK-Anzeigen enthalten, neben den oben beschriebenen polymerisierbaren Verbindungen, eine FK-Mischung ("Host-Mischung") enthaltend eine oder mehr, vorzugsweise zwei oder mehr niedermolekulare (d.h. monomere bzw. unpolymerisierte) Verbindungen. Letztere sind stabil bzw. unreaktiv gegenüber einer Polymerisationsreaktion unter den zur Polymerisation der polymerisierbaren Verbindungen verwendeten Bedingungen. Prinzipiell eignet sich als Host-Mischung jede zur Verwendung in herkömmlichen VA- und OCB-Anzeigen geeignete FK-Mischung.

Geeignete FK-Mischungen sind dem Fachmann bekannt und in der Literatur beschrieben. FK-Medien für VA-Anzeigen sind in EP 1 378 557 A1, FK-Medien für OCB-Anzeigen sind in EP 1 306 418 A1 und DE 102 24 046 A1 beschrieben. FK-Medien für FK-Anzeigen mit blauer Phase sind in WO 2006/063662 A1 sowie den darin zitierten Dokumenten beschrieben.

Besonders bevorzugte FK-Medien zur Verwendung in FK-Anzeigen mit blauer Phase werden im Folgenden beschrieben:
Vorzugsweise enthält ein erfindungsgemäßes FK-Medium mit blauer Phase
   - eine polymerisierbare Komponente A, vorzugsweise in einer Konzentration von 1 bis 25%, besonders bevorzugt von 2 bis 20%, ganz besonders bevorzugt von 3 bis 15%, enthaltend, vorzugsweise hauptsächlich bestehend aus, ganz besonders bevorzugt ausschließlich bestehend aus, einer oder mehrerer Verbindungen der Formel I und optional einer oder mehrerer zusätzlicher polymerisierbarer Verbindungen, und
   - eine flüssigkristalline Komponente B, enthaltend eine oder mehrere, vorzugsweise zwei oder mehr niedermolekulare (d.h. monomere und unpolymerisierbare) Verbindungen, vorzugsweise in einer Konzentration von 20-100%, vorzugsweise mit einer positiven dielektrischen Anisotropie, vorzugsweise hauptsächlich bestehend aus, ganz besonders bevorzugt ausschließlich bestehend aus, einer oder mehrerer Verbindungen der Formel II 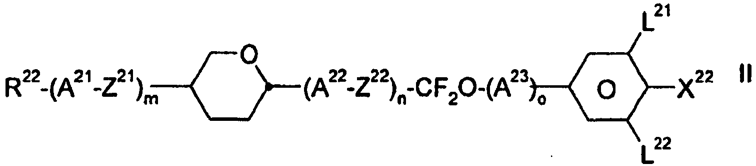 worin die einzelnen Reste folgende Bedeutung besitzen
      - R²²: H, F, CI, CN, NCS, SF₅ , SO₂CF₃ oder geradkettiges oder verzweigtes Alkyl mit 1 bis 20 C-Atomen, welches unsubstituiert, oder durch F, CI oder CN ein- oder mehrfach substituiert ist, und worin auch eine oder mehrere nicht benachbarte CH₂-Gruppen auch jeweils unabhängig voneinander durch -O-, -S-, -NH-, -NR⁰¹-, -SiR⁰¹R⁰²-, -CO-, -COO-, -OCO-, -OCO-O-, -S-CO-, -CO-S-, -CY⁰¹=CY⁰²- oder -C≡C- so ersetzt sein können, dass O- und/oder S-Atome nicht direkt miteinander verknüpft sind,
      - Y⁰¹, Y⁰²: jeweils unabhängig voneinander F, Cl oder CN, einder der Reste Y⁰¹ und Y⁰² auch H,
      - R⁰¹, R⁰²: jeweils unabhängig voneinander H oder Alkyl mit 1 bis 12 C-Atomen,
      - A²¹, A²², A²³: jeweils unabhängig voneinander und bei jedem Auftreten gleich oder verschieden 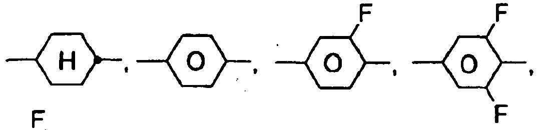 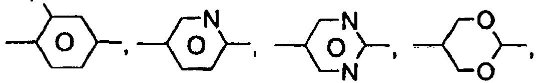
      - Z²¹, Z²²: jeweils unabhängig voneinander, und bei jedem Auftreten gleich oder verschieden, eine Einfachbindung, -(CH₂)₄-, -CH₂CH₂-, -CF₂-CF₂-, -CF₂-CH₂-, -CH₂-CF₂-, -CH=CH-, - CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- oder -O-CO-,
      - X²²: F, CI, -CN, -NCS, -SF₅, -SO₂CF₃, oder Alkyl, Alkenyl, Alkenyloxy, Alkylalkoxy oder Alkoxy mit 1 bis 3 C-Atomen, welches durch F, CI oder CN ein- oder mehrfach substituiert ist,
      - L²¹, L²²: jeweils unabhängig voneinander H oder F,
      - m: 0, 1 oder 2,
      - n: 0, 1, 2 oder 3,
      - o: 0, 1 oder 2, wobei
      m + n + o 0, 1, 2 oder 3, vorzugsweise 0, 1 oder 2 bedeutet,
   - optional eine flüssigkristalline Komponente C, enthaltend eine oder mehrere, vorzugsweise zwei oder mehr niedermolekulare (d.h. monomere und unpolymerisierbare) Verbindungen, vorzugsweise in einer Konzentration von 1 bis 25 %, vorzugsweise hauptsächlich bestehend aus, ganz besonders bevorzugt ausschließlich bestehend aus, einer oder mehrerer Verbindungen der Formel III 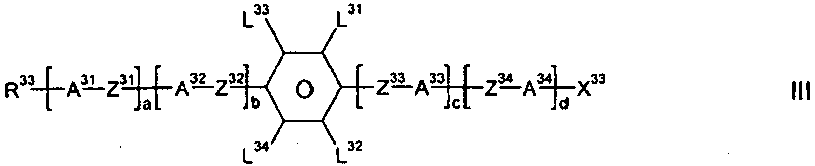 worin
      - a, b, c, d: jeweils unabhängig voneinander 0, 1 oder 2, wobei
      - a + b + c + d: 0, 1, 2, 3 oder 4 ist,
      - A³¹, A³², A³³, A³⁴: jeweils unabhängig voneinander, und bei jedem Auftreten gleich oder verschieden, 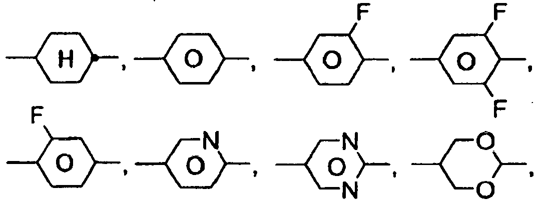
      - Z³¹, Z³², Z³³, Z³⁴: jeweils unabhängig voneinander, und bei jedem Auftreten gleich oder verschieden, eine Einfachbindung, -(CH₂)₄)-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- oder -O-CO-,
      - R³³: Alkyl oder Alkoxy mit 1 bis 15 C-Atomen, welches unsubstituiert, oder durch F, CI oder CN ein- oder mehrfach substituiert ist, und worin auch eine oder mehrere nicht benachbarte CH₂-Gruppen auch jeweils unabhängig voneinander durch -O-, -S-, -SiR^{x}R^{y}-, -
      - CH=CH-, -C≡C-,: -CO-O- und/oder -O-CO- so ersetzt sein können, dass O- und/oder S-Atome nicht direkt miteinander verknüpft sind, vorzugsweise ein geradkettiger Alkyl-, Alkoxy-, Alkenyl-, Alkenyloxy- oder -O-Alkylen-O-Rest mit bis zu 10 C-Artomen, welcher unsubstituiert oder ein- oder mehrfach durch F oder CI substituiert ist,
      - L³¹, L³², L³³, L³⁴: jeweils unabhängig voneinander H, F, Cl, CN, oder Alkyl oder Alkoxy mit 1 bis 15 C-Atomen, welches unsubstituiert, oder durch F, CI oder CN ein- oder mehrfach substituiert ist, und worin auch eine oder mehrere nicht benachbarte CH₂-Gruppen auch jeweils unabhängig voneinander durch -O-, -S-, -SiR^{x}R^{y}-, - CH=CH-, -C≡C-, -CO-O- und/oder -O-CO- so ersetzt sein können, dass O- und/oder S-Atome nicht direkt miteinander verknüpft sind, mit der Massgabe dass mindestens einer der Reste L³¹, L³², L³³ und L³⁴ von H verschieden ist,
      - X³³: F, Cl, CF₃, OCF₃, CN, NCS, -SF₅ oder -SO₂-R^{z},
      - R^{x} und R^{y}: jeweils unabhängig voneinander H, Alkyl oder Alkoxy mit 1 bis 7 C-Atomen, vorzugsweise Methyl, Ethyl, Propyl oder Butyl, und
      - R^{z}: Alkyl mit 1 bis 7 C-Atomen, welches unsubstituiert, oder durch F oder CI ein- oder mehrfach substituiert ist, vorzugsweise CF₃, C₂F₅ oder n-C₄F₉,
   - eine Komponente D, vorzugsweise in einer Konzentration von 1-20%, enthaltend eine oder mehrere optisch aktive und/oder chirale Verbindungen, vorzugsweise mit einer HTP ≥ 20 µm⁻¹, vorzugsweise ≥ 40 µm⁻¹, ganz besonders bevorzugt ≥ 60 µm⁻¹.

Die chirale Komponente D enthält vorzugsweise eine oder mehrere chirale Verbindungen mit mesogener Struktur und weist vorzugsweise eine oder mehrere Mesophasen auf, besonders bevorzugt mindestens cholesterische Phase. Bevorzugte chirale Verbindungen der Komponente D sind beispielsweise aus dem Stand der Technik bekannte und/oder kommerziell erhältliche chirale Dotierstoffe wie Cholesteryl-nonanoat (CN), R/S-811, R/S-1011, R/S-2011, R/S-3011, R/S-4011, R/S-5011 oder CB-15 (Merck KGaA, Darmstadt). Besonders bevorzugt sind chirale Dotierstoffe mit einer oder mehreren chiralen Gruppen und einer oder mehreren mesogenen Gruppen, wie beispielsweise in DE 34 25 503, DE 35 34 777, DE 35 34 778, DE 35 34 779, DE 35 34 780, DE 43 42 280, EP 01 038 941 und DE 195 41 820 offenbart. Ferner bevorzugt sind Sorbitole wie z.B. in WO 98/00428 A1 beschrieben, Hydrobenzoine wie z.B. in GB 2 328 207 A beschrieben, chirale Binaphthole wie z.B. WO 02/94805 A1 beschrieben, chirale Binaphtholacetale wie z.B. in WO 02/34739 A1 beschrieben, chirale TADDOLe wie z.B. in WO 02/06265 A1 beschrieben, oder chirale Verbindungen mit fluorierten Brückengruppen wie z.B. in WO 02/06196 A1 oder WO 02/06195 A1 beschrieben.

Der Klärpunkt des erfindungsgemäßen FK-Mediums mit blauer Phase liegt vorzugsweise im Bereich von -30 °C bis 100 °C.

Das FK-Medium enthält vorzugsweise eine, zwei, drei, vier oder mehr als vier chirale Verbindungen. Das FK-Medium enthält vorzugsweise chirale Verbindungen in einer Gesamtkonzentration von 0.01 bis 25%, bevorzugt 0.1-20%, besonders bevorzugt 0.5 bis 20%, ganz besonders 3-15%.

Der Anteil der Verbindungen der Formel I am Gesamtgehalt aller polymerisierbaren Verbindungen im FK-Medium, bzw. der Anteil der Verbindungen der Formel I an der polymerisierbaren Komponente A), beträgt vorzugsweise 20 bis 80%, besonders bevorzugt 40 bis 60%.

In einer weiteren bevorzugten Ausführungsform beträgt der Anteil der Verbindungen der Formel I am Gesamtgehalt aller polymerisierbaren Verbindungen im FK-Medium, bzw. der Anteil der Verbindungen der Formel I an der polymerisierbaren Komponente A), mindestens 50%, und besonders bevorzugt 60% bis 100%,.

Weitere besonders bevorzugte Ausführungsformen sind nachfolgend beschrieben:
- Das FK-Medium enthält eine, zwei oder drei Verbindungen der Formel I;
- Komponente B enthält zusätzlich zu den Verbindungen der Formel II eine oder mehrere Esterverbindungen der Formel Z: 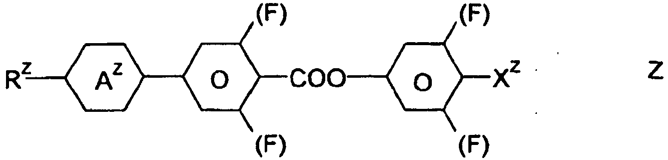 worin R^{z} eine der für R²² in Formel II angegebenen Bedeutungen hat, 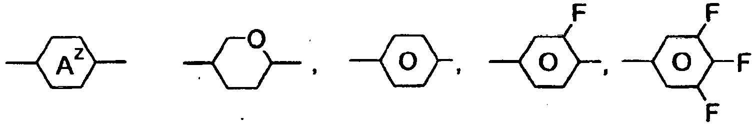 X^{z} F, CI, CN, NCS, OCF₃, CF₃ or SF₅ bedeutet, und (F) F oder H bedeutet,
   vorzugsweise in einer Konzentration von 5 bis 35%, besonders bevorzugt 10 bis 30%, ganz besonders bevorzugt von 10 bis 20%.
- Komponente B enthält zusätzlich zu den Verbindungen der Formel II eine oder mehrere Verbindungen der Formel N: 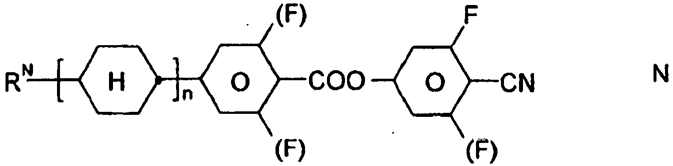 worin R^{N} eine der für R²² in Formel II angegebenen Bedeutungen hat und vorzugsweise Alkyl or Alkyl-C≡C bedeutet, "Alkyl" Alkyl mit 1 bis 7 C-Atomen, welches vorzugsweise geradkettig ist, (F) F oder H, und n 0 oder 1 bedeutet.
- Komponente B enthält zusätzlich zu den Verbindungen der Formel II eine oder mehrere Verbindungen der Formel E: 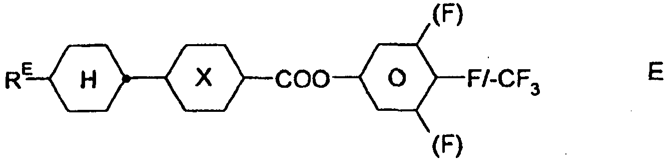 worin R^{E} eine der für R²² in Formel II angegebenen Bedeutungen hat und vorzugsweise C1-C7-Alkyl bedeutet, und 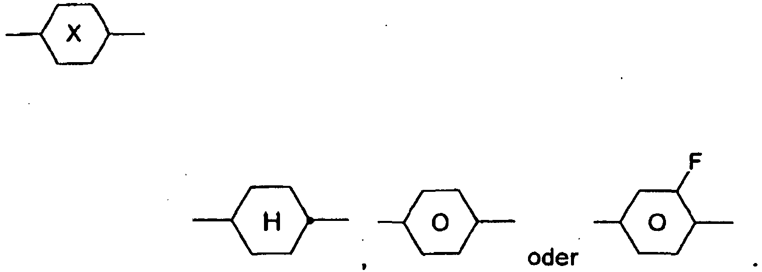 bedeutet, vorzugsweise in einer Konzentration von 10-30%, besonders bevorzugt von 15-25%.
- Das FK-Medium enthält zusätzlich eine oder mehrere Verbindungen der Formel Q1 und/oder Q2: 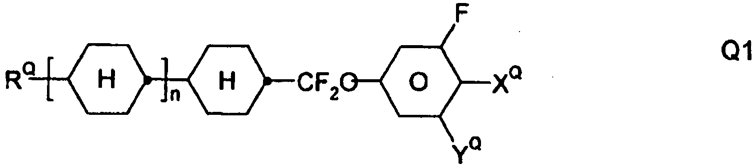 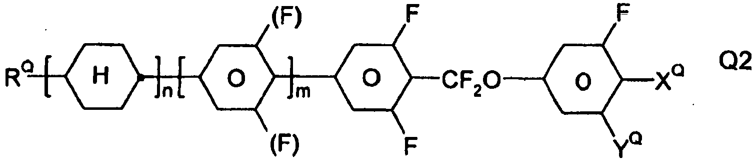 worin R^{Q} eine der für R²² in Formel II angegebenen Bedeutungen hat, X^{Q} eine der für X^{Z} in Formel Z angegebenen Bedeutungen hat, oder F bedeutet n und m jeweils unabhängig voneinander 0 oder 1 bedeuten und Y^{Q}.
- Das FK-Medium enthält zusätzlich eine oder mehrere Verbindungen der Formel Dx1 und/oder Dx2: 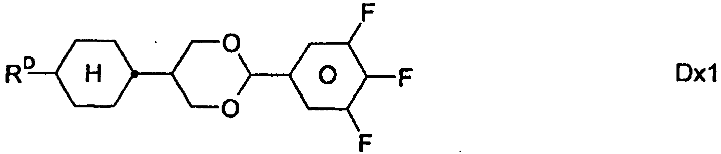 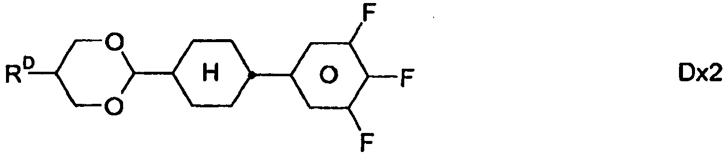 worin R^{D} eine der für R²² in Formel II angegebenen Bedeutungen hat.

Besonders bevorzugt sind FK-Medien, welche neben einer oder mehreren Verbindungen der Formel I eine oder mehrere Verbindungen der Formel II enthalten, insbesondere worin X²² F, Cl, CN, NCS, CF₃ oder OCF₃ bedeutet. Die Verbindungen der Formeln I, II, III, Z, N, E, Q1, Q2, Dx1 und Dx2 sind farblos, stabil und gut untereinander oder mit anderen flüssigkristallinen Substanzen mischbar.

Die einzelnen Komponenten und Verbindungen der Formeln I, II, III, Z, N, E, Q1, Q2, Dx1 und Dx2 der erfindungsgemäßen FK-Medien sind entweder bekannt, oder ihre Herstellungsweisen sind für den einschlägigen Fachmann aus dem Stand der Technik ohne weiteres abzuleiten, da sie auf in der Literatur beschriebenen Standardverfahren basieren.

Die Herstellung der erfindungsgemäßen FK-Medien erfolgt in an sich üblicher Weise, beispielsweise indem man eine oder mehrere der oben genannten Verbindungen mit einer oder mehreren polymerisierbaren Verbindungen wie oben definiert, und ggf. mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Das Verfahren zur Herstellung der erfindungsgemäßen FK-Medien ist ein weiterer Gegenstand der Erfindung.

Die erfindungsgemäßen FK-Medien für FK-Anzeigen mit blauer Phase können auch weitere, dem Fachmann bekannte und in der Literatur beschriebene Zusätze oder Additive enthalten, wie beispielsweise Polymerisationsinitiatoren, Inhibitoren, Stabilisatoren oder oberflächenaktive Substanzen. Diese können polymerisierbar oder unpolymerisierbar sein. Polymerisierbare Additive werden dementsprechend der polymerisierbaren Komponente zugerechnet.

Unpolymerisierbare Additive werden dementsprechend der flüssigkristallinen Komponente zugerechnet.

Der Aufbau der erfindungsgemäßen FK-Anzeigen mit blauer Phase mit Polarisatoren, Elektrodensubstraten und oberflächenbehandelten Elektrodenschichten entspricht dem konventionellen und dem Fachmann bekannten Aufbau für Anzeigen dieses Typs, wie im Stand der Technik beschrieben, beispielsweise in DE 102 17 273 A1, DE 102 41 301, DE 102 17 273 A1, DE 102 41 301, DE 102 536 06, DE 103 13 979.

Eine erfindungsgemäße FK-Anzeige enthält vorzugsweise folgende Komponenten
- ein oder zwei Substrate,
- eine Elektrodenanordnung mit zwei Elektroden auf nur einem der beiden Substrate oder mit jeweils einer Elektrode auf je einem der beiden Substrate,
- ein oder zwei Polarisatoren, und
- einer zwischen den beiden Substraten befindlichen Schicht eines erfindungsgemäßen FK-Mediums,
wobei die Anzeige bei einer Temperatur betrieben wird, bei der das FK-Medium im nicht-geschalteten Zustand eine optisch isotrope Phase, vorzugsweise eine blaue Phase aufweist.

Der Phasenübergang des FK-Mediums in die blaue Phase erfolgt üblicherweise ausgehend von einer bei tieferen Temperaturen als die blaue Phase existierenden cholesterischen Phase. Die Betriebstemperatur der erfindungsgemäßen FK-Anzeige (d.h. nach Polymerstabilisierung) liegt vorzugsweise über der Temperatur des Phasenübergangs des FK-Mediums in die blaue Phase (d.h. üblicherweise der Übergang cholesterische Phase - blaue Phase), besonders bevorzugt von 0,1 bis 50°, ganz besonders bevorzugt von 0, bis 40° über dieser Phasenübergangstemperatur. Weiterhin liegt die Betriebstemperatur der FK-Anzeige vorzugsweise unterhalb der Temperatur des Phasenübergangs des FK-Mediums von der blauen Phase in die isotrope Phase (auch als Klärpunkt bezeichnet). Die Anzeige kann jedoch auch in der isotropen Phase, d.h. oberhalb des Klärpunkts, betrieben werden.

Die erfindungsgemäßen FK-Medien mit blauer Phase können zusätzlich zu den obengenannten Verbindungen der Formeln II und III, und zusätzlich oder alternativ zu den obengenannten Verbindungen der Formeln Z, N, E, Q1, Q2, Dx1 und Dx2, auch weitere flüssigkristalline Verbindungen enthalten, um z.B. die physikalischen Eigenschaften anzupassen. Solche Verbindungen sind dem Fachmann bekannt. Ihre Konzentration in den FK-Medien ist vorzugsweise 0 bis 30%, besonders bevorzugt 0 bis 20%, ganz besonders bevorzugt 5 bis 15 %.

Vorzugsweise weist die flüssigkristalline Komponente eines erfindungsgemäßen FK-Mediums (d.h. vor Polymerstabilisierung) einen Temperaturbereich der blauen Phase auf, bzw. beim Auftreten mehrerer sequentieller blauer Phasen einen kombinierten Temperaturbereich aller blauen Phasen, dessen gesamte Breite 2°C oder mehr, bevorzugt 5°C oder mehr, besonders bevorzugt 10°C oder mehr, ganz besonders bevorzugt 20°C oder mehr, beträgt.

Vorzugsweise zeigt die flüssigkristalline Komponente eines erfindungsgemäßen FK-Mediums (d.h. vor Polymerstabilisierung) einen Temperaturbereich der blauen Phase(n) mindestens im Bereich von 10°C bis 30°C, besonders bevorzugt von 10°C bis 40°C, ganz besonders bevorzugt von 0°C bis 50°C.

Dabei bedeutet "mindestens" vor- und nachstehend, dass sich die blaue(n) Phase(n) auch unterhalb des jeweils angegebenen unteren Grenzwertes und/oder oberhalb des jeweils angegebenen oberen Grenzwertes erstrecken können.

In einer weiteren bevorzugten Ausführungsform zeigt die flüssigkristalline Komponente eines erfindungsgemäßen FK-Mediums (d.h. vor Polymerstabilisierung) einen Temperaturbereich der blauen Phase(n) mindestens im Bereich von 20°C bis 40°C, besonders bevorzugt mindestens von 30°C bis 80°C, ganz besonders bevorzugt mindestens von 30°C bis 90°C. Ferner bevorzugt sind FK-Medien mit einem Temperaturbereich der blauen Phase(n) mindestens von -20°C bis 50°C.

Vorzugsweise zeigt ein erfindungsgemäßes FK-Medium mit der polymerisierten Komponente (d.h. nach Polymerstabilisierung) einen Temperaturbereich der blauen Phase(n) mindestens im Bereich von 30°C bis 70°C, bevorzugt von 20°C bis 70°C, besonders bevorzugt von 0°C bis 80°C, ganz besonders bevorzugt von -20°C bis 80°C.

Die Phasenübergangstemperaturen eines erfindungsgemäßen FK-Mediums enthaltend die polymerisierbare Komponente, insbesondere der Klärpunkt und/oder die Temperatur des Übergangs von der cholesterischen Phase in die blaue Phase (T(Ch,BP), auch als T(N*,BP) bezeichnet) und/oder die Temperatur des Übergangs von der blauen Phase in die isotrope Phase (T(BP,I)), werden vorzugsweise durch die Polymerisation der polymerisierbaren Komponente nicht verringert. Das bedeutet, dass die Polymerstabilisierung die blaue(n) Phase(n) vorzugsweise so erfolgt, dass eine oder mehrere der oben angegebenen Phasenübergangstemperaturen (T(Ch,BP), T(BP,I)) nicht zu tieferen Temperaturen verschoben werden, d.h. die blaue(n) Phase(n) wird vorzugsweise mindestens zu tieferen Temperaturen hin, und besonders bevorzugt sowohl zu tieferen als auch zu höheren Temperaturen hin verbreitert.

Geeignete FK-Medien zur Verwendung in PSA-Anzeigen, insbesondere in PSA-VA-, PSA-OCB-, PSA-IPS-, PSA-FFS- oder PSA-TN-Anzeigen, werden im Folgenden beschrieben.

Vorzugsweise enthält ein FK-Medium zur Verwendung In PSA-VA-, PSA-OCB-, PSA-IPS-, PSA-FFS- oder PSA-TN-Anzeigen:
- < 5%, besonders bevorzugt < 1 %, ganz besonders bevorzugt < 0.5 % der polymerisierbaren Komponente,
- > 95%, besonders bevorzugt > 99% der flüssigkristallinen Komponente,
- < 5 Gew.-%, besonders bevorzugt < 1 Gew.-%, ganz besonders bevorzugt < 0.5 Gew.-% an polymerisierbaren Verbindungen, insbesondere polymerisierbaren Verbindungen der oben genannten Formeln I oder deren Unterformeln,
- eine, zwei oder drei erfindungsgemäße polymerisierbare Verbindungen der Formel I oder deren Unterformeln,
- eine polymerisierbare Komponente, die ausschließlich erfindungsgemäße polymerisierbare Verbindungen der Formel I oder deren Unterformeln enthält,
- eine flüssigkristalline Komponente, die eine FK-Verbindung oder eine FK-Mischung ist, die eine nematische Flüssigkristallphase aufweist,
- eine polymerisierbare und/oder flüssigkristalline Komponente, die ausschließlich achirale Verbindungen enthält,
- eine polymerisierbare Komponente, die eine oder mehrere polymerisierbare Verbindungen mit einer polymerisierbaren Gruppe (monoreaktiv) und eine oder mehrere erfindungsgemäße polymerisierbare Verbindungen mit zwei oder mehr, vorzugsweise zwei polymerisierbaren Gruppen (di- oder multireaktiv) enthält, vorzugsweise ausgewählt aus Verbindungen der Formel I oder deren Unterformeln, und optional aus den oben genanten Comonomeren ausgewählt aus der Liste enthaltend die Formeln M1-M29,
- eine polymerisierbare Komponente, die ausschließlich erfindungsgemäße polymerisierbare Verbindungen mit zwei polymerisierbaren Gruppen (direaktiv) enthält, vorzugsweise ausgewählt aus Verbindungen der Formel I oder deren Unterformeln, und optional zusätzlich aus den oben genanten Comonomeren der Liste enthaltend die Formeln M1-M29,
- außer den erfindungsgemäßen polymerisierbaren Verbindungen, insbesondere der Formel I oder deren Unterformeln, sowie den Comonomeren, keine Verbindungen, die eine endständige Vinyloxygruppe (-O-CH=CH₂) aufweisen,
- 1 bis 5, vorzugsweise 1, 2 oder 3 polymerisierbare Verbindungen, vorzugsweise ausgewählt aus erfindungsgemäßen polymerisierbare Verbindungen, insbesondere der Formel I oder deren Unterformeln.

Geeignete FK-Medien zur Verwendung In PSA-VA-Anzeigen werden im Folgenden genannt:
a) FK-Medium, welches eine oder mehrere Verbindungen der Formel CY und/oder PY enthält: 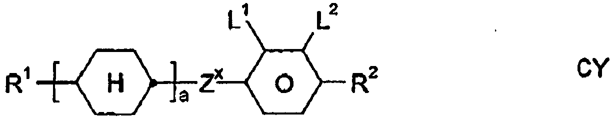 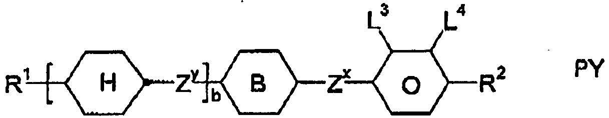 worin die einzelnen Reste folgende Bedeutung besitzen
   - a: 1 oder 2,
   - b: 0 oder 1, 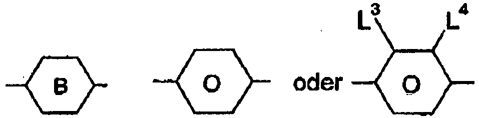
   - R¹ und R²: jeweils unabhängig voneinander Alkyl mit 1 bis 12 C-Atomen, wobei auch eine oder zwei nicht benachbarte CH₂-Gruppen durch -O-, -CH=CH-, -CO-, -OCO- oder - COO- so ersetzt sein können, dass O-Atome nicht direkt miteinander verknüpft sind, vorzugsweise Alkyl oder Alkoxy mit 1 bis 6 C-Atomen,
   - Z^{x} und Z^{y}: jeweils unabhängig voneinander -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung,
   - L¹⁻⁴: jeweils unabhängig voneinander F, Cl, OCF₃, CF₃, CH₃, CH₂F, CHF₂.

Vorzugsweise bedeuten beide Reste L¹ und L² F, oder einer der Reste L¹ und L² F und der andere Cl, bzw. beide Reste L³ und L⁴ F, oder einer der Reste L³ und L⁴ F und der andere Cl.

Die Verbindungen der Formel CY sind vorzugsweise ausgewählt aus der Gruppe bestehend aus folgenden Unterformeln 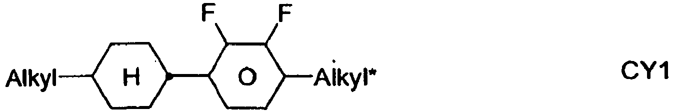 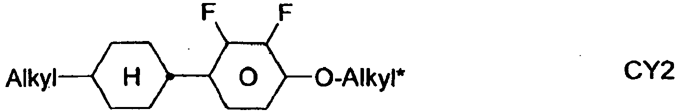 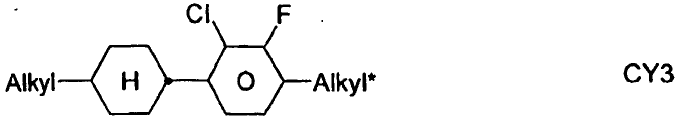 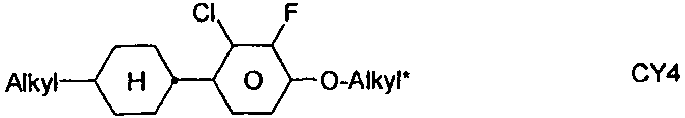 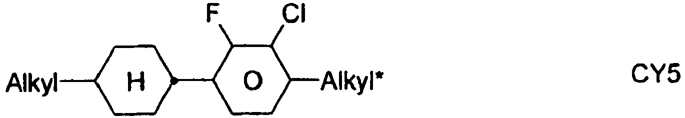 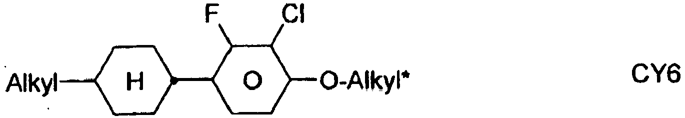 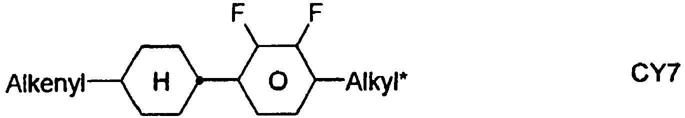 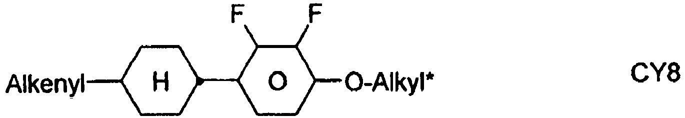 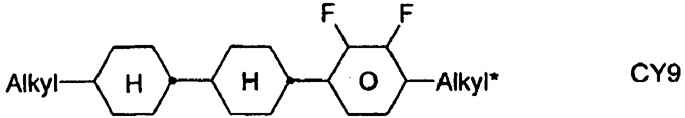 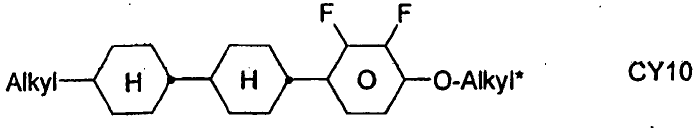 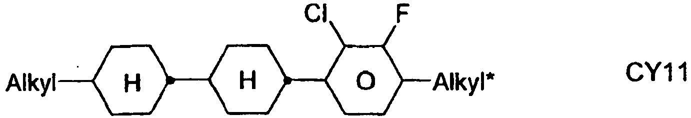 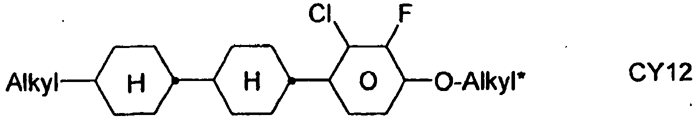 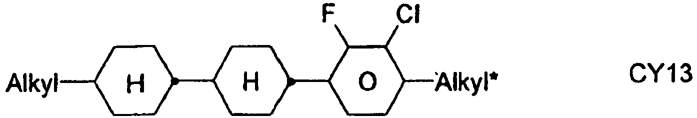 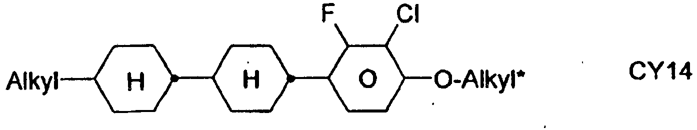 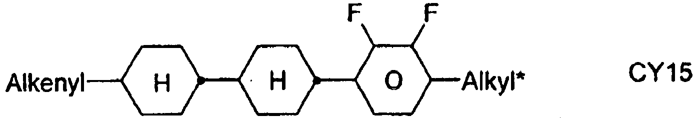 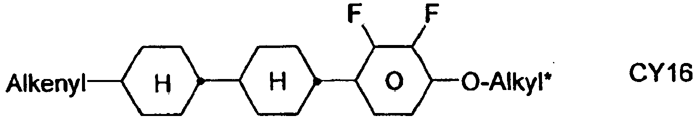 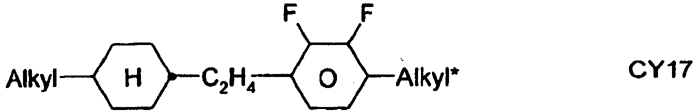 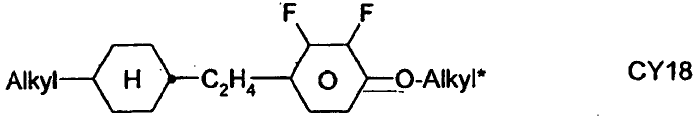 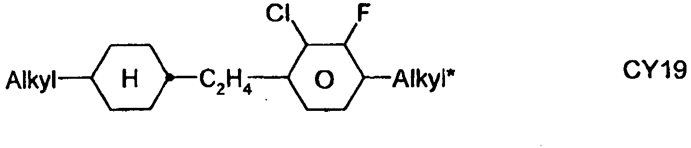 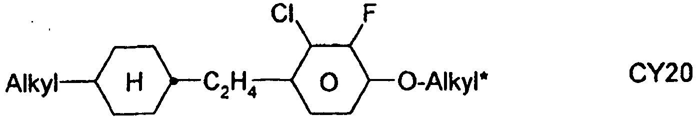 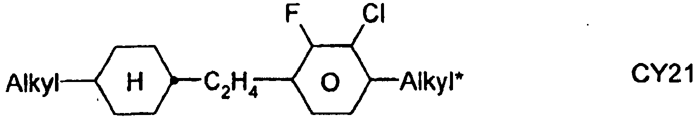 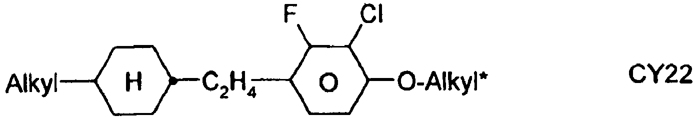 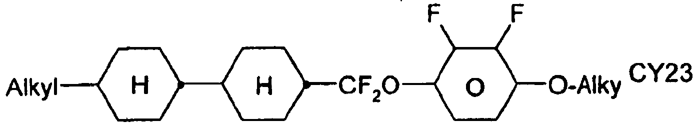 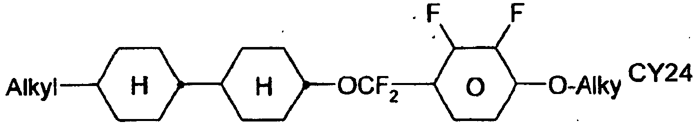 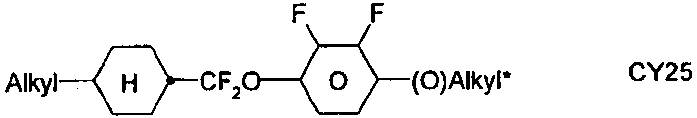 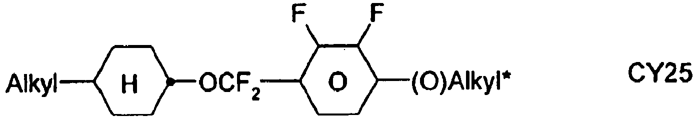 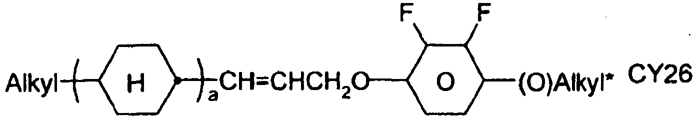 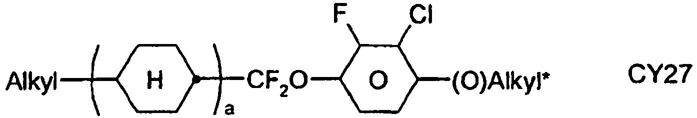 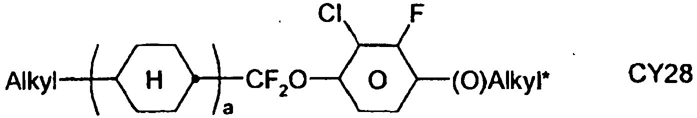 worin a 1 oder 2, Alkyl und Alkyl* jeweils unabhängig voneinander einen geradkettigen Alkylrest mit 1-6 C-Atomen, und Alkenyl einen geradkettigen Alkenylrest mit 2-6 C-Atomen, und (O) ein Sauerstoffatom oder eine Einfachbindung bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.

Die Verbindungen der Formel PY sind vorzugsweise ausgewählt aus der Gruppe bestehend aus folgenden Unterformeln: 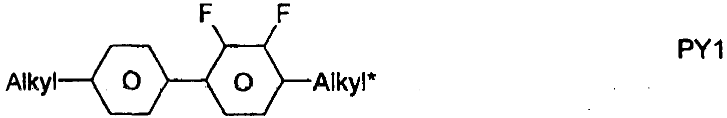 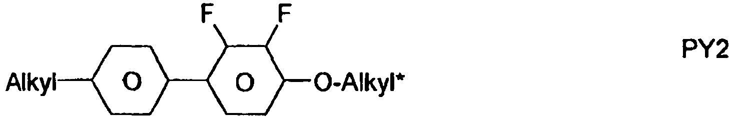 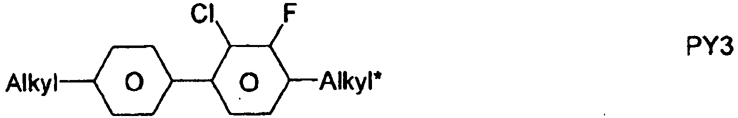 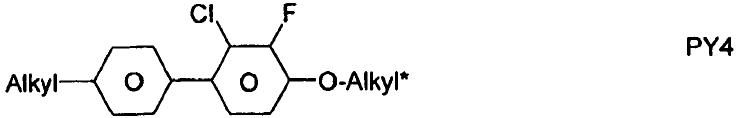 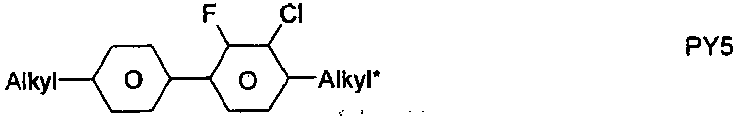 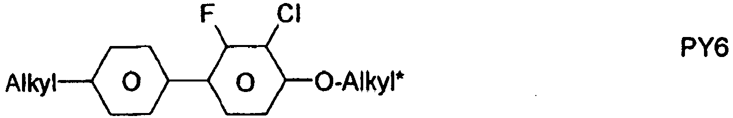 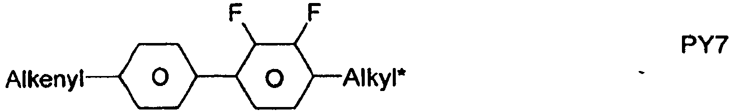 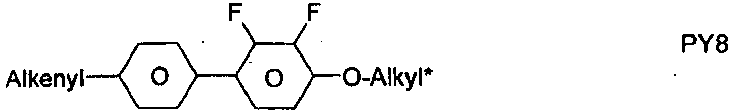 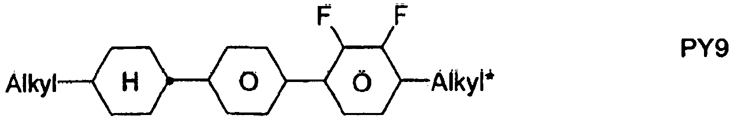 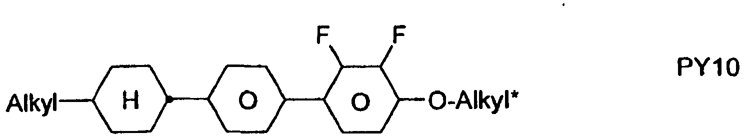 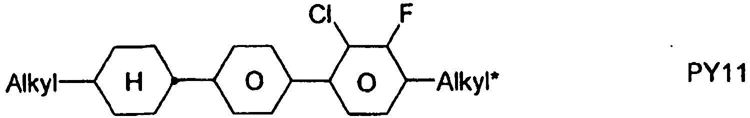 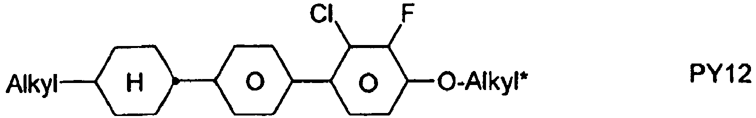 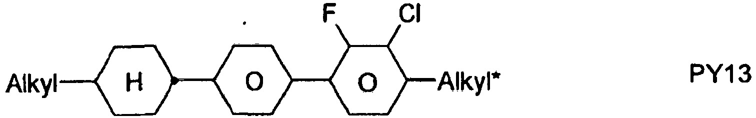 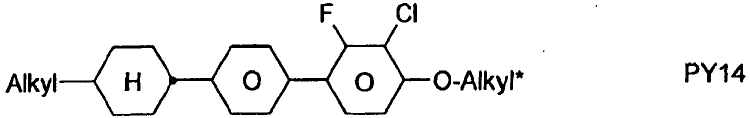 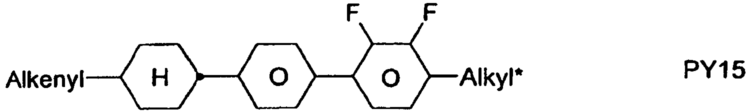 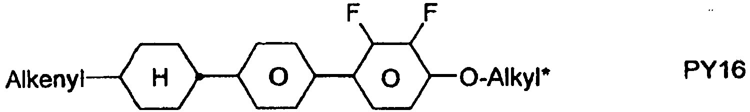 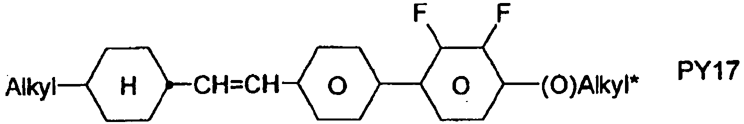 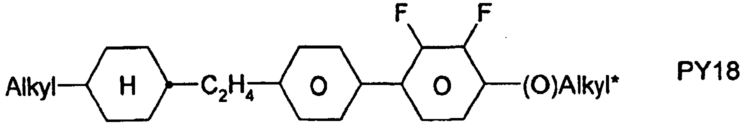 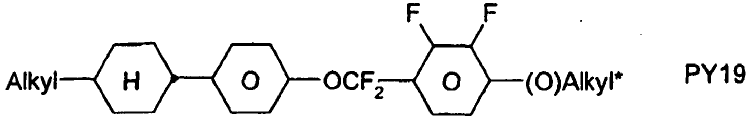 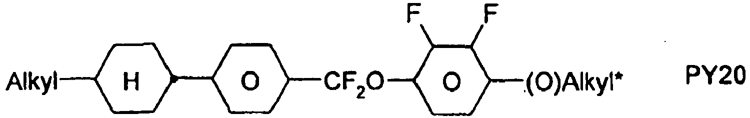 worin Alkyl und Alkyl* jeweils unabhängig voneinander einen geradkettigen Alkylrest mit 1-6 C-Atomen und Alkenyl einen geradkettigen Alkenylrest mit 2-6 C-Atomen, und (O) ein Sauerstoffatom oder eine Einfachbindung bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
b) FK-Medium, welches zusätzlich eine oder mehrere Verbindungen der folgenden Formel enthält: 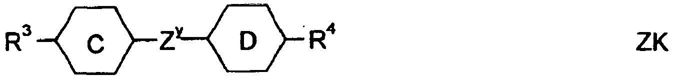 worin die einzelnen Reste folgende Bedeutung besitzen 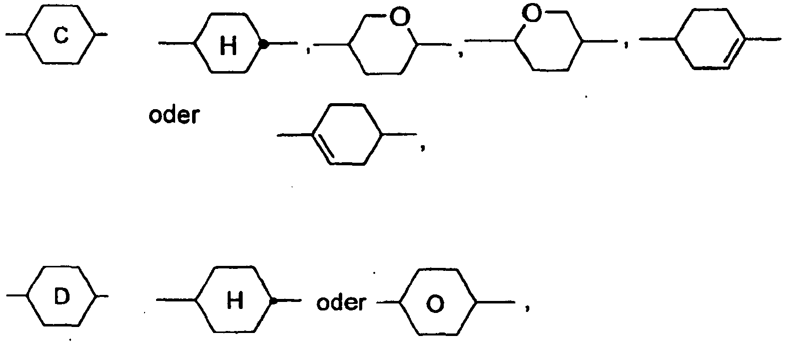
   - R³ und R⁴: jeweils unabhängig voneinander Alkyl mit 1 bis 12 C-Atomen, worin auch eine oder zwei nicht benachbarte CH₂-Gruppen durch -O-, -CH=CH-, -CO-, -O-CO- oder -CO-O- so ersetzt sein können, dass O-Atome nicht direkt miteinander verknüpft sind,
   - Z^{y}: -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung.

   Die Verbindungen der Formel ZK sind vorzugsweise ausgewählt aus der Gruppe bestehend aus folgenden Unterformeln: 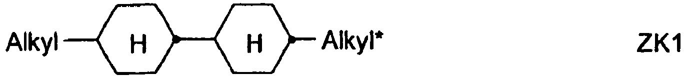 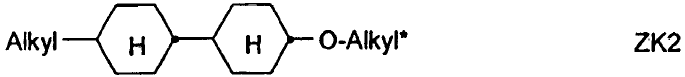 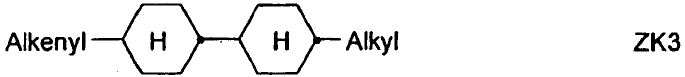 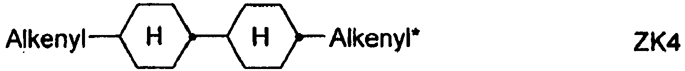 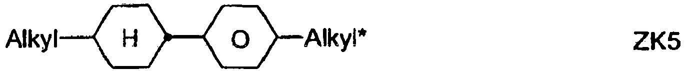 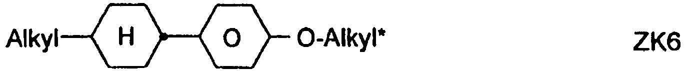 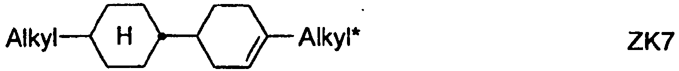 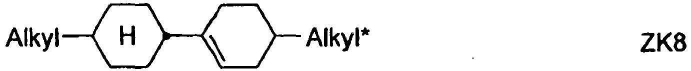 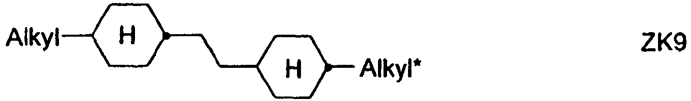 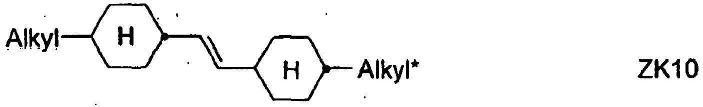 worin Alkyl und Alkyl* jeweils unabhängig voneinander einen geradkettigen Alkylrest mit 1-6 C-Atomen bedeuten, und Alkenyl einen geradkettigen Alkenylrest mit 2-6 C-Atomen bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
c) FK-Medium, welches zusätzlich eine oder mehrere Verbindungen der folgenden Formel enthält: 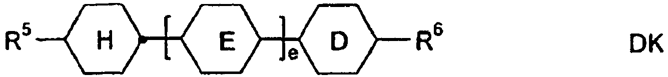 worin die einzelnen Reste bei jedem Auftreten gleich oder verschieden folgende Bedeutung haben:
   - R⁵ und R⁶: jeweils unabhängig voneinander eine der oben für R¹ angegebenen Bedeutungen, 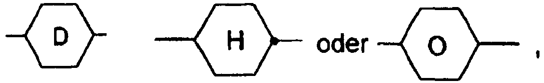 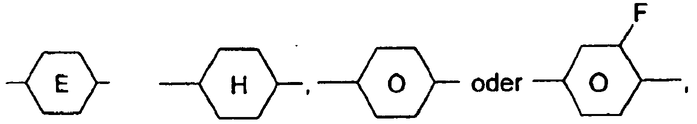 und
   - e: 1 oder 2.

   Die Verbindungen der Formel DK sind vorzugsweise ausgewählt aus der Gruppe bestehend aus folgenden Unterformeln: 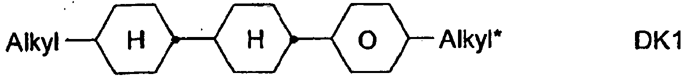 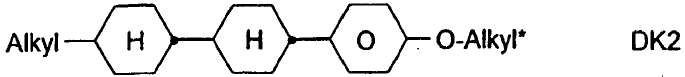 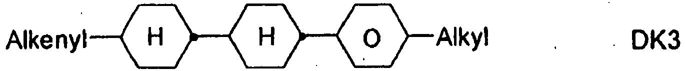 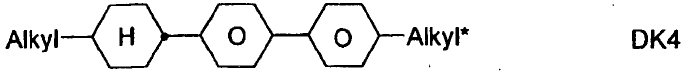 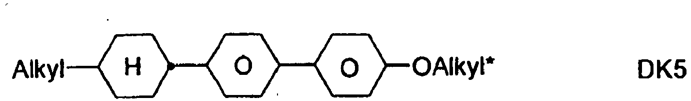 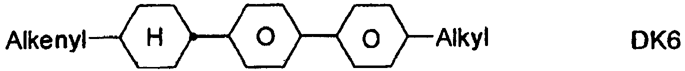 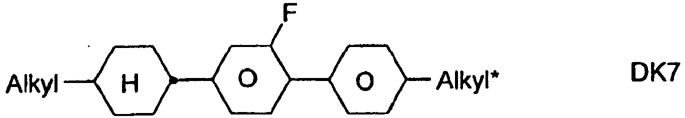 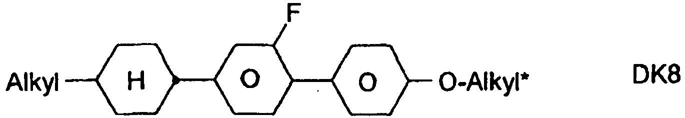 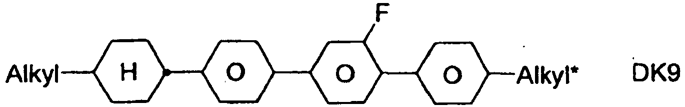 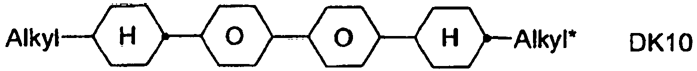 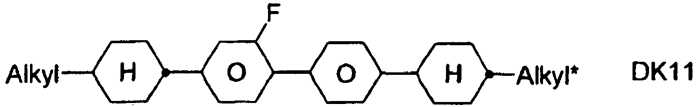 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
d) FK-Medium, welches zusätzlich eine oder mehrere Verbindungen der folgenden Formel enthält: 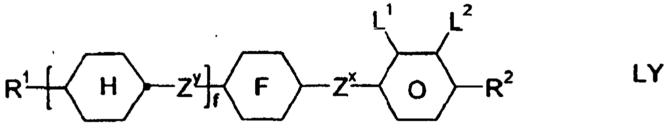 worin die einzelnen Reste folgende Bedeutung besitzen 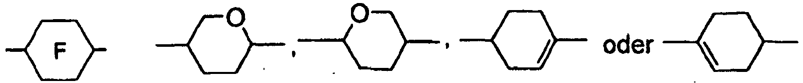
   - f: 0 oder 1,
   - R¹ und R²: jeweils unabhängig voneinander Alkyl mit 1 bis 12 C-Atomen, wobei auch eine oder zwei nicht benachbarte CH₂-Gruppen durch -O-, -CH=CH-, -CO-, -OCO- oder - COO- so ersetzt sein können, dass O-Atome nicht direkt miteinander verknüpft sind,
   - Z^{x} und **Z**^{y}: jeweils unabhängig voneinander -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung,
   - L¹ und L²: jeweils unabhängig voneinander F, Cl, OCF₃, CF₃, CH₃, CH₂F, CHF₂.

   Vorzugsweise bedeuten beide Reste L¹ und L² F oder einer der Reste L¹ und L² F und der andere Cl.
   Die Verbindungen der Formel LY sind vorzugsweise ausgewählt aus der Gruppe bestehend aus folgenden Unterformeln 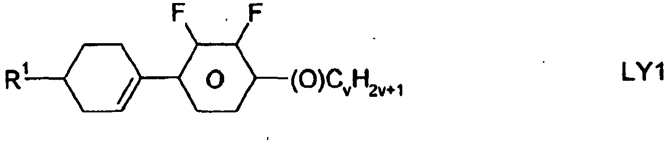 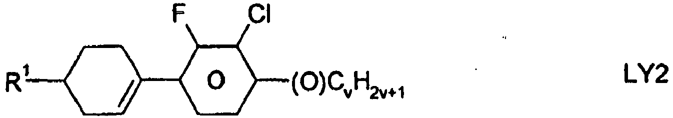 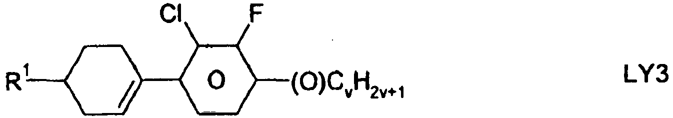 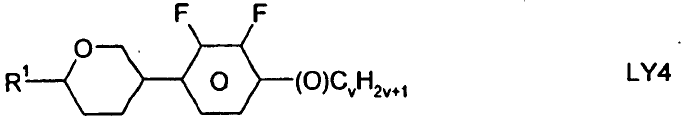 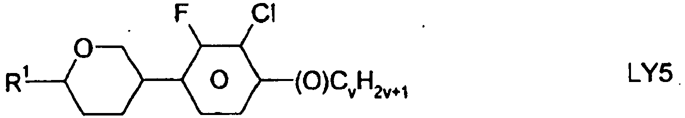 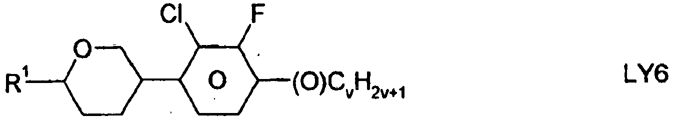 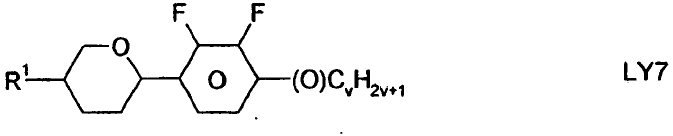 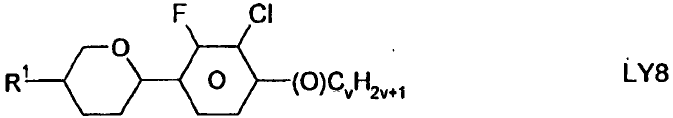 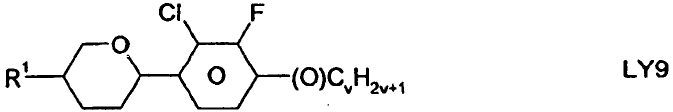 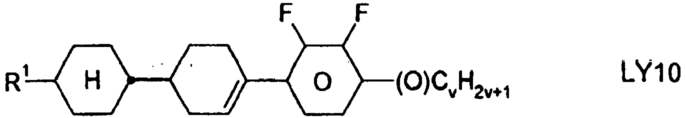 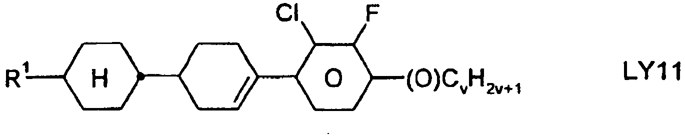 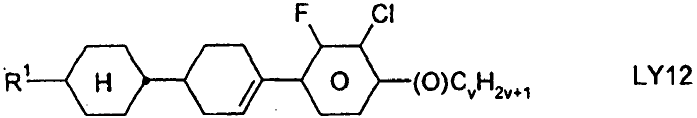 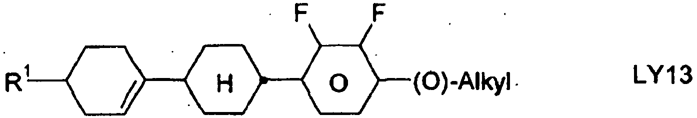 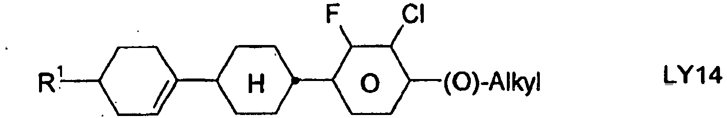 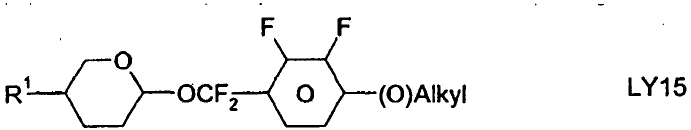 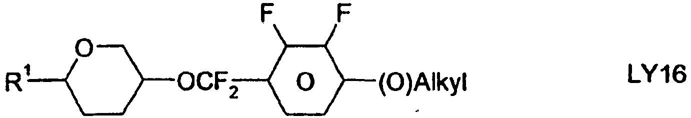 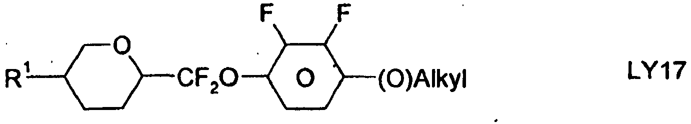 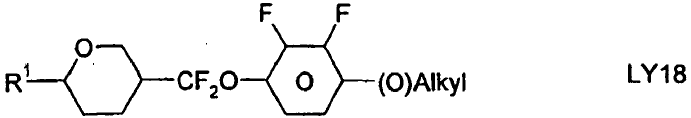 worin R¹ die oben angegebene Bedeutung hat, Alkyl einen geradkettigen Alkylrest mit 1-6 C-Atomen, (O) ein Sauerstoffatom oder eine Einfachbindung und v eine ganze Zahl von 1 bis 6 bedeuten. R¹ bedeutet vorzugsweise geradkettiges Alkyl mit 1 bis 6 C-Atomen oder geradkettiges Alkenyl mit 2 bis 6 C-Atomen, insbesondere CH₃, C₂H₅, n-C₃H₇, n-C₄H₉, n-C₅H₁₁, CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
e) FK-Medium, welches zusätzlich eine oder mehrere Verbindungen ausgewählt aus der Gruppe bestehend aus folgenden Formeln enthält: 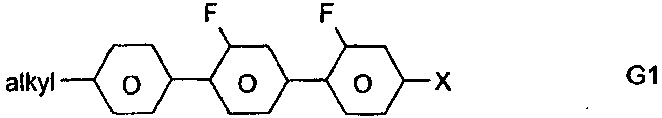 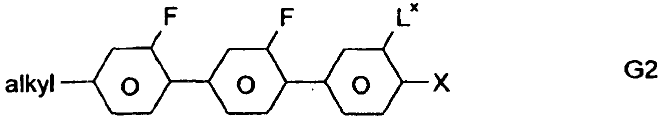 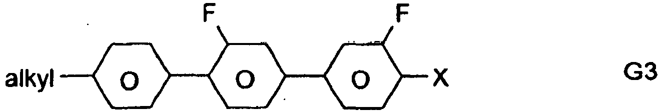 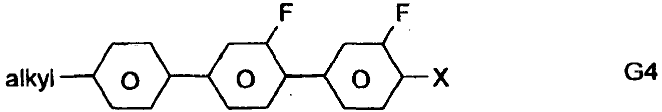 worin alkyl C₁₋₆-alkyl, L^{x} H oder F und X F, Cl, OCF₃, OCHF₂ oder OCH=CF₂ bedeutet. Besonders bevorzugt sind Verbindungen der Formel G1, worin X F bedeutet.
f) FK-Medium, welches zusätzlich eine oder mehrere Verbindungen ausgewählt aus der Gruppe bestehend aus folgenden Formeln enthält: 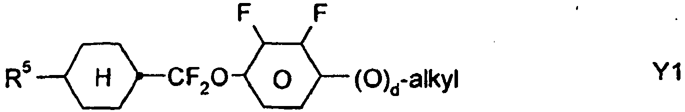 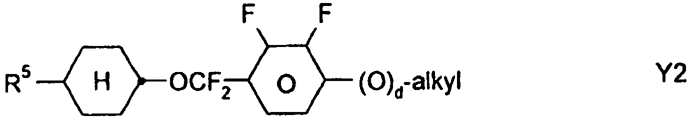 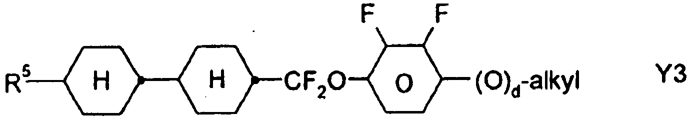 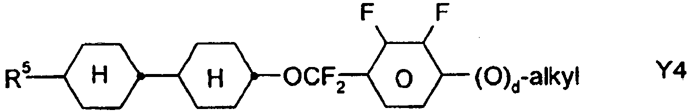 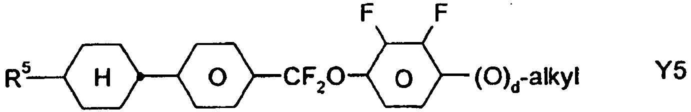 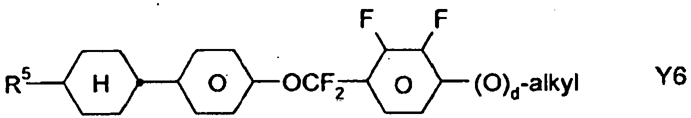 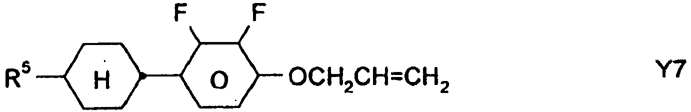 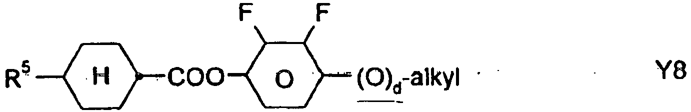 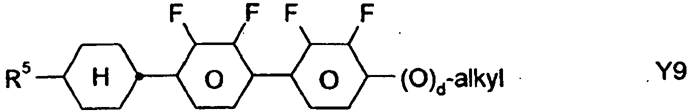 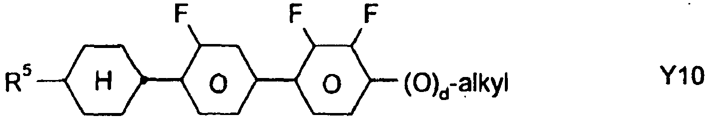 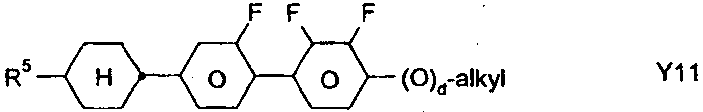 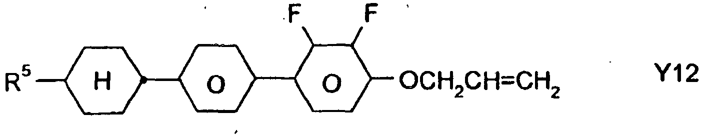 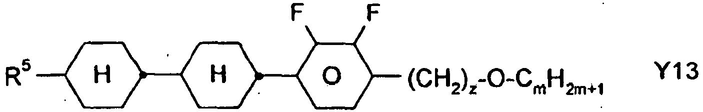 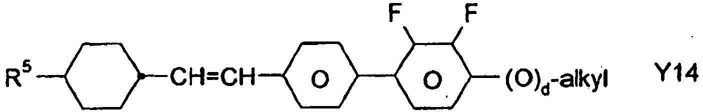 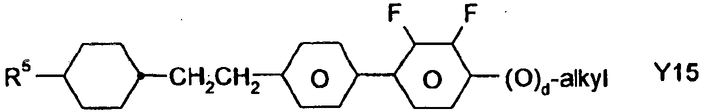 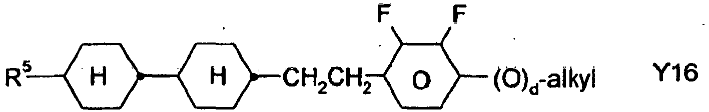 worin R⁵ eine der oben für R¹ angegebenen Bedeutungen besitzt, alkyl C₁₋₆-alkyl, d 0 oder 1, und z und m jeweils unabhängig voneinander eine ganze Zahl von 1 bis 6 bedeuten. R⁵ ist in diesen Verbindungen besonders bevorzugt C₁₋₆-alkyl oder -alkoxy oder C₂₋₆-alkenyl, d ist vorzugsweise 1. Vorzugsweise enthält das erfindungsgemäße FK-Medium eine oder mehrere Verbindungen der oben genannten Formeln in Mengen von ≥ 5 Gew.%.
g) FK-Medium, welches zusätzlich eine oder mehrere Biphenylverbindungen ausgewählt aus der Gruppe bestehend aus folgenden Formeln enthält: 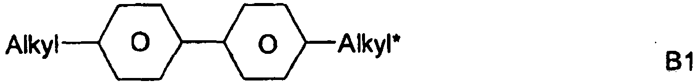 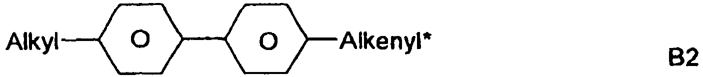 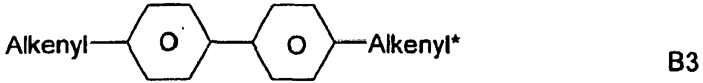 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃(CH₂)₂-CH=CH-, CH₃-(CH₂)₃-CH=CH- oder CH₃-CH=CH-(CH₂)₂-.
   Der Anteil der Biphenyle der Formeln B1 bis B3 in der FK-Mischung beträgt vorzugsweise mindestens 3 Gew.%, insbesondere ≥ 5 Gew.%.
   Die Verbindungen der Formel B2 sind besonders bevorzugt.
   Die Verbindungen der Formel B1 bis B3 sind vorzugsweise ausgewählt aus der Gruppe bestehend aus folgenden Unterformeln 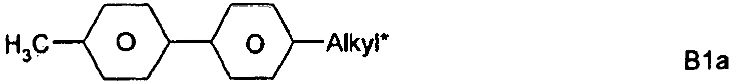 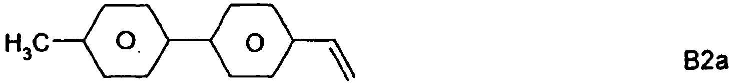 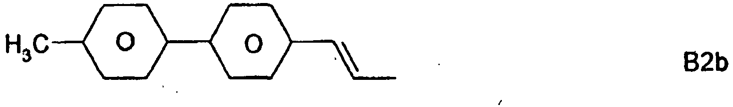 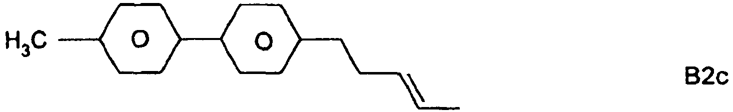 worin Alkyl* einen Alkylrest mit 1-6 C-Atomen bedeutet. Insbesondere bevorzugt enthält das erfindungsgemäße Medium eine oder mehrere Verbindungen der Formeln B1a und/oder B2c.
h) FK-Medium, welches zusätzlich eine oder mehrere Terphenylverbindungen der folgenden Formel enthält: 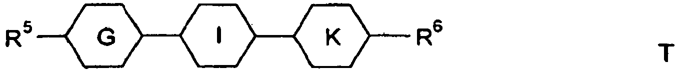 worin R⁵ und R⁶ jeweils unabhängig voneinander eine der oben für R¹ angegebenen Bedeutungen besitzen und 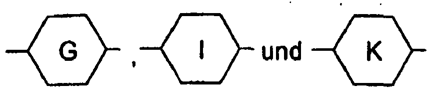 jeweils unabhängig voneinander 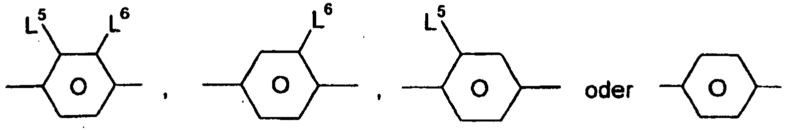 bedeuten, worin L⁵ F oder Cl, vorzugsweise F, und L⁶ F, Cl, OCF₃, CF₃, CH₃, CH₂F oder CHF₂, vorzugsweise F, bedeuten.
   Die Verbindungen der Formel T sind vorzugsweise ausgewählt aus der Gruppe bestehend aus folgenden Unterformeln 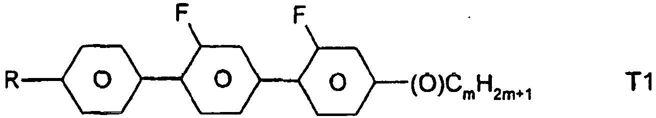 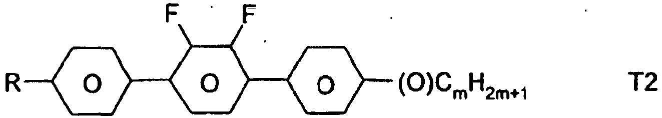 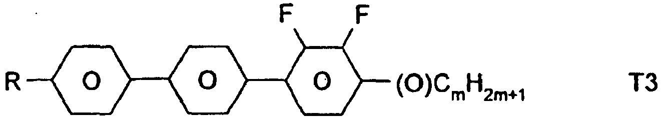 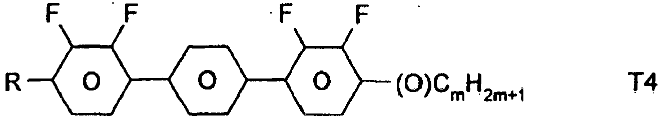 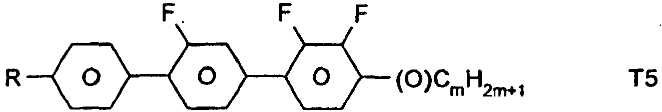 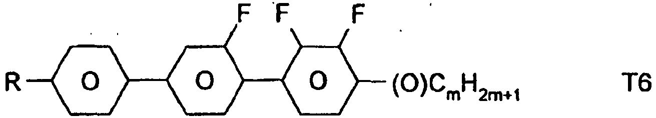 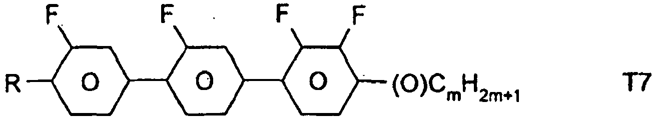 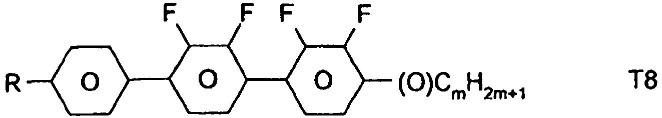 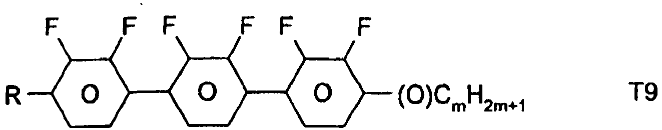 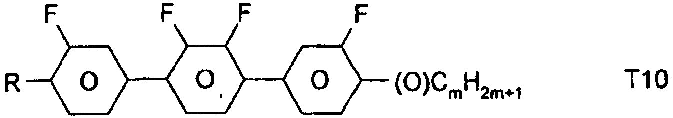 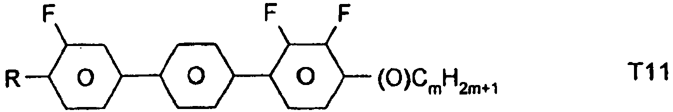 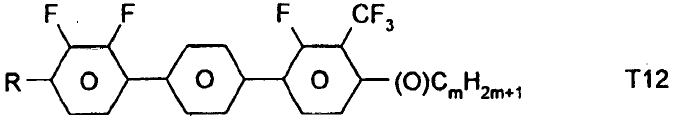 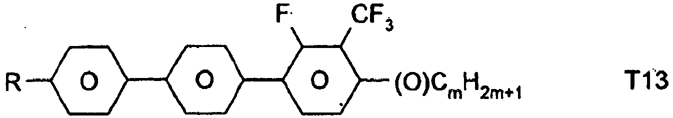 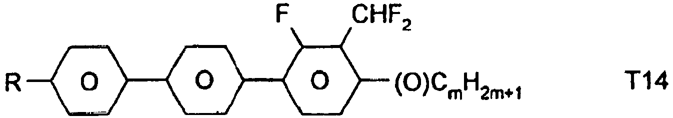 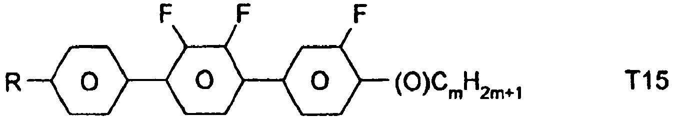 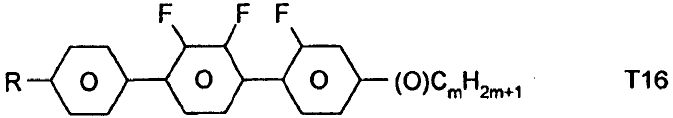 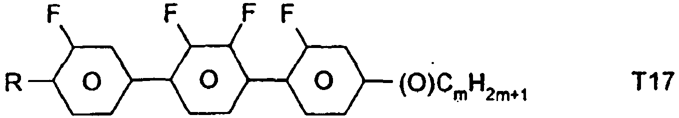 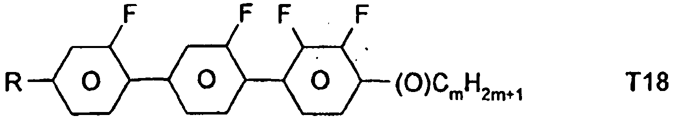 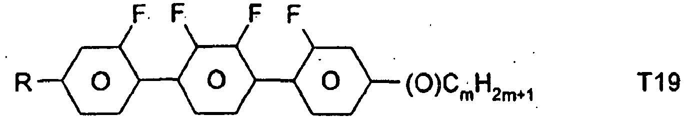 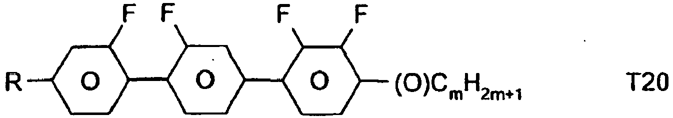 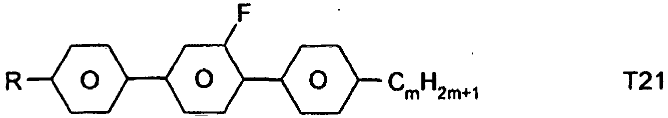 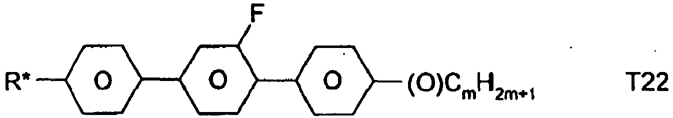 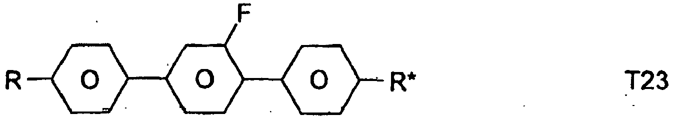 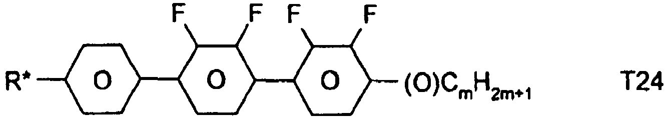 worin R einen geradkettigen Alkyl- oder Alkoxyrest mit 1-7 C-Atomen, R* einen geradkettigen Alkenylrest mit 2-7 C-Atomen, (O) ein Sauerstoffatom oder eine Einfachbindung, und m eine ganze Zahl von 1 bis 6 bedeutet. R* bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Vorzugsweise bedeutet R Methyl, Ethyl, Propyl, Butyl, Pentyl, Hexyl Methoxy, Ethoxy, Propoxy, Butoxy oder Pentoxy.
   Das FK-Medium enthält die Terphenyle der Formeln T und deren bevorzugte Unterformeln vorzugsweise in einer Menge von 0,5-30 Gew.%, insbesondere von 1-20 Gew.%.
   Besonders bevorzugt sind Verbindungen der Formeln T1, T2, T3 und T21. In diesen Verbindungen bedeutet R vorzugsweise Alkyl, ferner Alkoxy jeweils mit 1-5 C-Atomen.
   Vorzugsweise werden die Terphenyle in erfindungsgemäßen Mischungen eingesetzt, wenn der Δn-Wert der Mischung ≥ 0,1 sein soll. Bevorzugte Mischungen enthalten 2-20 Gew.% einer oder mehrerer Terphenyl-Verbindungen der Formel T, vorzugsweise ausgewählt aus der Gruppe der Verbindungen T1 bis T22.
i) FK-Medium, welches zusätzlich eine oder mehrere Verbindungen ausgewählt aus der Gruppe bestehend aus folgenden Formeln enthält: 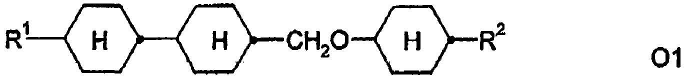 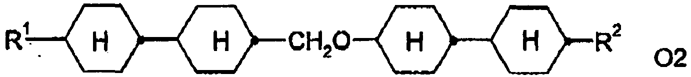 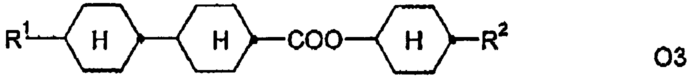 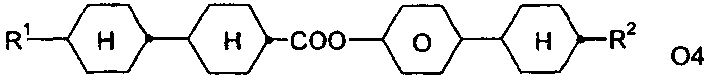 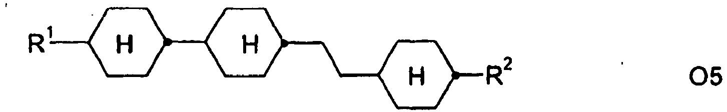 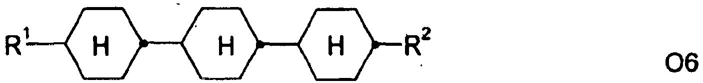 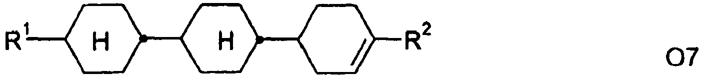 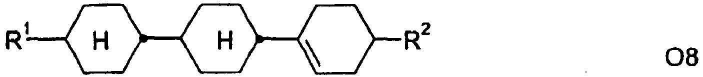 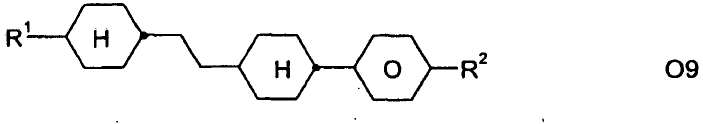 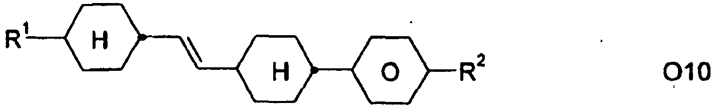 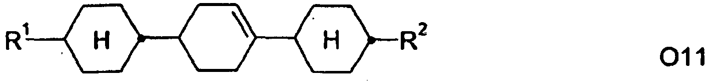 worin R¹ und R² die oben angegebenen Bedeutungen haben, und vorzugsweise jeweils unabhängig voneinander geradkettiges Alkyl mit 1 bis 6 C-Atomen oder geradkettiges Alkenyl mit 2 bis 6 C-Atomen bedeuten.
   Bevorzugte Medien enthalten eine oder mehrere Verbindungen ausgewählt aus den Formeln O1, 03 und 04.
k) FK-Medium, welches zusätzlich eine oder mehrere Verbindungen der folgenden Formel enthält: 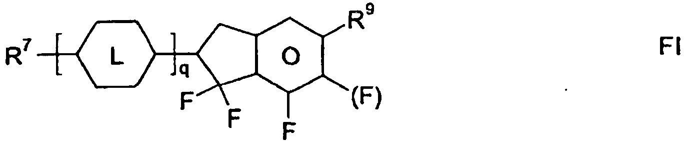 worin 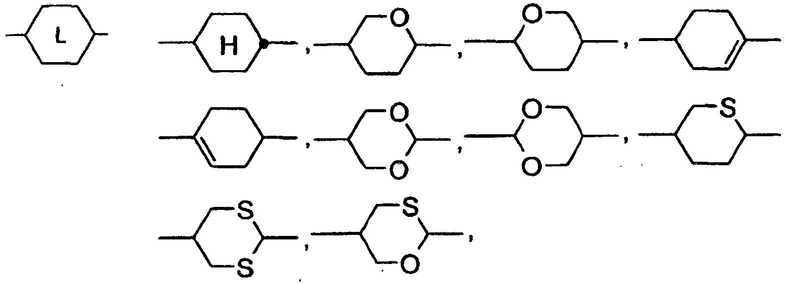 R⁹ H, CH₃, C₂H₅ oder n-C₃H₇, (F) einen optionalen Fluorsubstituenten und q 1, 2 oder 3 bedeutet, und R⁷ eine der für R¹ angegebenen Bedeutungen hat, vorzugsweise in Mengen von > 3 Gew.%, insbesondere ≥ 5 Gew.%, und ganz besonders bevorzugt von 5-30 Gew.%.
   Besonders bevorzugte Verbindungen der Formel IF sind ausgewählt aus der Gruppe bestehend aus folgenden Unterformeln: 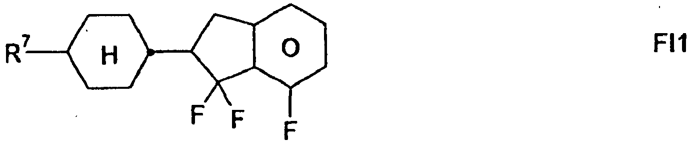 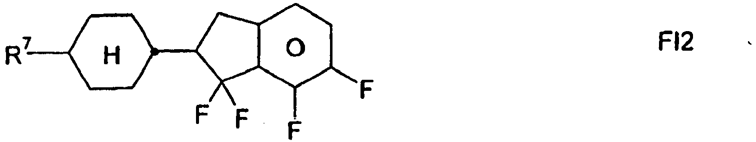 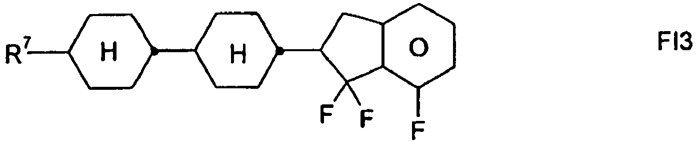 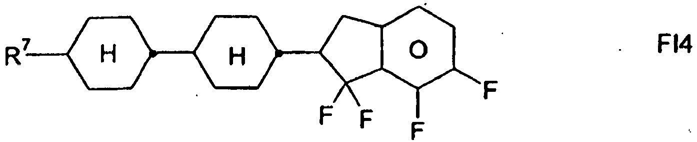 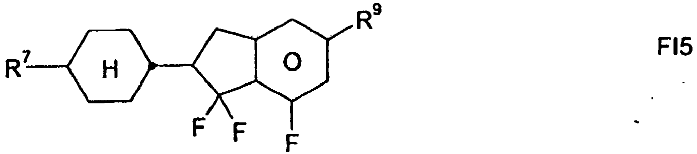 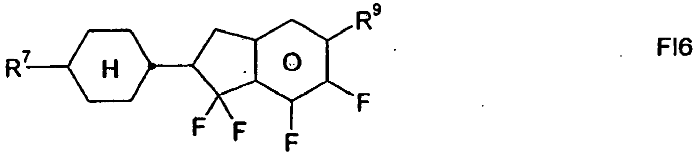 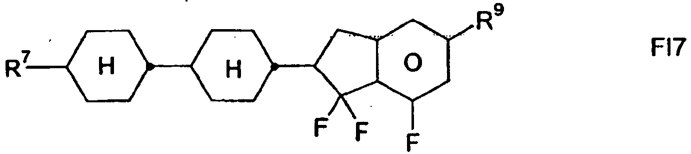 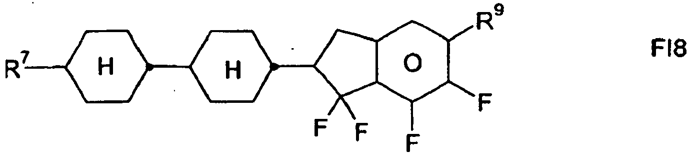 worin R⁷ vorzugsweise geradkettiges Alkyl bedeutet und R⁹ CH₃, C₂H₅ oder n-C₃H₇ bedeutet. Besonders bevorzugt sind die Verbindungen der Formel F11, F12 und F13.
m) FK-Medium, welches zusätzlich eine oder mehrere Verbindungen ausgewählt aus der Gruppe bestehend aus folgenden Formeln enthält: 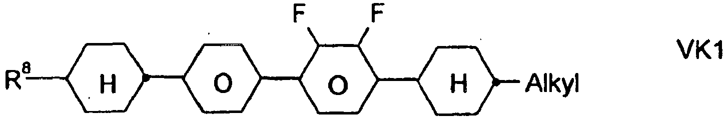 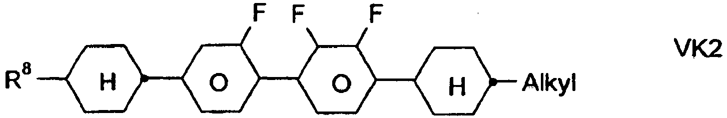 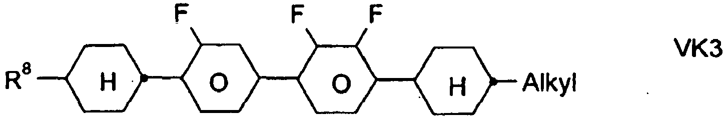 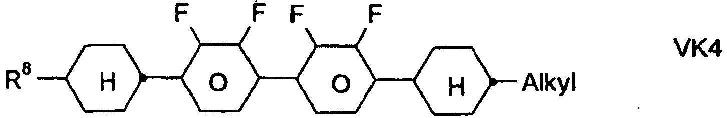 worin R⁸ die für R¹ angegebene Bedeutung hat und Alkyl einen geradkettigen Alkylrest mit 1-6 C-Atomen bedeutet.
n) FK-Medium, welches zusätzlich eine oder mehrere Verbindungen enthält, die eine Tetrahydronaphthyl- oder Naphthyl-Einheit aufweisen, wie z.B. die Verbindungen ausgewählt aus der Gruppe bestehend aus folgenden Formeln: 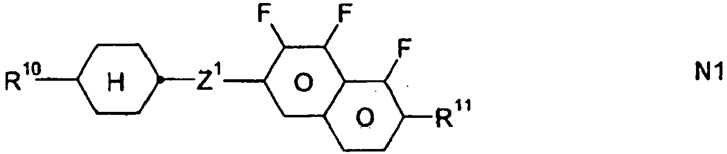 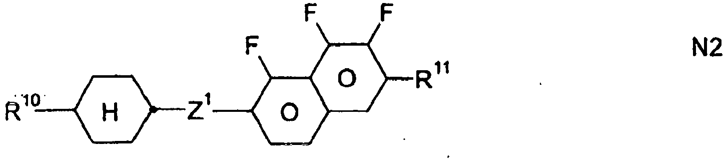 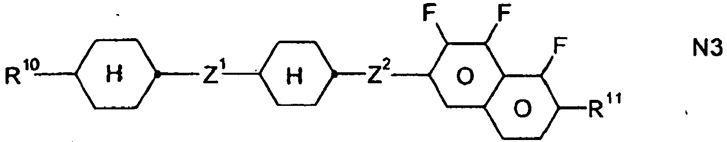 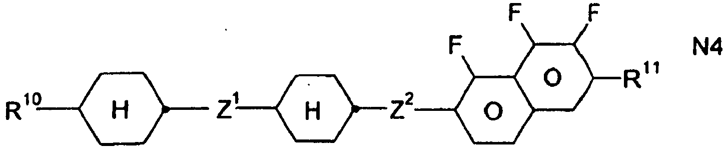 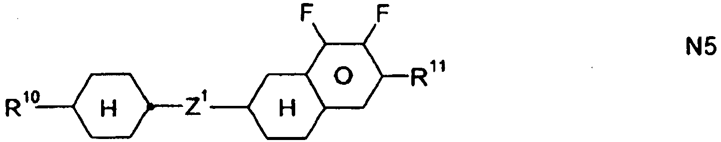 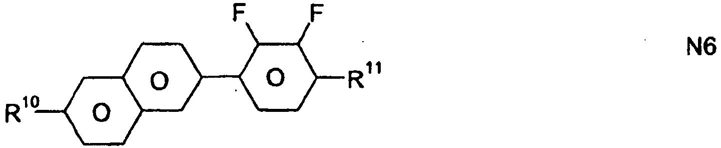 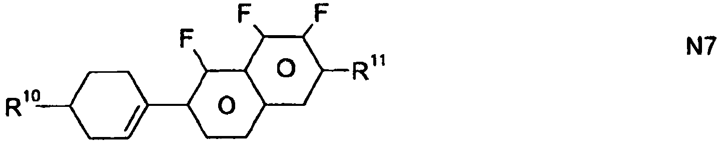 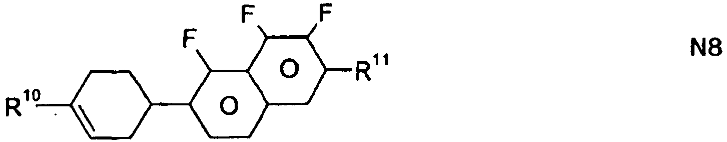 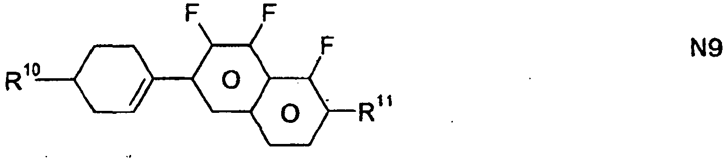 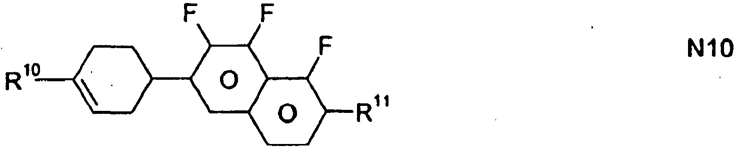 worin R¹⁰ und R¹¹ jeweils unabhängig voneinander eine der für R¹ angegebenen Bedeutungen haben, vorzugsweise geradkettiges Alkyl oder Alkoxy mit 1 bis 6 C-Atomen oder geradkettiges Alkenyl mit 2 bis 6 C-Atomen bedeuten, und Z¹ und Z² jeweils unabhängig voneinander -C₂H₄-, -CH=CH-, -(CH₂)₄-, -(CH₂)₃O-, -O(CH₂)₃-, -CH=CH-CH₂CH₂-, -CH₂CH₂CH=CH-, -CH₂O-, -OCH₂-, -CO-O-, -O-CO-, -C₂F₄-, -CF=CF-, -CF=CH-, -CH=CF-, -CH₂- oder eine Einfachbindung bedeuten.
o) FK-Medium, welches zusätzlich eine oder mehrere eine oder mehrere Difluordibenzochromane und/oder Chromane der folgenden Formeln enthält: 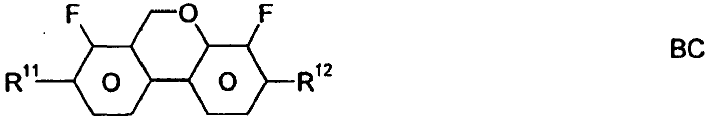 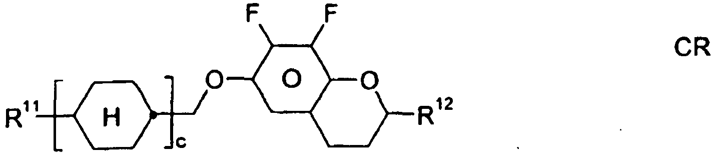 worin R¹¹ und R¹² jeweils unabhängig voneinander die oben angegebene Bedeutung aufweisen, und c 0 oder 1 bedeutet, vorzugsweise in Mengen von 3 bis 20 Gew.%, insbesondere in Mengen von 3 bis 15 Gew.%.
   Besonders bevorzugte Verbindungen der Formeln BC und CR sind ausgewählt aus der Gruppe bestehend aus folgenden Unterformeln: 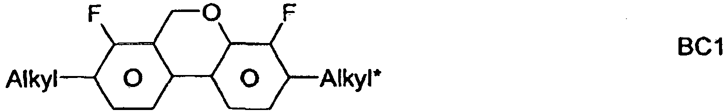 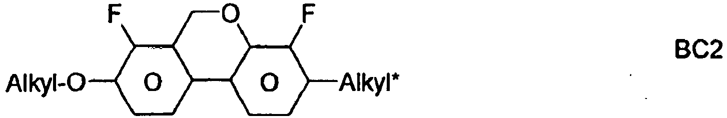 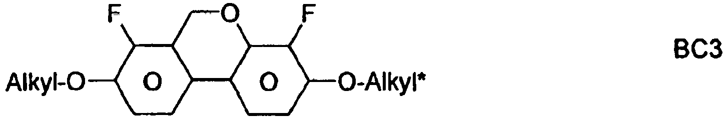 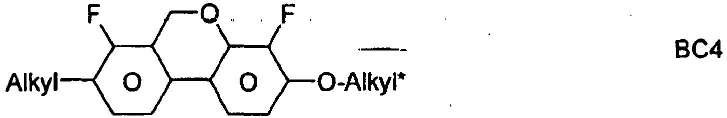 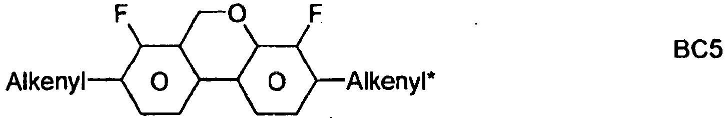 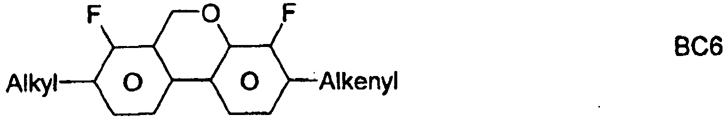 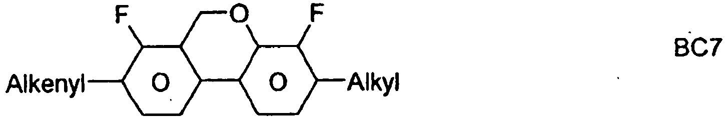 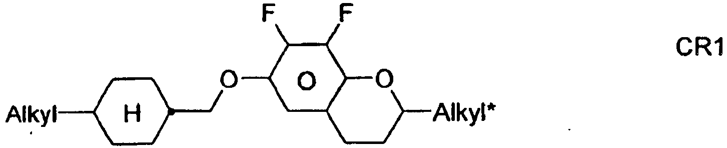 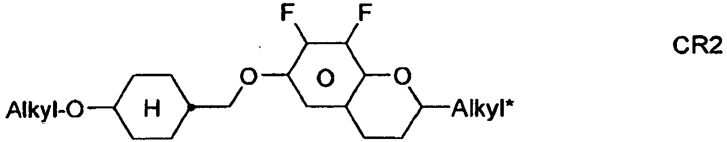 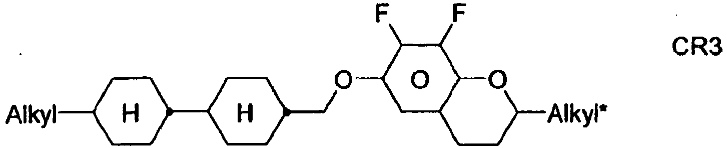 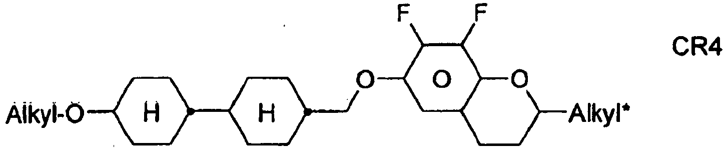 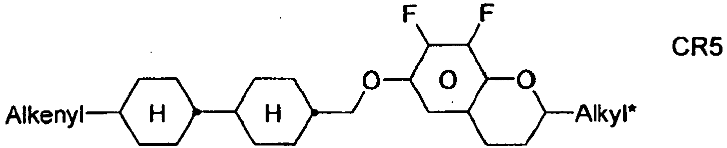 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Ganz besonders bevorzugt sind Mischungen enthaltend eine, zwei oder drei Verbindungen der Formel BC-2.
p) FK-Medium, welches zusätzlich eine oder mehrere fluorierte Phenanthrene und/oder Dibenzofurane der folgenden Formeln enthält: 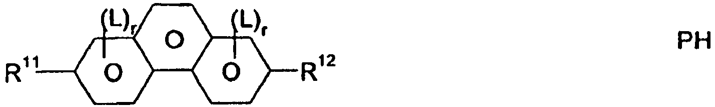 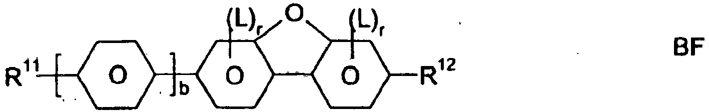 worin R¹¹ und R¹² jeweils unabhängig voneinander die oben angegebenen Bedeutungen besitzen, b 0 oder 1, L F und r 1, 2 oder 3 bedeutet.
   Besonders bevorzugte Verbindungen der Formeln PH und BF sind ausgewählt aus der Gruppe bestehend aus folgenden Unterformeln: 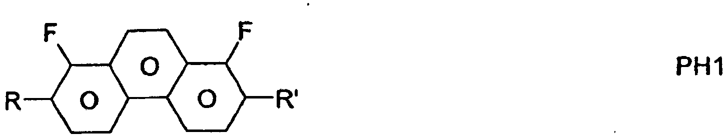 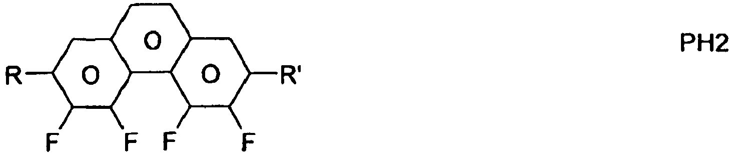 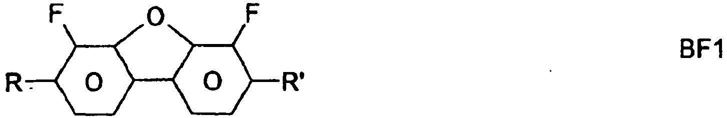 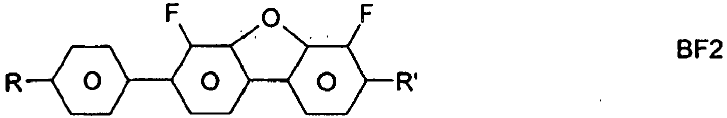 worin R und R' jeweils unabhängig voneinander einen geradkettigen Alkyl- oder Alkoxyrest mit 1-7 C-Atomen bedeuten.
q) FK-Medium, welches 1 bis 8, vorzugsweise 1 bis 5 Verbindungen der Formel CY1, CY2, PY1 und/oder PY2 enthält. Der Anteil dieser Verbindungen im Gesamtgemisch beträgt vorzugsweise 5 bis 60 %, besonders bevorzugt 10 bis 35 %. Der Gehalt dieser einzelnen Verbindungen beträgt vorzugsweise jeweils 2 bis 20 %.
r) FK-Medium, welches 1 bis 8, vorzugsweise 1 bis 5 Verbindungen der Formel CY9, CY10, PY9 und/oder PY10 enthält. Der Anteil dieser Verbindungen im Gesamtgemisch beträgt vorzugsweise 5 bis 60 %, besonders bevorzugt 10 bis 35 %. Der Gehalt dieser einzelnen Verbindungen beträgt vorzugsweise jeweils 2 bis 20 %.
s) FK-Medium, welches 1 bis 10, vorzugsweise 1 bis 8 Verbindungen der Formel ZK enthält, insbesondere Verbindungen der Formel ZK1, ZK2 und/oder ZK6. Der Anteil dieser Verbindungen im Gesamtgemisch beträgt vorzugsweise 3 bis 25 %, besonders bevorzugt 5 bis 45 %. Der Gehalt dieser einzelnen Verbindungen beträgt vorzugsweise jeweils 2 bis 20 %.
t) FK-Medium, worin der Anteil an Verbindungen der Formel CY, PY und ZK im Gesamtgemisch mehr als 70 %, vorzugsweise mehr als 80 % beträgt,
u) PSA-VA-Anzeige, worin der "pretilt"-Winkel vorzugsweise ≤ 86°, beträgt.

Besonders bevorzugte FK-Medien zur Verwendung in PSA-OCB-, PSA-TN-, PSA-IPS- oder PSA-FFS-Anzeigen werden im Folgenden genannt:
a) FK-Medium, welches eine oder mehrere Verbindungen ausgewählt aus der Gruppe bestehend aus folgenden Formeln enthält: 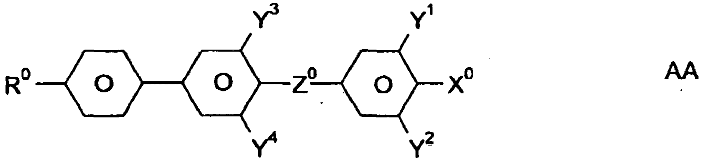 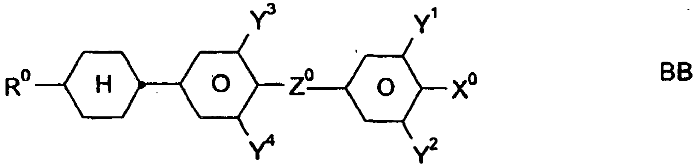 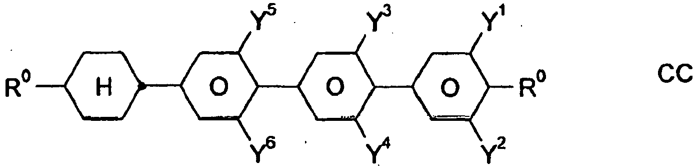 worin
   - R⁰: bei jedem Auftreten gleich oder verschieden n-Alkyl, Alkoxy, Oxaalkyl, Fluoralkyl oder Alkenyl mit jeweils bis zu 9 C-Atomen,
   - X⁰: F, Cl oder jeweils halogeniertes Alkyl, Alkenyl, Alkenyloxy oder Alkoxy mit jeweils bis zu 6 C-Atomen,
   - Z⁰: -CF₂O- oder eine Einfachbindung,
   - Y¹⁻⁶: jeweils unabhängig voneinander H oder F,
   bedeuten.
   X⁰ ist vorzugsweise F, Cl, CF₃, CHF₂, OCF₃, OCHF₂, OCFHCF₃, OCFHCHF₂, OCFHCHF₂, OCF₂CH₃, OCF₂CHF₂, OCF₂CHF₂, OCF₂CF₂CHF₂, OCF₂CF₂CHF₂, OCFHCF₂CF₃, OCFHCF₂CHF₂, OCF₂CF₂CF₃, OCF₂CF₂CClF₂, OCClFCF₂CF₃ oder CH=CF₂, besonders bevorzugt F oder OCF₃.
   Die Verbindungen der Formel AA sind vorzugsweise ausgewählt aus der Gruppe bestehend aus folgenden Formeln: 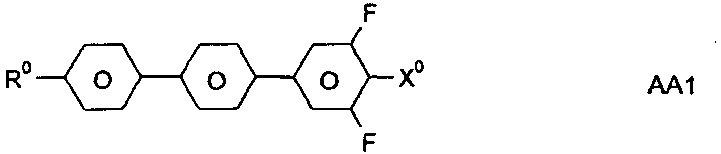 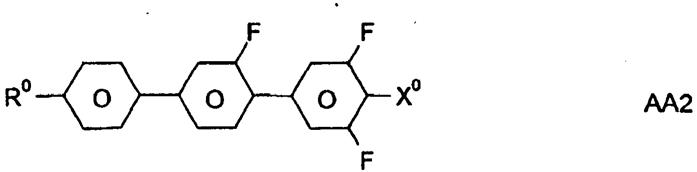 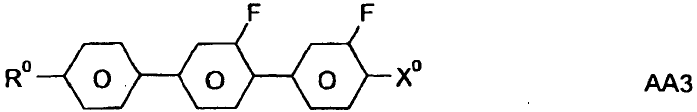 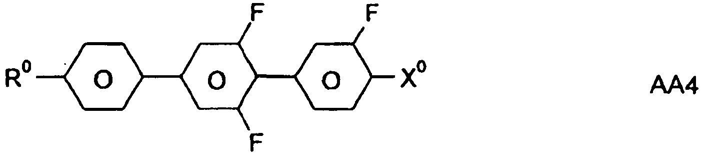 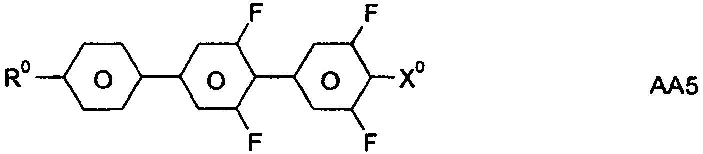 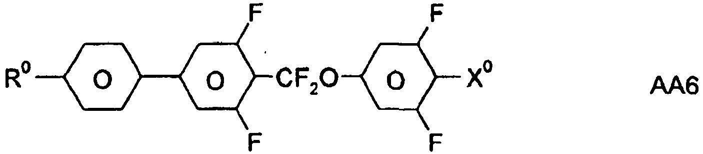 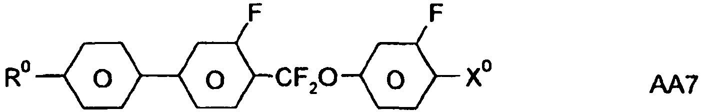 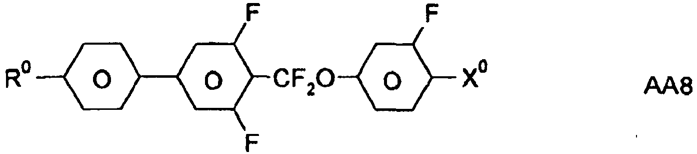 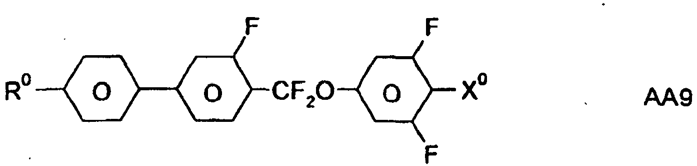 worin R⁰ und X⁰ die oben angegebene Bedeutung besitzen, und X⁰ vorzugsweise F bedeutet. Besonders bevorzugt sind Verbindungen der Formeln AA2 und AA6.
   Die Verbindungen der Formel BB sind vorzugsweise ausgewählt aus der Gruppe bestehend aus folgenden Formeln: 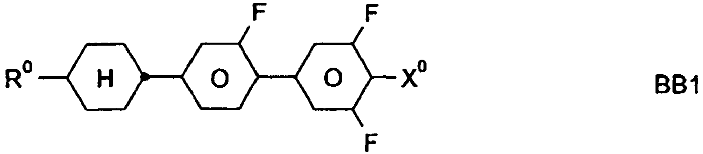 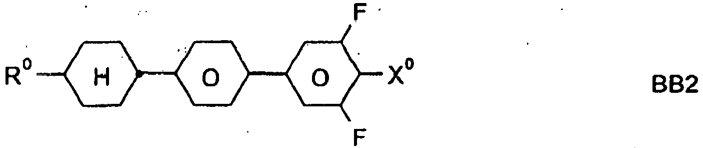 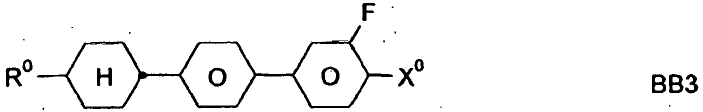 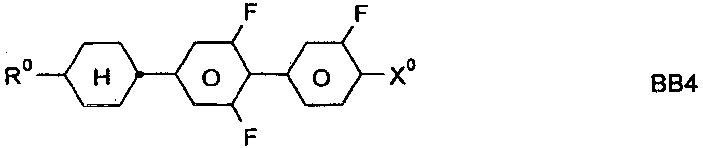 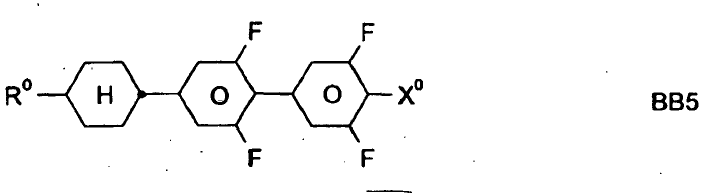 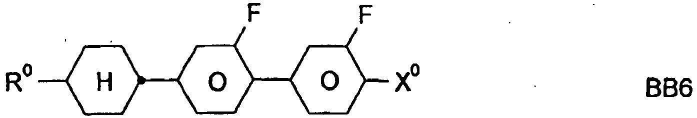 worin R⁰ und X⁰ die oben angegebene Bedeutung besitzen, und X⁰ vorzugsweise F bedeutet. Besonders bevorzugt sind Verbindungen der Formeln BB1, BB2 und BB5.
   Die Verbindungen der Formel CC sind vorzugsweise ausgewählt aus folgender Formel: 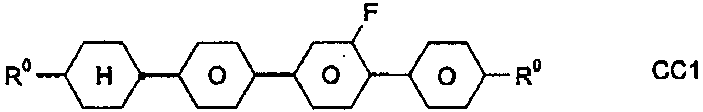 worin R⁰ bei jedem Auftreten gleich oder verschieden die oben angegebene Bedeutung besitzt, und vorzugsweise Alkyl mit 1 bis 6 C-Atomen bedeutet.

Die Kombination von Verbindungen der oben genannten FK-Medien a)-y) mit den oben beschriebenen polymerisierten Verbindungen bewirkt in den FK-Medien niedrige Schwellenspannungen, niedrige Rotationsviskositäten und sehr gute Tieftemperaturstabilitäten bei gleichbleibend hohen Klärpunkten und hohen HR-Werten, und erlaubt die schnelle Einstellung eines besonders niedrigen "pretilt"-Winkels in PSA-Anzeigen. Insbesondere zeigen die FK-Medien in PSA-Anzeigen deutlich verringerte Schaltzeiten, insbesondere auch der Graustufenschaltzeiten.

Vorzugsweise weist die Flüssigkristallmischung einen nematischen Phasenbereich von mindestens 80 K, besonders bevorzugt von mindestens 100 K, und eine Rotationsviskosität von nicht mehr als 250, vorzugsweise nicht mehr als 200 mPa·s, bei 20°C auf.

Die Vergleichs-FK-Medien zur Verwendung in Anzeigen des PSA-VA-Typs weisen eine negative dielektrische Anisotropie Δε auf, vorzugsweise von etwa -0,5 bis -10, insbesondere von etwa -2,5 bis -7,5 bei 20°C und 1 kHz.

In Anzeigen des VA-Typs sind die Moleküle in der Schicht des FK-Mediums im ausgeschalteten Zustand senkrecht zu den Elektrodenflächen (homöotrop) oder gekippt homöotrop (engl. "tilted") orientiert. Bei Anlegen einer elektrischen Spannung an die Elektroden findet eine Umorientierung der FK-Moleküle mit den Moleküllängsachsen parallel zu den Elektrodenflächen statt.

In Anzeigen des OCB-Typs sind die Moleküle in der Schicht des FK-Mediums eine "bend"-Orientierung auf. Bei Anlegen einer elektrischen Spannung findet eine Umorientierung der FK-Moleküle mit den Moleküllängsachsen senkrecht zu den Elektrodenflächen statt.

FK-Medien zur Verwendung in Anzeigen des PSA-OCB-Typs weisen vorzugsweise eine positive dielektrische Anisotropie Δε auf, vorzugsweise von etwa +4 bis +17 bei 20°C und 1 kHz.

Die Doppelbrechung Δn in FK-Medien zur Verwendung in Anzeigen des VA-Typs liegt vorzugsweise unter 0,16, besonders bevorzugt zwischen 0,06 und 0,14, insbesondere zwischen 0,07 und 0,12.

Die Doppelbrechung Δn in FK-Medien zur Verwendung in Anzeigen des OCB-Typs liegt vorzugsweise zwischen 0,14 und 0,22, insbesondere zwischen 0,16 und 0,22.

Die Doppelbrechung Δn in FK-Medien zur Verwendung In Anzeigen des PSA-TN-, PSA-IPS-, oder PSA-FFS-Typs liegt vorzugsweise zwischen 0,07 und 0,15, insbesondere zwischen 0,08 und 0,13. Die dielektrische Anisotropie dieser Medien liegt vorzugsweise zwischen +2 und +17, insbesondere zwischen +3 und +15.

Die FK-Medien können auch weitere, dem Fachmann bekannte und in der Literatur beschriebene Zusätze oder Additive enthalten, wie beispielsweise Polymerisationsinitiatoren, Inhibitoren, Stabilisatoren, oberflächenaktive Substanzen oder chirale Dotierstoffe. Diese können polymerisierbar oder unpolymerisierbar sein. Polymerisierbare Additive werden dementsprechend der polymerisierbaren Komponente zugerechnet. Unpolymerisierbare Additive werden dementsprechend der flüssigkristallinen Komponente zugerechnet.

Die FK-Medien können beispielsweise einen oder mehrere chirale Dotierstoffe enthalten, vorzugsweise solche ausgewählt aus der Gruppe bestehend aus Verbindungen der nachfolgenden Tabelle B.

Ferner können den FK-Medien beispielsweise 0 bis 15 Gew.-% pleochroitische Farbstoffe zugesetzt werden, ferner Nanopartikel, Leitsalze, vorzugsweise Ethyl-dimethyldodecylammonium-4-hexoxybenzoat, Tetrabutylammoniumtetraphenylborat oder Komplexsalze von Kronenethern (vgl. z.B. Haller et al., Mal. Cryst. Uq. Cryst. 24, 249-258 (1973)) zur Verbesserung der Leitfähigkeit, oder Substanzen zur Veränderung der dielektrischen Anisotropie, der Viskosität und/oder der Orientierung der nematischen Phasen. Derartige Substanzen sind z.B. in DE-A 22 09 127, 22 40 864, 23 21 632, 23 38 281, 24 50 088, 26 37 430 und 28 53 728 beschrieben.

Die einzelnen Komponenten der Vergleichsformen a)-z) sind entweder bekannt, oder ihre Herstellungsweisen sind für den einschlägigen Fachmann aus dem Stand der Technik ohne weiteres abzuleiten, da sie auf in der Literatur beschriebenen Standardverfahren basieren. Entsprechende Verbindungen der Formel CY werden beispielsweise in EP-A-0 364 538 beschrieben. Entsprechende Verbindungen der Formel ZK werden beispielsweise in DE-A-26 36 684 und DE-A-33 21 373 beschrieben.

Die Herstellung der FK-Medien erfolgt in an sich üblicher Weise, beispielsweise indem man eine oder mehrere der oben genannten Verbindungen mit einer oder mehreren polymerisierbaren Verbindungen wie oben definiert, und ggf. mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton,
Chloroform oder Methanol, zu mischen und das Lösungsmittel nach Durchmischung wieder zu entfernen, beispielsweise durch Destillation. Das Verfahren zur Herstellung der erfindungsgemäßen FK-Medien ist ein weiterer Gegenstand der Erfindung.

Es versteht sich für den Fachmann von selbst, daß die FK-Medien auch Verbindungen enthalten können, worin beispielsweise H, N, O, Cl, F durch die entsprechenden Isotope ersetzt sind.

Der Aufbau der PSA-Anzeigen entspricht der für PSA-Anzeigen üblichen Geometrie, wie er im eingangs zitierten Stand der Technik beschrieben ist. Es sind Geometrien ohne Protrusions bevorzugt, insbesondere diejenigen, bei denen darüber hinaus die Elektrode auf der Colour FilterSeite unstrukturiert ist und lediglich die Elektrode auf der TFT-Seite Schlitze aufweist. Besonders geeignete und bevorzugte Elektrodenstrukturen für PSA-VA-Anzeigen sind beispielsweise in US 2006/0066793 A1 beschrieben.

Die folgenden Beispiele erläutern die vorliegende Erfindung ohne sie zu begrenzen. Sie zeigen dem Fachmann jedoch bevorzugte Mischungskonzepte mit bevorzugt einzusetzenden Verbindungen und deren jeweiligen Konzentrationen sowie deren Kombinationen miteinander. Außerdem illustrieren die Beispiele, welche Eigenschaften und Eigenschftskombinationen zugänglich sind.

Folgende Abkürzungen werden verwendet:
(n, m, z: jeweils unabhängig voneinander 1, 2, 3, 4, 5 oder 6)

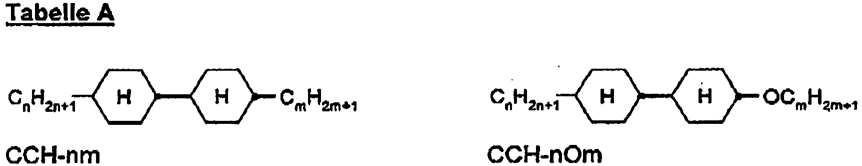

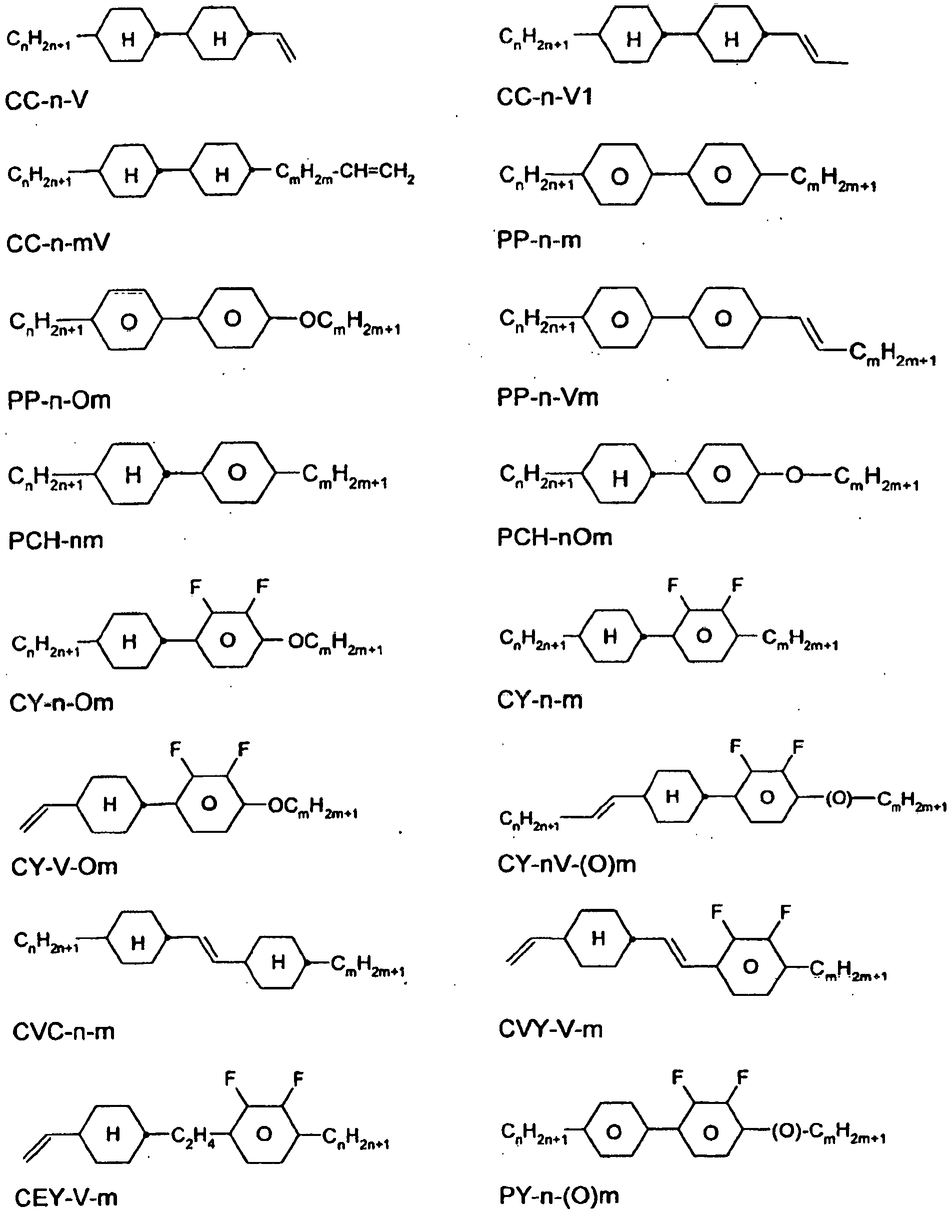

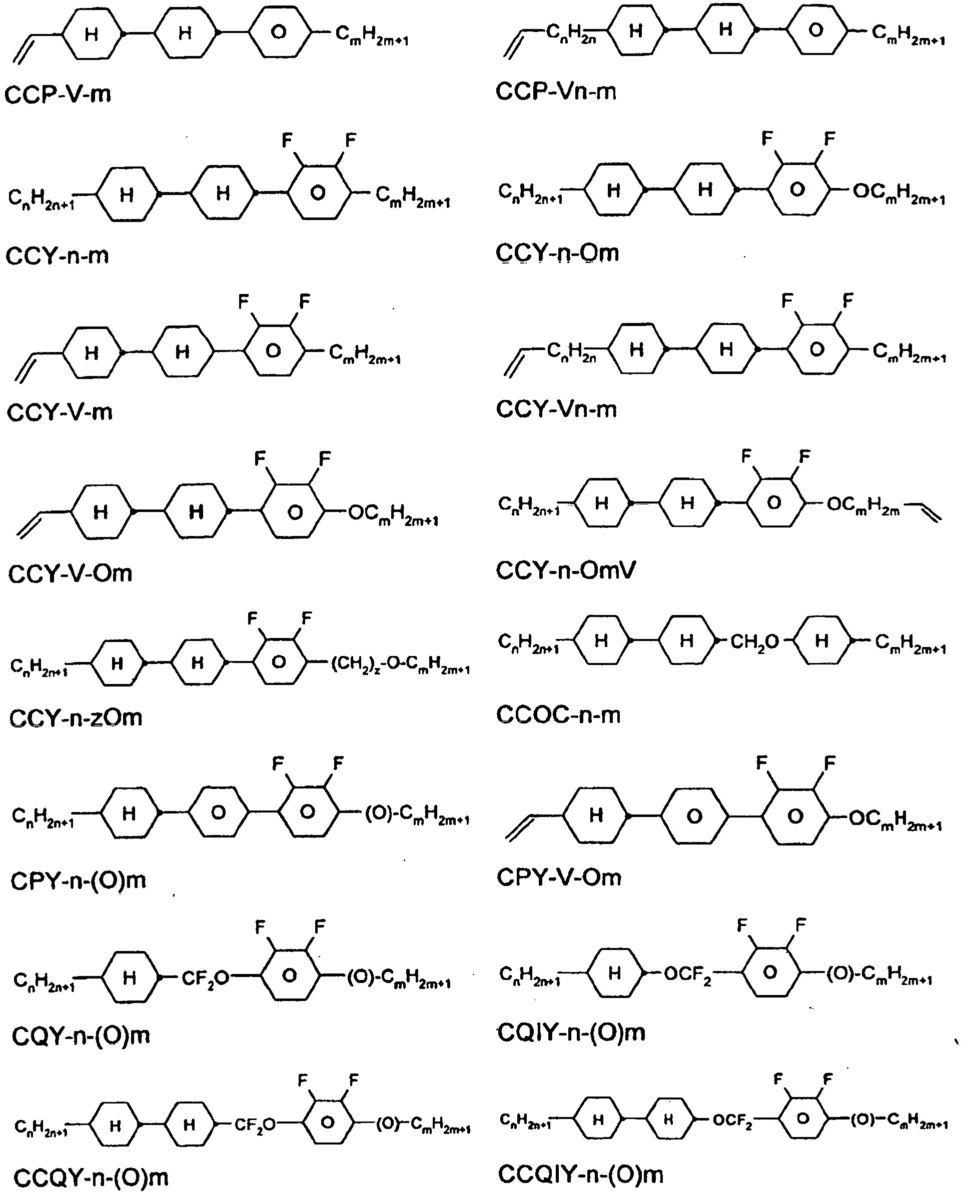

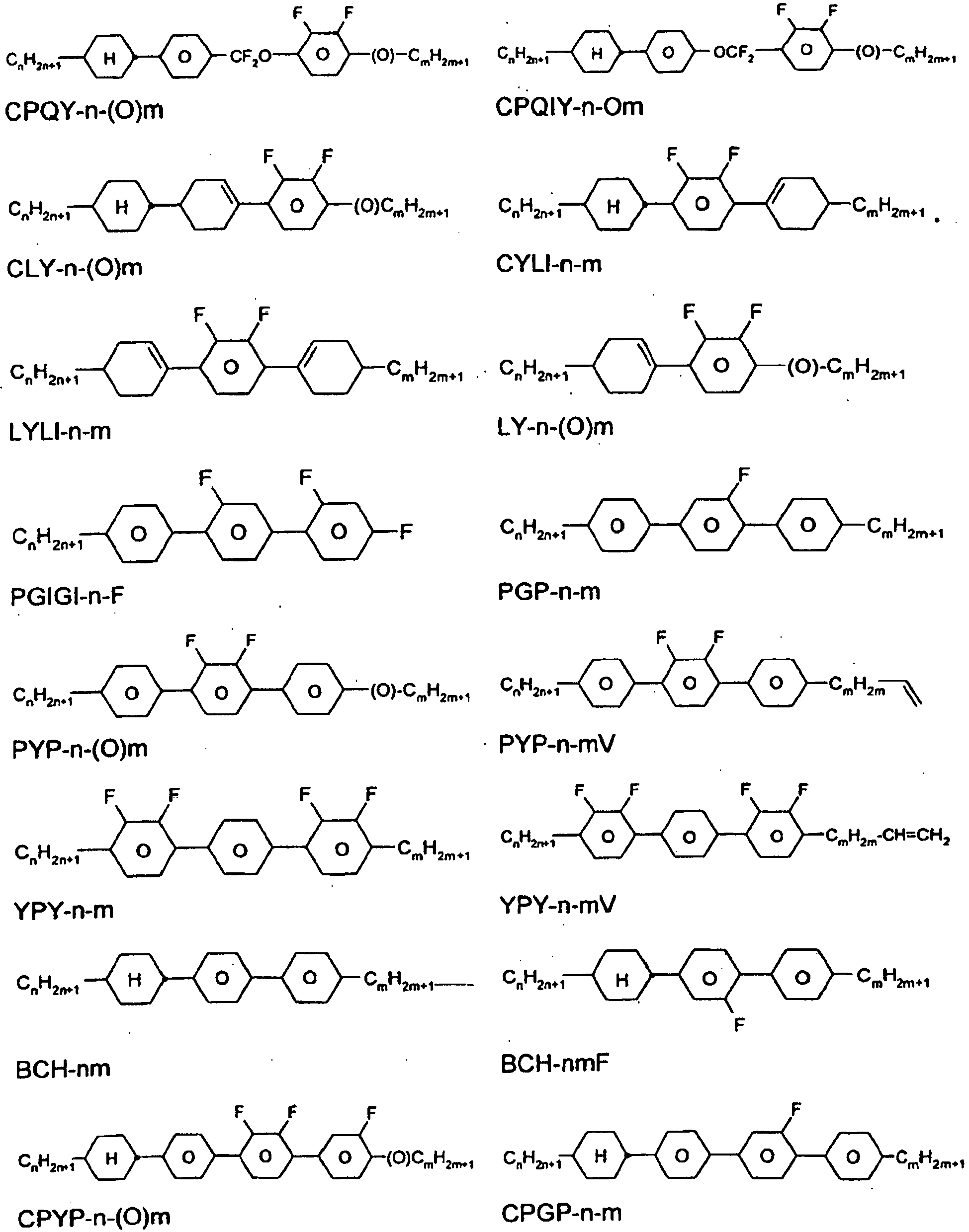

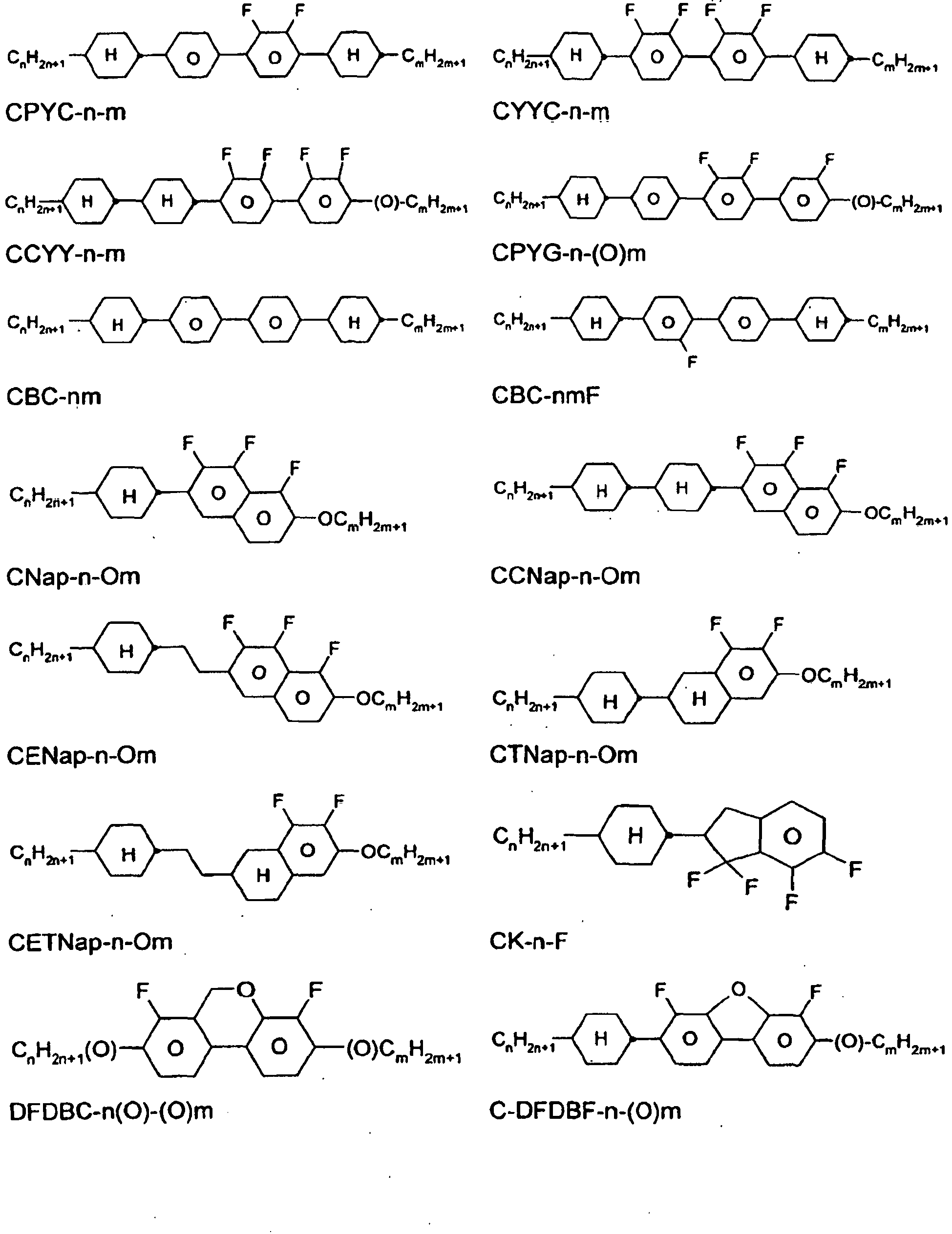

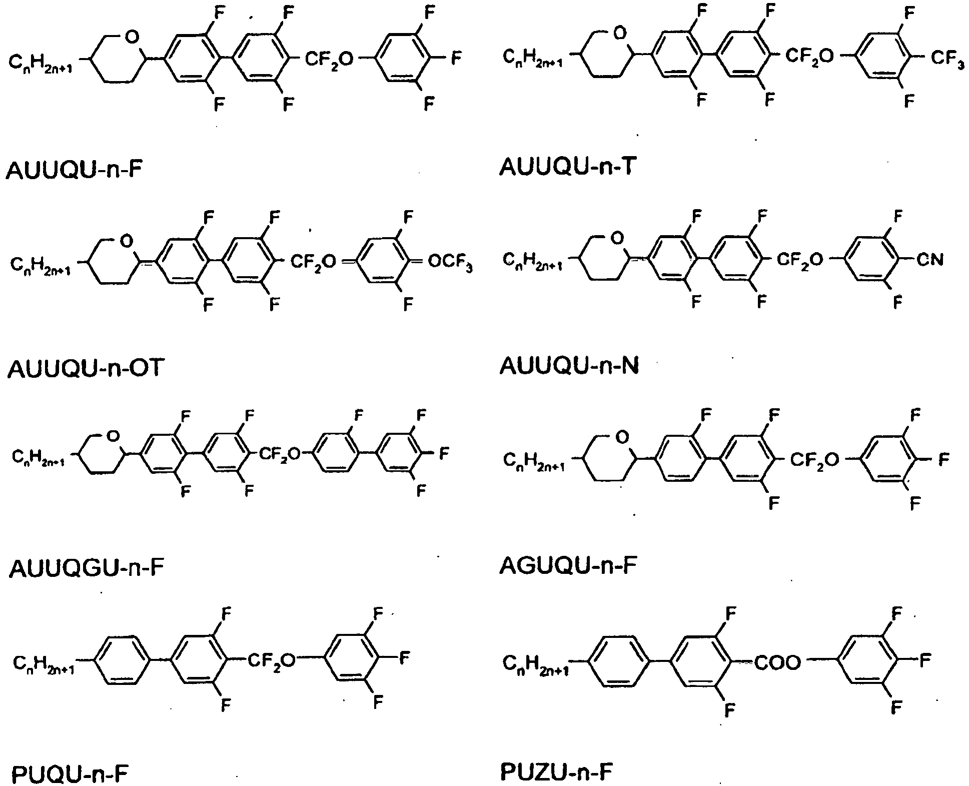

In einer bevorzugten Ausführungsform der vorliegenden Erfindung enthalten die erfindungsgemäßen FK-Medien eine oder mehrere Verbindungen ausgewählt aus der Gruppe bestehend aus Verbindungen der Tabelle A.

**Tabelle B**

| | |
|---|---|
| In der Tabelle B werden mögliche chirale Dotierstoffe angegeben, die den erfindungsgemäßen FK-Medien zugesetzt werden können. | |
| 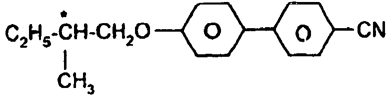 | 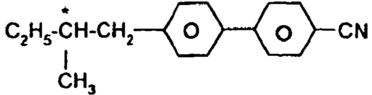 |
| **C 15** | **CB 15** |
| 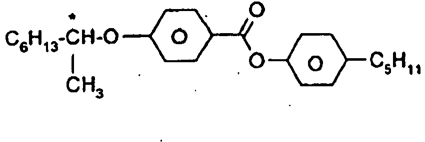 | 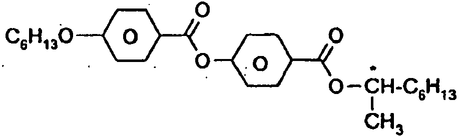 |
| **CM 21** | **R/S-811** |
| 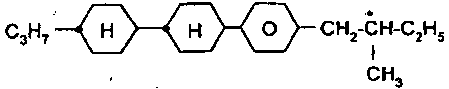 | 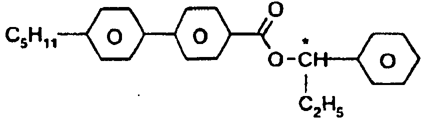 |
| **CM 44** | **CM 45** |
| 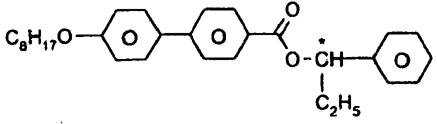 | 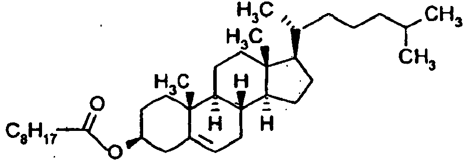 |
| **CM 47** | **CN** |
| 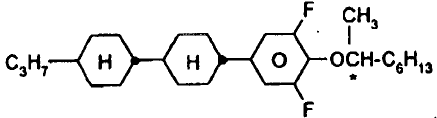 | 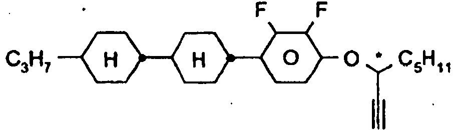 |
| **R/S-2011** | **R/S-3011** |
| 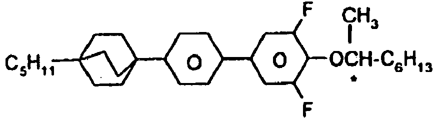 | 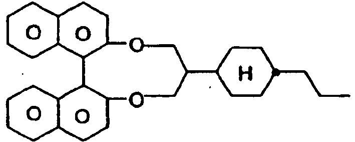 |
| **R/S-4011** | **R/S-5011** |
| 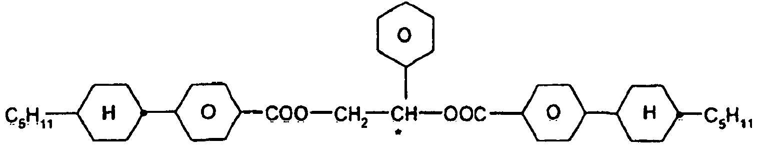 | |
| **R/S-1011** | |

Vorzugsweise enthalten die FK-Medien 0 bis 10 Gew.%, insbesondere 0,01 bis 5 Gew.%, besonders bevorzugt 0,1 bis 3 Gew.% an Dotierstoffen. Vorzugsweise enthalten die FK-Medien einen oder mehrere Dotierstoffe ausgewählt aus der Gruppe bestehend aus Verbindungen der Tabelle B.

**Tabelle C**

| | |
|---|---|
| In der Tabelle C werden mögliche Stabilisatoren angegeben, die den erfindungsgemäßen FK-Medien zugesetzt werden können. (n bedeutet hier eine ganze Zahl von 1 bis 12, vorzugsweise 1, 2, 3, 4, 5, 6, 7 oder 8, endständige Methylgruppen sind nicht gezeigt). | |
| 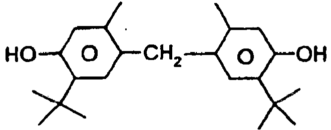 | 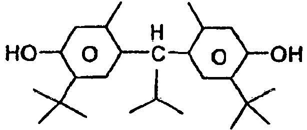 |
| 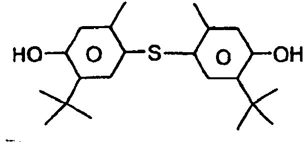 | 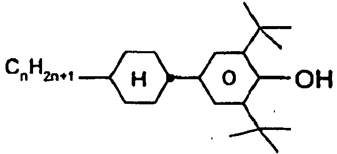 |
| 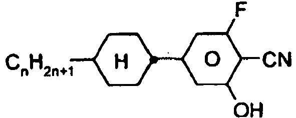 | 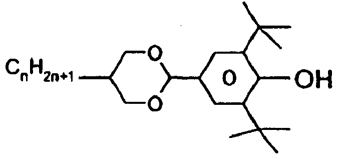 |
| 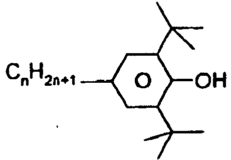 | 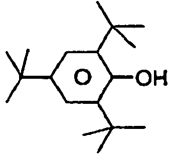 |
| 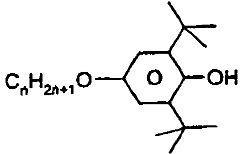 | 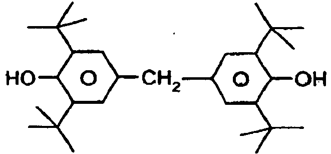 |
| 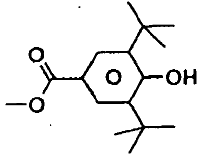 | 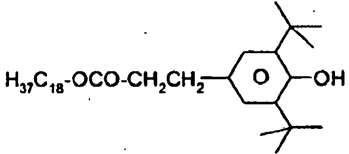 |
| 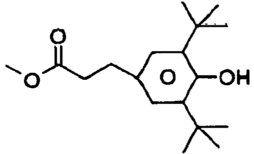 | 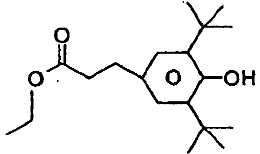 |
| 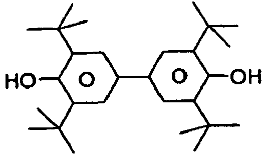 | 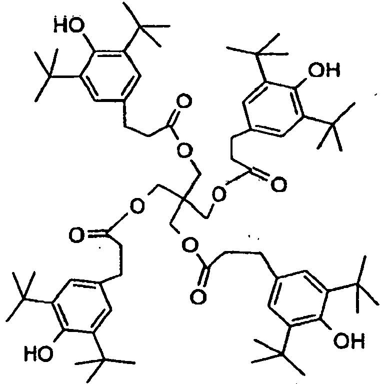 |
| 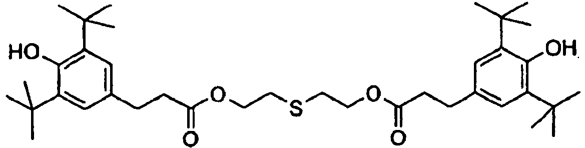 | |
| 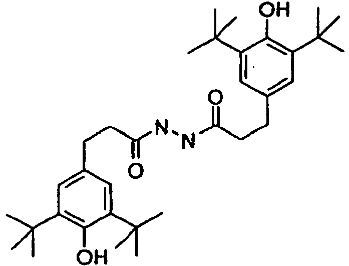 | 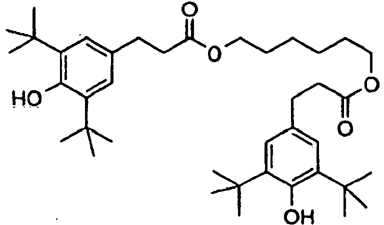 |
| 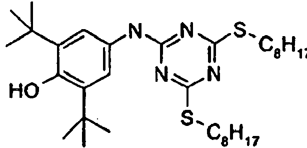 | 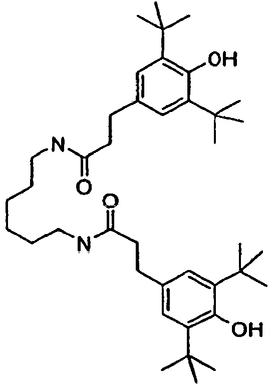 |
| 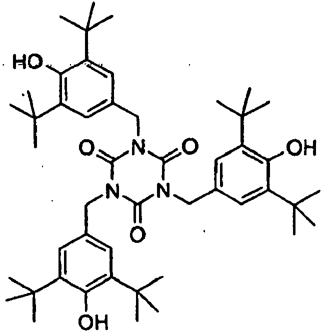 | 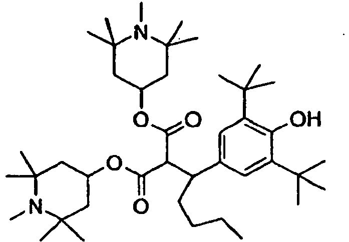 |
| 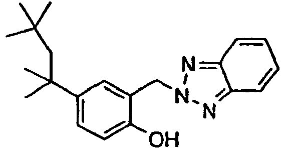 | 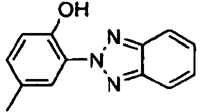 |
| 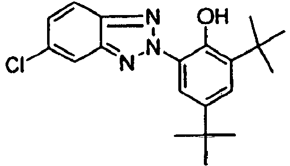 | 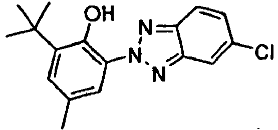 |
| 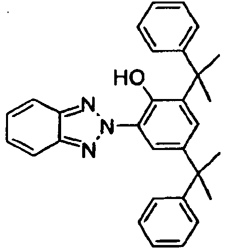 | 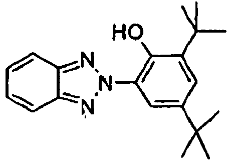 |
| 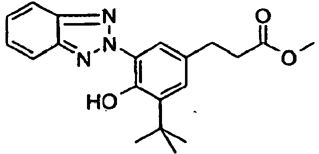 | 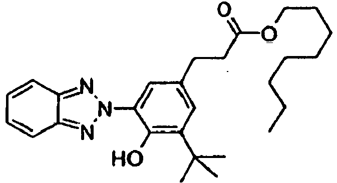 |
| 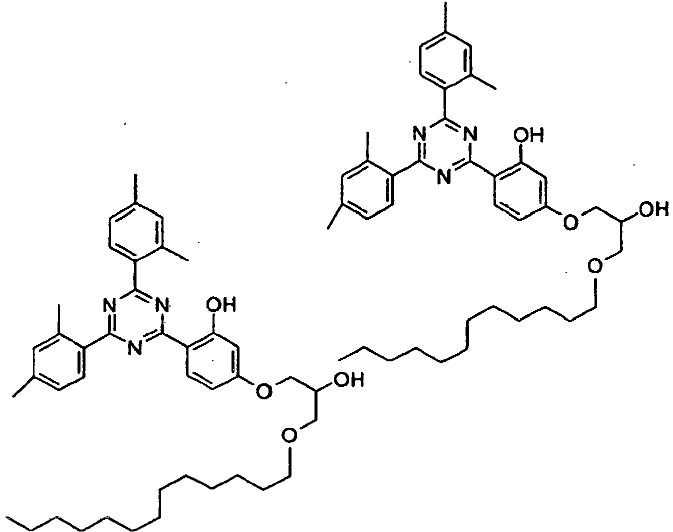 | |
| 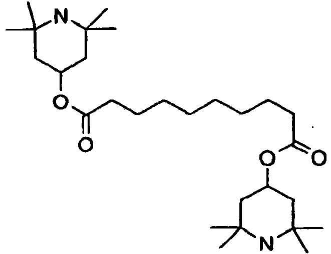 | 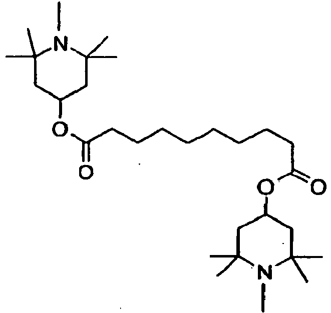 |

Vorzugsweise enthalten die FK-Medien 0 bis 10 Gew.%, insbesondere 1 ppm bis 5 Gew.%, besonders bevorzugt 1 ppm bis 1 Gew.% an Stabilisatoren. Vorzugsweise enthalten die FK-Medien einen oder mehrere Stabilisatoren ausgewählt aus der Gruppe bestehend aus Verbindungen der Tabelle C.

**Tabelle D**

| |
|---|
| In der Tabelle E sind Beispielverbindungen zusammengestellt, die in den FK-Medien gemäß der vorliegenden Erfindung vorzugsweise als reaktive mesogene Verbindungen verwendet werden können. |
| 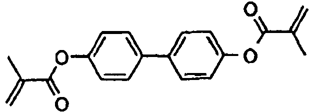 |
| **RM-1** |
| 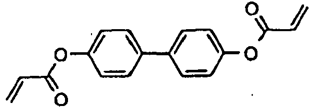 |
| **RM-2** |
| 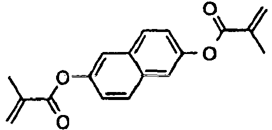 |
| **RM-3** |
| 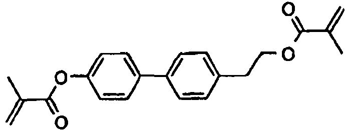 |
| **RM-4** |
| 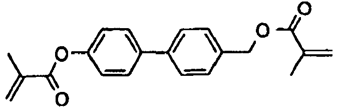 |
| **RM-5** |
| 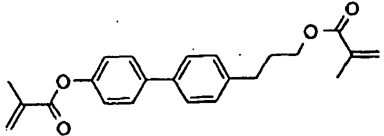 |
| **RM-6** |
| 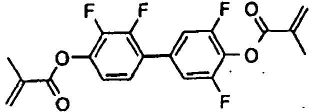 |
| **RM-7** |
| 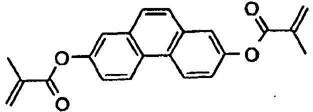 |
| **RM-8** |
| 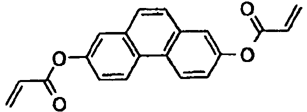 |
| **RM-9** |
| 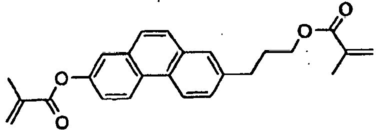 |
| **RM-10** |
| 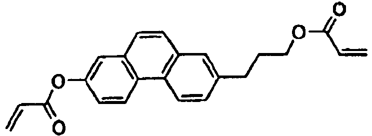 |
| **RM-11** |
| 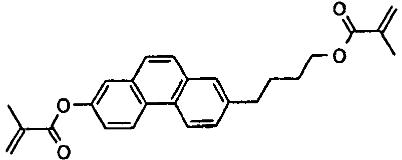 |
| **RM-12** |
| 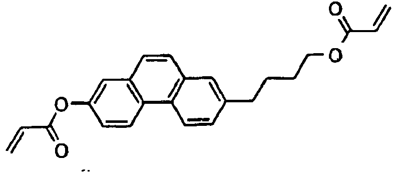 |
| **RM-13** |
| 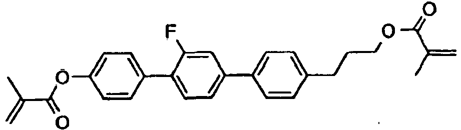 |
| **RM-14** |
| 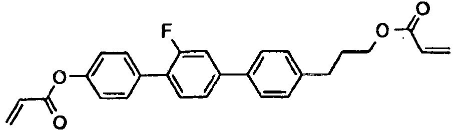 |
| **RM-15** |
| 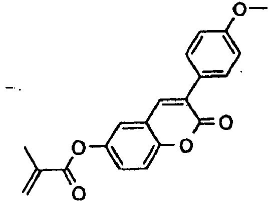 |
| **RM-16** |
| 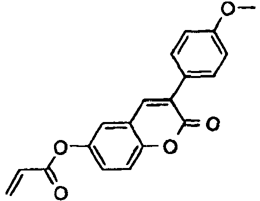 |
| **RM-17** |
| 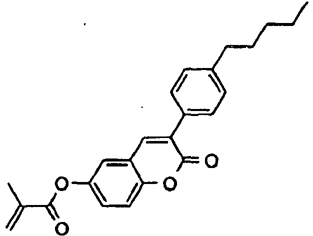 |
| **RM-18** |
| 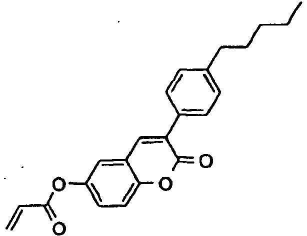 |
| **RM-19** |
| 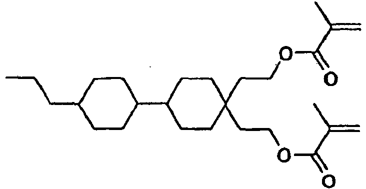 |
| **RM-20** |
| 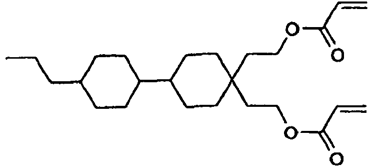 |
| **RM-21** |
| 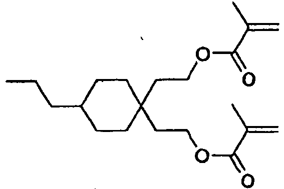 |
| **RM-22** |
| 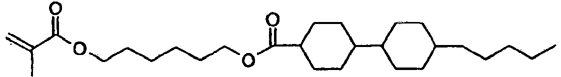 |
| **RM-23** |
| 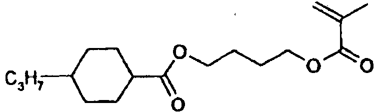 |
| **RM-24** |
| 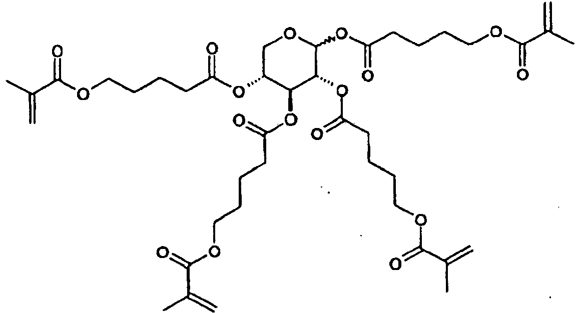 |
| **RM-25** |
| 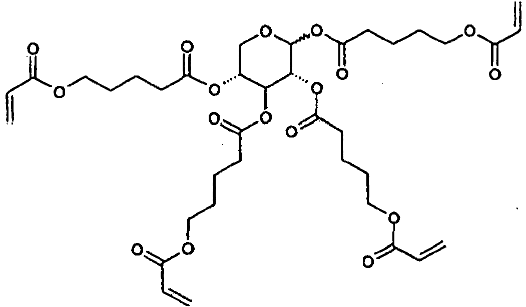 |
| **RM-26** |
| 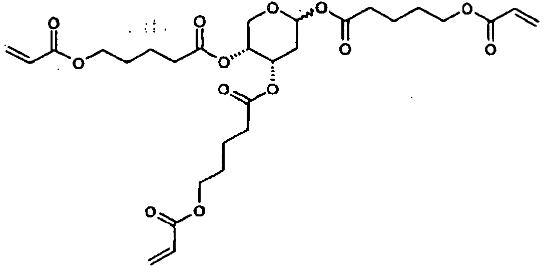 |
| **RM-27** |
| 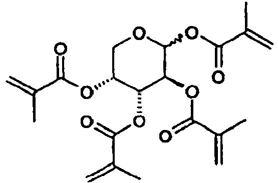 |
| **RM-28** |

In einer bevorzugten Ausführungsform der vorliegenden Erfindung enthalten die mesogenen Medien eine oder mehrere Verbindungen ausgewählt aus der Gruppe der Verbindungen der Tabelle D.

Außerdem werden folgende Abkürzungen und Symbole verwendet:
- Vₒ: Schwellenspannung, kapazitiv [V] bei 20°C,
- nₑ: außerordentlicher Brechungsindex bei 20°C und 589 nm,
- nₒ: ordentlicher Brechungsindex bei 20°C und 589 nm,
- Δn: optische Anisotropie bei 20°C und 589 nm,
- ε_{⊥}: dielektrische Permittivität senkrecht zum Direktor bei 20°C und 1 kHz,
- ε_{∥}: dielektrische Permittivität parallel zum Direktor bei 20°C und 1 kHz,
- Δε: dielektrische Anisotropie bei 20°C und 1 kHz,
- Kp., T(N,I): Klärpunkt [°C],
- γ₁: Rotationsviskosität bei 20°C [mPa·s],
- K₁: elastische Konstante, "splay"-Deformation bei 20°C [pN],
- K₂: elastische Konstante, "twist"-Deformation bei 20°C [pN],
- K₃: elastische Konstante, "bend"-Deformation bei 20°C [pN].

Soweit nicht explizit anders vermerkt, sind in der vorliegenden Anmeldung alle Konzentrationen in Gewichtsprozent angegeben und beziehen sich auf die entsprechende Gesamtmischung, enthaltend alle festen oder flüssigkristallinen Komponenten, ohne Lösungsmittel.

Soweit nicht explizit anders vermerkt, sind in der vorliegenden Anmeldung alle angegebenen Werte für Temperaturen, wie z. B. der Schmelzpunkt T(C,N), der Übergang von der smektischen (S) zur nematischen (N) Phase T(S,N) und der Klärpunkt T(N,I), in Grad Celsius (°C) angegeben. Fp. bedeutet Schmelzpunkt, Kp. = Klärpunkt. Ferner bedeuten Tg = Glaszustand, K = kristalliner Zustand, N = nematische Phase, S = smektische Phase und 1 = isotrope Phase. Die Angaben zwischen diesen Symbolen stellen die Übergangstemperaturen dar.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C und Δn wird bei 589 nm und Δε bei 1 kHz bestimmt, sofern nicht jeweils explizit anders angegeben.

Der Begriff "Schwellenspannung" bezieht sich für die vorliegende Erfindung auf die kapazitive Schwelle (V₀), auch Freedericksz-Schwelle genannt, sofern nicht explizit anders angegeben. In den Beispielen kann auch, wie allgemein üblich, die optische Schwelle für 10 % relativen Kontrast (V₁₀) angegeben werden.

Die zur Messung der kapazitiven Schwellenspannung verwendete Anzeige besteht aus zwei planparallelen Glasträgerplatten im Abstand von 20 µm, welche auf den Innenseiten jeweils ein Elektrodenschicht sowie eine darüberliegende, ungeriebene Orientierungsschicht aus Polyimid aufweisen, die eine homöotrope Randorientierung der Flüssigkristallmoleküle bewirken.

Die zur Messung der Tiltwinkel verwendete Anzeige bzw. Testzelle besteht aus zwei planparallelen Glasträgerplatten im Abstand von 4 µm, welche auf den Innenseiten jeweils eine Elektrodenschicht sowie eine darüberliegende Orientierungsschicht aus Polyimid aufweisen, wobei die beiden Polyimidschichten antiparallel zueinander gerieben werden und eine homöotrope Randorientierung der Flüssigkristallmoleküle bewirken.

Die polymerisierbaren Verbindungen werden in der Anzeige bzw. Testzelle durch Bestrahlung mit UVA-Licht (üblicherweise 365nm) einer definierten Intensität für eine vorgegebene Zeit polymerisiert, wobei gleichzeitig eine Spannung an die Anzeige angelegt wird (üblicherweise 10V bis 30V Wechselstrom, 1 kHz). In den Beispielen wird, falls nicht anders angegeben, eine Quecksilberdampflampe mit 50 mW/cm² verwendet, die Intensität wird mit einem Standard-UV-Meter (Fabrikat Ushio UNI meter) gemessen, der mit einem Bandpassfilter bei 365nm ausgerüstet ist.

Der Tiltwinkel wird per Drehkristall-Experiment (Autronic-Melchers TBA-105) bestimmt. Ein niedriger Wert (d.h. eine große Abweichung vom 90°-Winkel) entspricht dabei einem großen Tilt.

Der VHR -Wert wird wie folgt gemessen: Zur FK-Host-Mischung werden 0.3% einer polymerisierbaren monomeren Verbindung zugesetzt, und die dadurch entstandene Mischung in TN-VHR-Testzellen gefüllt (90° gerieben, Orientierungsschicht TN-Polyimid, Schichtdicke d ≈ 6 µm). Der HR-Wert wird nach 5min bei 100°C vor und nach 2h UV-Belastung (suntest) bei 1V, 60Hz, 64µs pulse bestimmt (Messgerät: Autronic-Melchers VHRM-105).

Zur Untersuchung der Tieftemperaturstabilität, auch als "LTS" (low temperature stability) bezeichnet, d.h. der Stabilität der FK-Mischung gegen spontane Auskristallisation einzelner Komponenten bei tiefen Temperaturen, werden Fläschchen mit 1g FK/RM-Mischung bei -10°C eingelagert und es wird regelmäßig überprüft, ob die Mischungen auskristallisiert waren.

Die sogenannte "HTP" ("helical twisting power") bezeichnet das helikale Verdrillungsvermögen einer optisch aktiven bzw. chiralen Substanz in einem FK-Medium (in µm). Falls nicht anders angegeben, wird die HTP in der kommerziell erhältlichen nematischen FK-Hostmischung MLD-6260 (Merck KGaA) bei einer Temperatur von 20°C gemessen.

### Beispiel 1

### Verbindung (1): Acrylsäure-5-{4-[(4-{[4-(5-acryloyloxy-pent-1-inyl)-phenyl]-difluor-methoxy}-3-methyl-phenoxy)-difluor-methyl]-phenyl}-pent-4-inylester

### 1.1. 1,4-Bis-[(4-bromophenyl)-difluormethoxy]-2-methyl-benzol

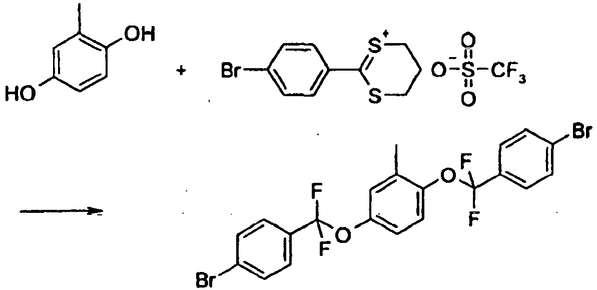

102 g (0,24 mol) 2-(4-Bromphenyl)-5,6-dihydro-4H-[1,3]dithiin-1-yliumtriflat werden in 500 ml Dichlormethan bei -70 °C vorgelegt, mit 38 ml (0,27 mol) Triethylamin versetzt und eine Lösung von 9,0 g (72,5 mmol) 2-Methylhydrochinon in 100 ml Dichlormethan hinzugefügt. Nach 30 min werden 117 ml (0,73 mol) Triethylamin-trishydrofluorid hinzugegeben und anschließend eine Lösung von 37 ml (0,72 mol) Brom in 100 ml Dichlormethan zutropfen gelassen. Anschließend wird 1 h gerührt, die Kühlung entfernt, der Ansatz auf 5 °C auftauen gelassen und auf 1 l 10proz. Natronlauge gegeben. Die wäßr. Phase wird abgetrennt und dreimal mit Dichlormethan extrahiert. Die vereinigten org. Phasen werden mit Wasser gewaschen, über Natriumsulfat getrocknet und das Lösungsmittel i. Vak. entfernt. Der Rückstand wird mit Heptan/Toluol (4:1) über Kieselgel filtriert und das Rohprodukt aus Ethanol umkristallisiert. Man erhält 1,4-Bis-[(4-bromphenyl)-difluormethoxy]-2-methyl-benzol als farblosen Feststoff.

### 1.2. 5-{4-[(4-{Difluor-[4-(5-hydroxypent-1-inyl)-phenyl]-methoxy}-3-methyl-phenoxy)-difluor-methyl]-phenyl}-pent-4-in-1-ol

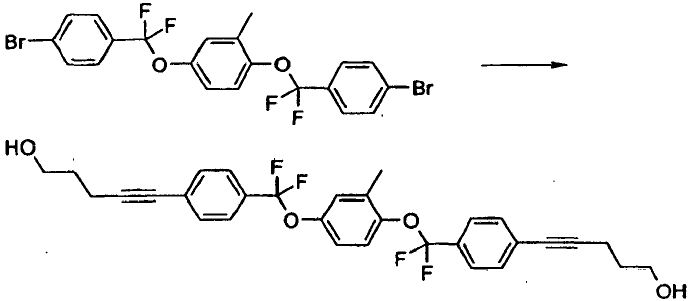

29,5 g (55,2 mmol) 1,4-Bis-[(4-bromphenyl)-difluormethoxy]-2-methyl-benzol werden in 400 ml DMF und 30 ml Triethylamin vorgelegt, mit 1 g (5,2 mmol) Kupfer(I)iodid sowie 4,0 g (5,7 mmol) Bis(triphenylphosphin)palladium(II)chlorid versetzt, auf 60 °C erwärmt und eine Lösung von 17,0 g (0,2 mol) 1-Pentinol in 100 ml DMF hinzugefügt. Der Ansatz wird über Nacht bei 75 °C rühren gelassen, mit MTB-Ether versetzt und zweimal mit Wasser gewaschen. Die wäßrige Phase wird mit MTB-Ether extrahiert, die vereinigten org. Phasen mit ges. Natriumchloridlösung gewaschen und über Natriumsulfat getrocknet. Das Lösungsmittel wird i. Vak. entfernt und der Rückstand aus Heptan/Toluol (1:1) umkristallisiert. Man erhält 5-{4-[(4-{Difluor-[4-(5-hydroxypent-1-inyl)-phenyl]-methoxy}-3-methyl-phenoxy)difluor-methyl]-phenyl}pent-4-in-1-ol als farblosen Feststoff.

### 1.3 Acrylsäure-5-{4-[(4-{[4-(5-acrylovloxy-pent-1-inyl)-phenyl]-difluormethoxy}-3-methyl-phenoxy)-difluor-methyl]-phenyl}-pent-4-inylester

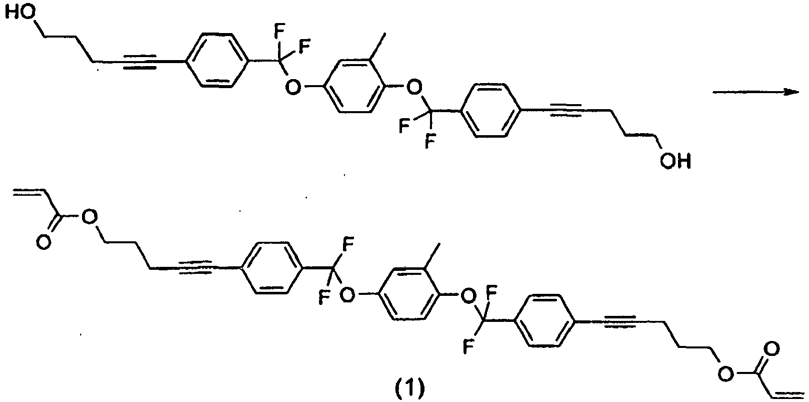

2,20 g (4,07 mmol) 5-{4-[(4-{Difluor-[4-(5-hydroxypent-1-inyl)-phenyl]-methoxy}-3-methyl-phenoxy)-difluor-methyl]-phenyl}-pent-4-in-1-ol werden in 70 ml Dichlormethan vorgelegt und unter Eiskühlung mit 2,5 ml (18 mmol) Triethylamin und anschließend mit 1,2 ml (15 mmol) Acrylsäurechlorid in 10 ml Dichlormethan versetzt. Die Kühlung wird entfernt und der Ansatz 2 h bei Raumtemp. rühren gelassen. Das Lösungsmittel wird i. Vak. entfernt und der Rückstand mit Hepten(MTB-Ether (3:2) an Kieselgel chromatographiert. Man erhält den Acrylsäure-5-{4-[(4-{[4-(5-acryloyloxy-pent-1-inyl)-phenyl]-difluor-methoxy}-3-methyl-phenoxy)-difluor-methyl]-phenyl}-pent-4-inylester als farblosen Feststoff vom Schmp. 51 °C.
Phasenverhalten Tg -50 K 51 l.

### Beispiel 2

### Verbindung (2): Methacrylsäure-5-{4-[4-[(4{[4-(5-methacryloyloxy-pentyl)-ehenyl]-difluormethoxy}-3-methyl-phenoxy)-difluormethyl]-phenyl}-pentylester

### 2.1 5-{4[(4-{Difluor-[4-(5-hydroxypentyl)-phenyl]-methoxy}-2-methyl-phenoxy)-difluormethyl]-phenyl}-pentan-1-ol

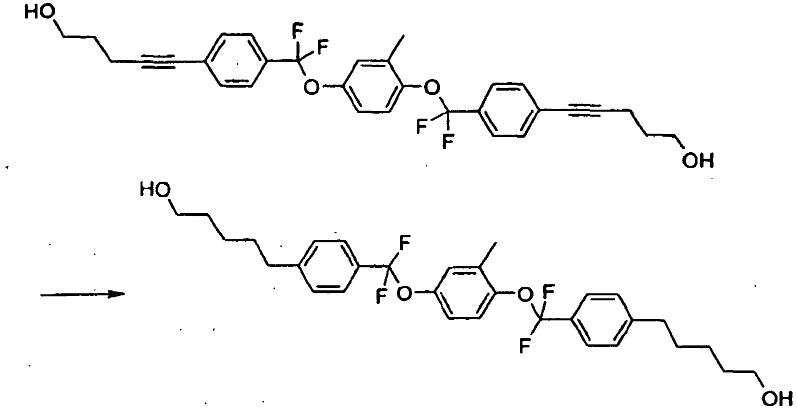

5-{4-[(4-{Difluor-[4-(5-hydroxypent-1-inyl)-phenyl]-methoxy}-3-methyl-phenoxy)-difluor-methyl]-phenyl}-pent-4-in-1-ol werden in THF an Palladium-Aktivkohlekatalysator bis zum Stillstand hydriert. Der Katalysator wird abfiltriert und das Filtrat i. Vak. eingeengt. Man erhält 5-{4-[(4-{Difluor-[4-(5-hydroxy-pentyl)-phenyl]-methoxy}-2-methyl-phenoxy)-difluormethyl]-phenyl}-pentan-1-ol, das für weitere Umsetzungen rein genug ist.

### 2.2. Methacrylsäure-5{4-[(4-{[4-(5-methacryloyloxy-pentyl)-phenyl]-difluormethoxy}-3-methyl-phenoxy)-difluormethyl]-phenyl}-pentylester

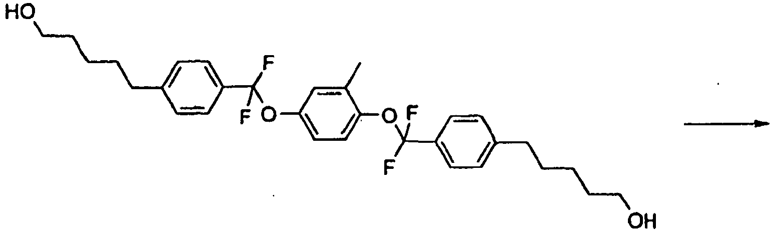

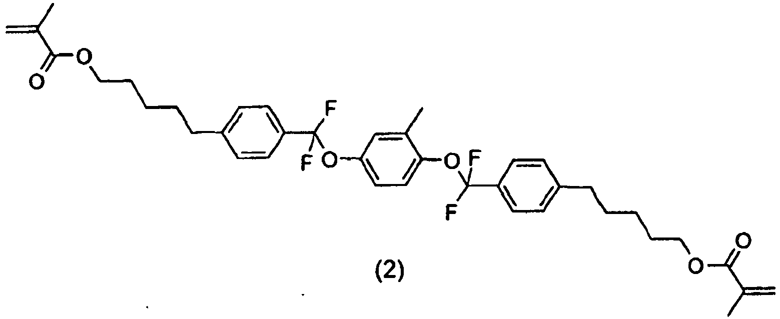

Aus 5-{4-[(4-{Difluor-[4-(5-hydroxy-pentyl)-phenyl]-methoxy}-2-methyl-phenoxy)-difluormethyl]-phenyl}pentan-1-ol und Methacrylsäurechlorid erhält man in Analogie zu der unter 1.3.beschriebenen Synthese den Methacrylsäure-5-{4-[(4-{[4-(5-methacryloytoxy-pentyl)-phenyl]-dilluormethoxy}-3-methyl-phenoxy)-difluormethyl]-phenyl}-pentylester als farblosen Feststoff vom Schmp. 26 °C.
Phasenverhalten Tg -56 K 26 N (-11) l

### Beispiel 3-25

Die folgenden erfindungsgemäßen Verbindungen werden in zu Beispiel 1 und 2 analoger Weise hergestellt.

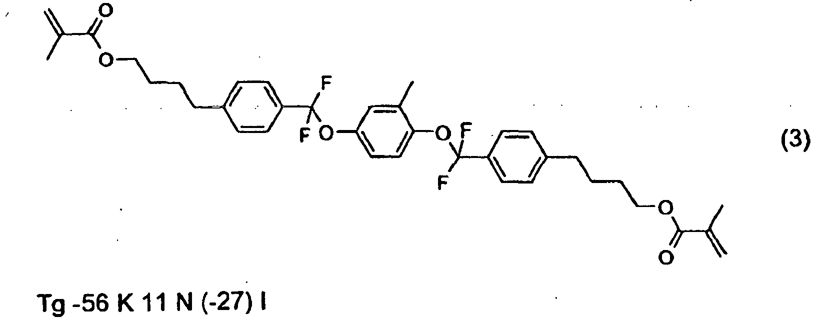

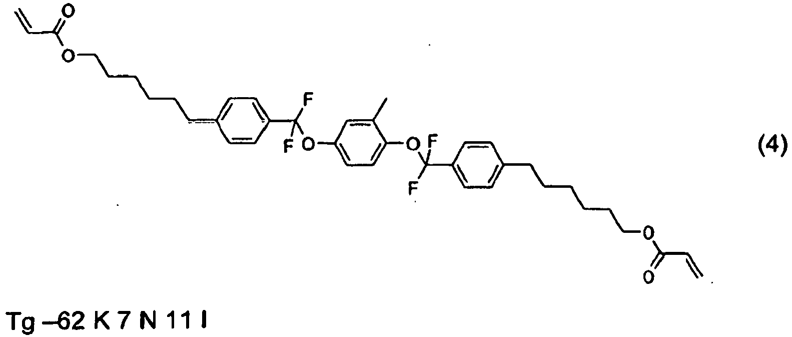

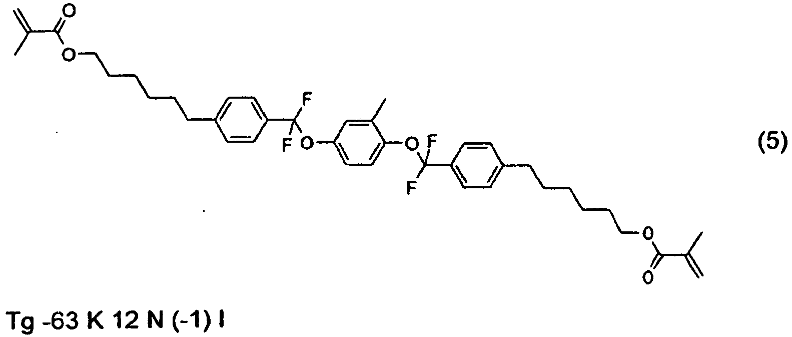

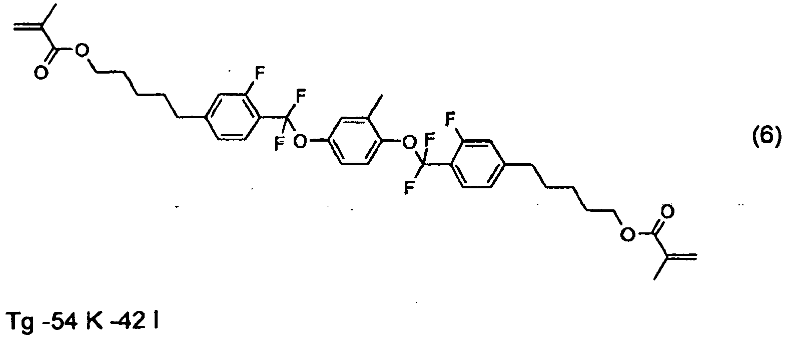

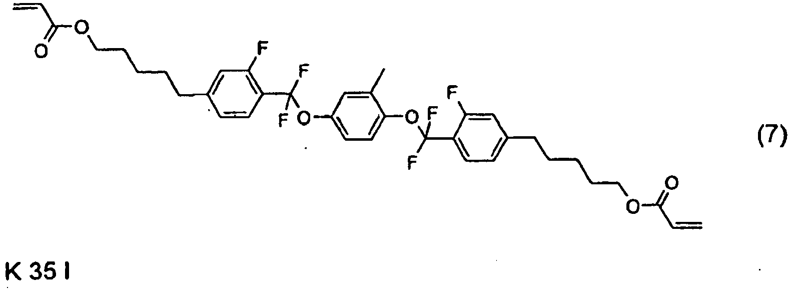

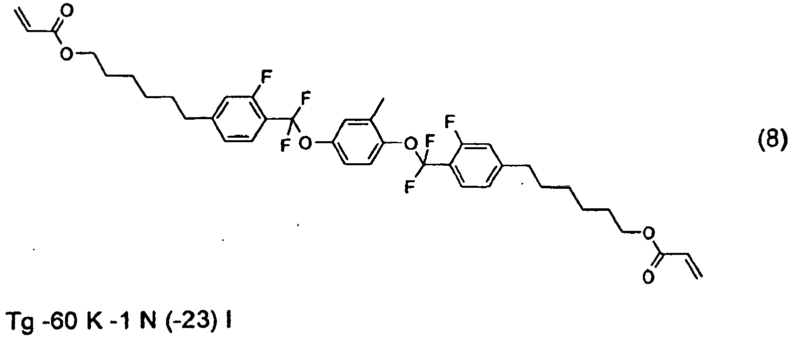

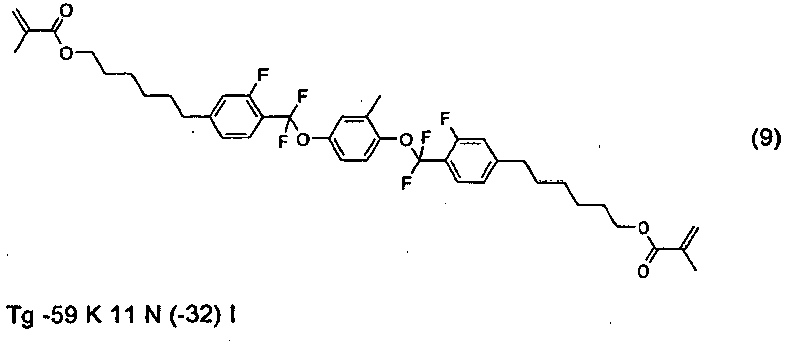

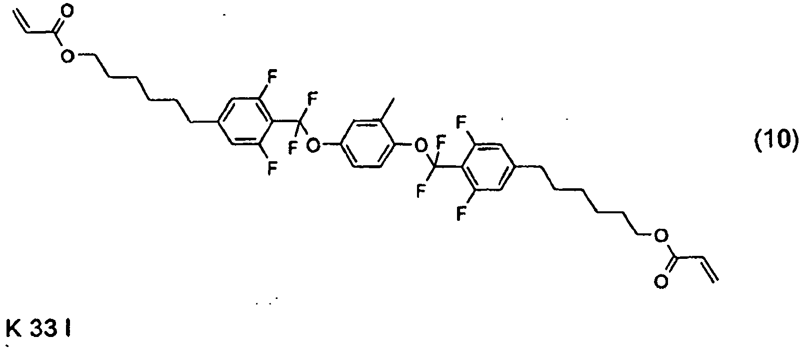

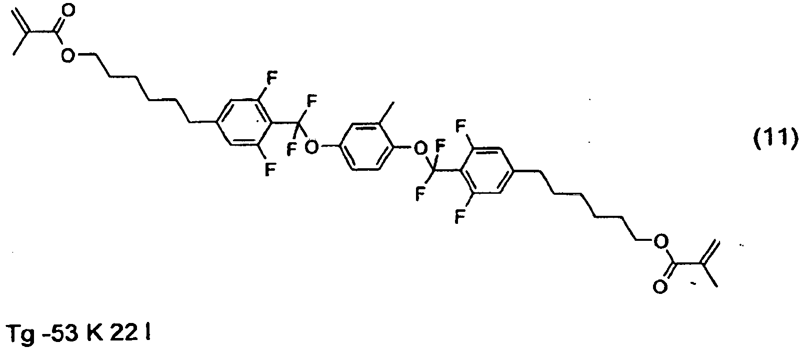

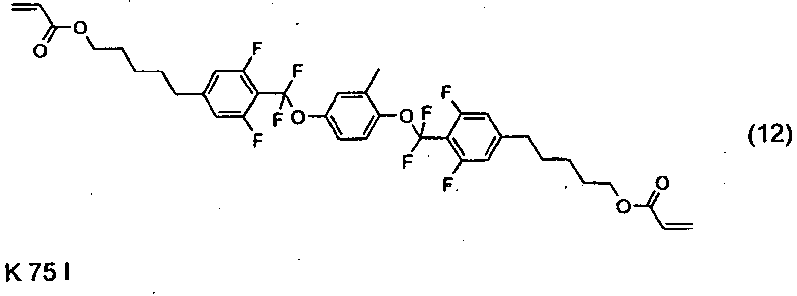

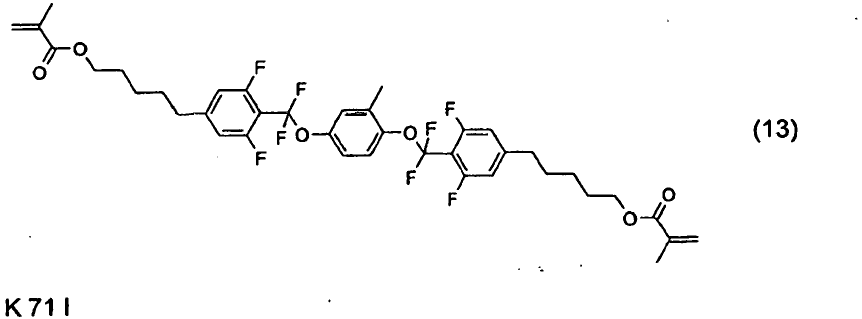

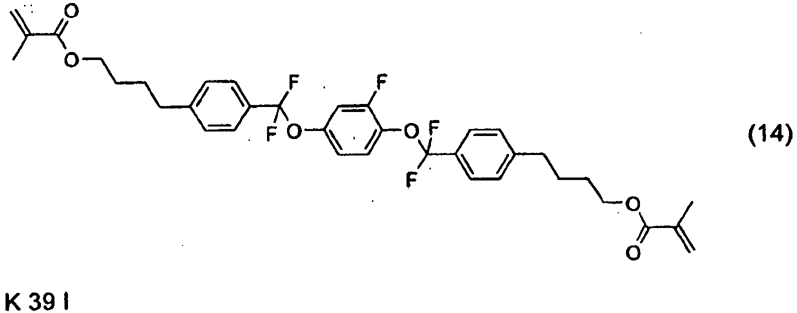

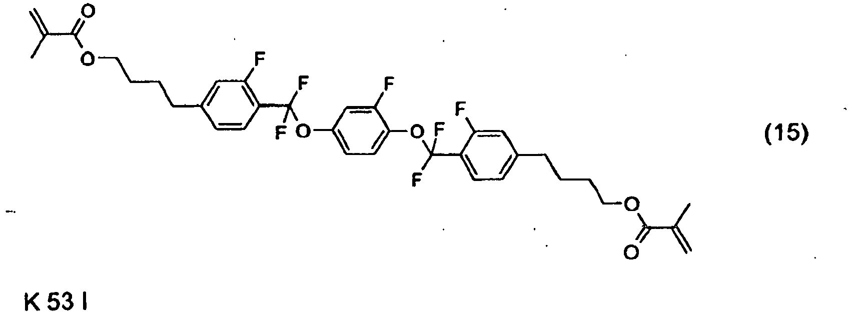

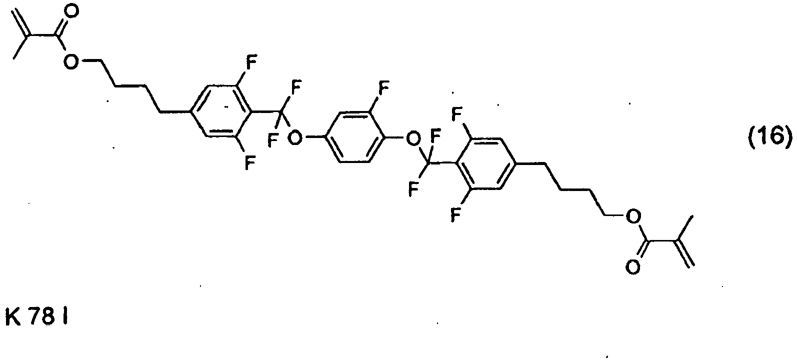

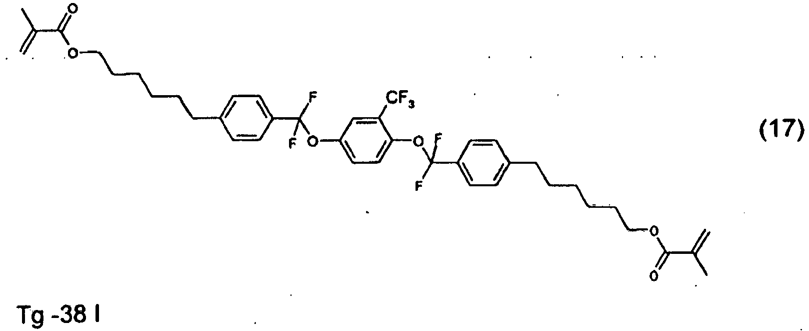

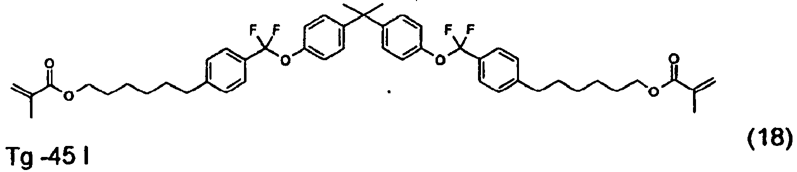

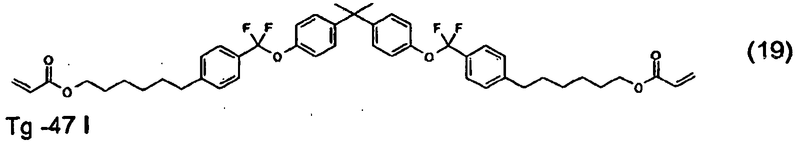

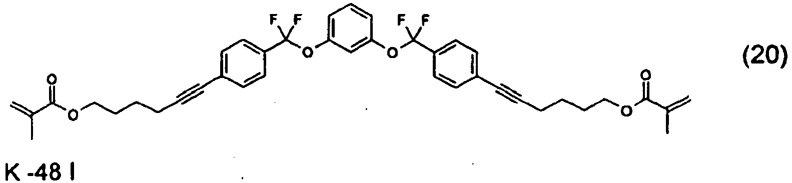

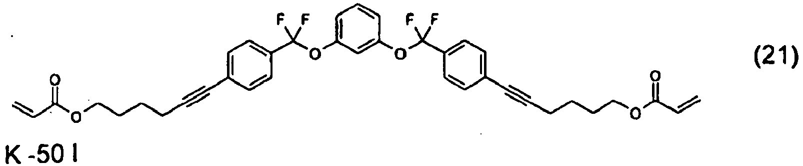

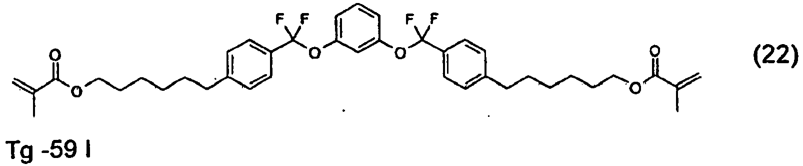

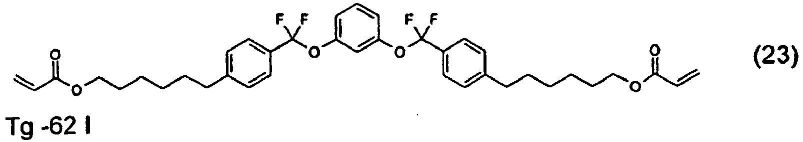

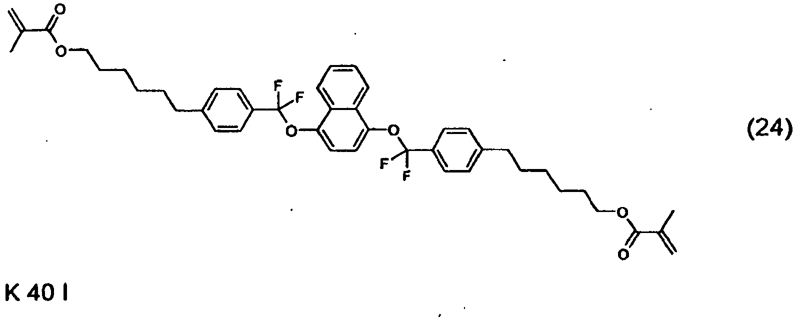

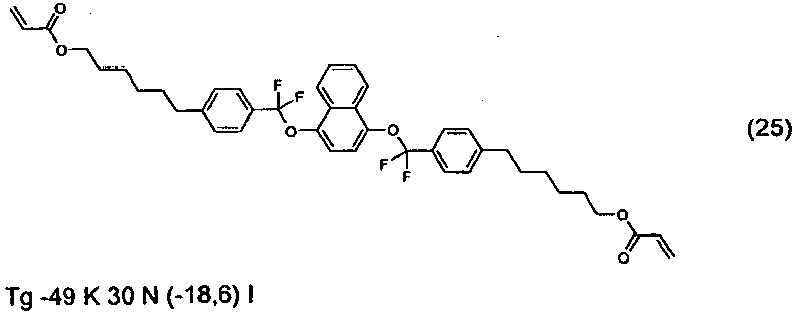

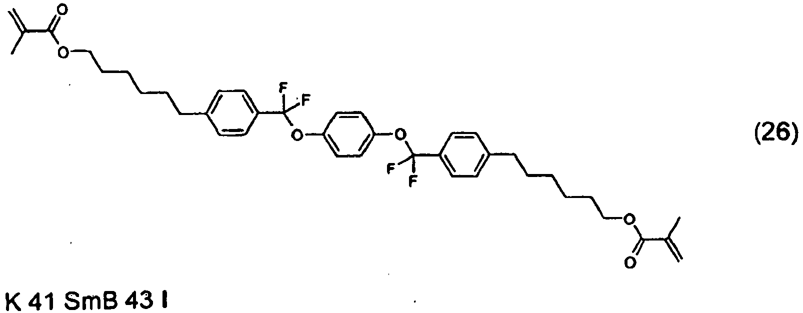

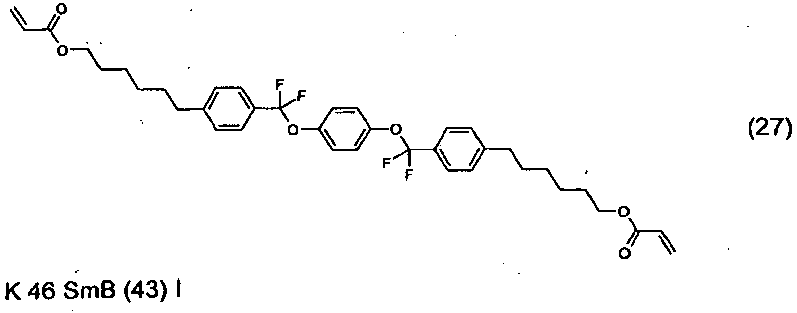

### Anwendungsbeispiel 1

Die folgenden Monomere werden verwendet: 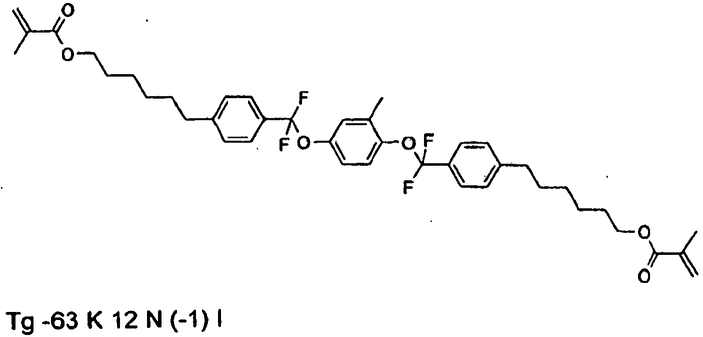 **Monomer (5) aus Beispiel 5**

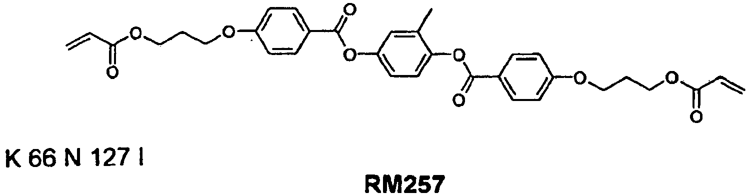

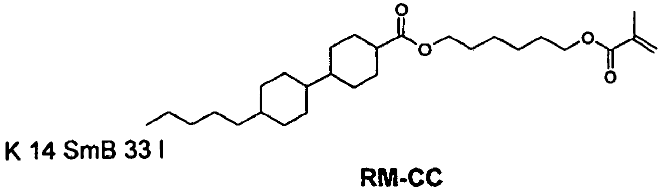

Die folgenden Zusatzstoffe werden verwendet:

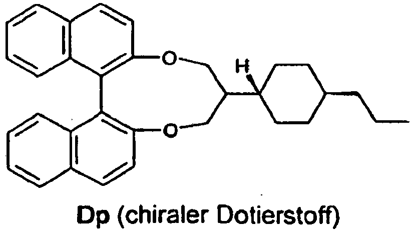

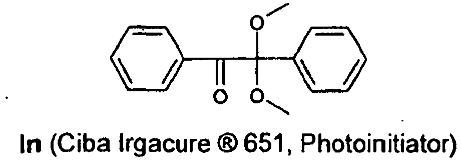

Die folgende Basismischung (Host) H1 wird verwendet:

| Zusammensetzung | | Eigenschaften | |
|---|---|---|---|
| Komponente | Anteil | T(N, I): | 66,6 °C |
| Akronym | Gew.-% | Δn (20°C, 589 nm): | 0.148 |
| PUQU-3-F | 5.00 | | |
| AGUQU-3-F | 13.00 | | |
| AUUQU-2-F | 6.00 | | |
| AUUQU-3-F | 10.00 | | |
| AUUQU-4-F | 6.00 | | |
| AUUQU-5-F | 9.00 | | |
| AUUQU-7-F | 6.00 | | |
| AUUQU-3-T | 8.00 | | |
| AUUQU-3-OT | 12.00 | | |
| PUZU-2-F | 6.00 | | |
| PUZU-3-F | 10.00 | | |
| PUZU-5-F | 9.00 | | |
| Σ | 100.00 | | |

Aus der Basismischung H1 werden durch Beimischung der oben aufgeführten Monomere und Zusatzstoffe die erfindungsgemäße polymerisierbare FK-Mischung M1, enthaltend das erfindungsgemäße Monomer (5) aus Beispiel 5, sowie die Vergleichsmischungen V1-V3, enthaltend das Monomer RM 257 aus dem Stand der Technik, hergestellt. Die Zusammensetzungen der Mischungen sind in Tabelle 1 gezeigt.

**Tabelle 1**

| | **M1** | **V1** | **V2** | **V3** |
|---|---|---|---|---|
| **Komponente** | **Anteil [Gew.-%]** | | | |
| H1 | 85 | 85 | 85 | 86.3 |
| Dp | 3,8 | 3,8 | 3,8 | 2,5 |
| In | 0,2 | 0,2 | 0,2 | 0,2 |
| (5) | 6,0 | 0 | 0 | 0 |
| RM257 | 0 | 6,0 | 5,0 | 5,0 |
| RM-CC | 5,0 | 5,0 | 6,0 | 6,0 |

Die Mischungen werden vor der Polymerisation wie nachfolgend beschrieben charakterisiert. Danach werden die reaktiven Komponenten durch einmalige Bestrahlung (180 s) in der blauen Phase polymerisiert, und die erhaltenen Medien werden erneut charakterisiert.

### Beschreibung der Polymerisation

Vor der Polymerisation einer Probe werden die Phaseneigenschaften des Mediums in einer Testzelle von ca. 10 Mikrometer Dicke und einer Fläche von 2x2,5 cm festgestellt. Die Füllung erfolg durch Kapillarwirkung bei einer Temperatur von 75 °C. Die Messung erfolgt unter einem Polarisationsmikroskop mit Heiztisch bei einem Temperaturverlauf von 1 °C/min.

Die Polymerisation der Medien wird durch Bestrahlung mit einer UV-Lampe (Dymax, Bluewave 200, 365 nm Interferenzfilter) mit einer effektiven Leistung von ca. 3,0 mW/cm² für 180 Sekunden durchgeführt. Die Polymerisation erfolgt direkt in der elektrooptischen Testzelle.

Die Polymerisation erfolgt anfangs bei einer Temperatur, in der das Medium in der blauen Phase I (BP-I) vorliegt. Die Polymerisation erfolgt in mehreren Teilschritten, die nach und nach zu einer vollständigen Polymerisation führen. Der Temperaturbereich der blauen Phase ändert sich in der Regel während der Polymerisation. Zwischen jedem Teilschritt wird daher die Temperatur so angepasst, dass das Medium nach wie vor in der blauen Phase vorliegt. In der Praxis kann dies so erfolgen, dass nach jedem Bestrahlungsvorgang von ca. 5 s oder länger die Probe unter dem Polarisationsmikroskop beobachtet wird. Wird die Probe dunkler, so deutet dies auf einen Übergang in die isoptrope Phase hin. Die Temperatur für den nächsten Teilschritt wird entsprechend verringert.

Die gesamte Bestrahlungszeit, die zu der maximalen Stabilisierung führt, beträgt typischerweise 180 s bei der angegebenen Bestrahlungsleistung. Weitere Polymerisationen können nach einem optimierten Bestrahlungs-Temperatur-Programm durchgeführt werden.

Alternativ kann die Polymerisation auch in einem einzigen Bestrahlungsschritt durchgeführt werden, insbesondere wenn schon vor der Polymerisation eine breite blaue Phase vorliegt.

### Elektrooptische Charakterisierung

Nach der oben beschriebenen Polymerisation und Stabilisierung der blauen Phase wird die Phasenbreite der blauen Phase bestimmt. Die elektrooptische Charakterisierung erfolgt anschließend bei verschiedenen Temperaturen innerhalb und ggf. auch außerhalb dieses Bereichs.

Die verwendeten Testzellen sind auf einer Seite mit Interdigitalelektroden auf der Zellenoberfläche ausgestattet. Der Zellspalt, der Elektrodenabstand und die Elektrodenbreite betragen typischerweise jeweils 10 Mikrometer. Dieses einheitliche Maß wird nachfolgend als Spaltbreite bezeichnet. Die mit Elektroden belegte Fläche beträgt ca. 0,4 cm². Die Testzellen besitzen keine Orientierungsschicht ('alignment layer').

Die Zelle befindet sich für die elektrooptische Charakterisierung zwischen gekreuzten Polarisationsfiltern, wobei die Längsrichtung der Elektroden einen Winkel von 45° mit den Achsen des Polarisationsfilter einnimmt. Die Messung erfolgt mit einem DMS301 (Autronic-Melchers) im rechten Winkel zur Zellebene, oder mittels einer hochempfindlichen Kamera am Polarisationsmikroskop. Im spannungslosen Zustand ergibt die beschriebene Anordnung ein im Wesentlichen dunkles Bild (Definition 0 % Transmission).

Zuerst werden die charakteristischen Betriebsspannungen und dann die Schaltzeiten an der Testzelle gemessen. Die Betriebsspannung an den Zellelektroden wird in Form von Rechteckspannung mit alternierendem Vorzeichen (Frequenz 100 Hz) und variabler Amplitude wie im Folgenden beschrieben angelegt.

Während die Betriebspannung erhöht wird, wird die Transmission gemessen. Das Erreichen des Maximalwerts der Transmission legt die charakteristische Größe der Betriebsspannung V₁₀₀ fest. Gleichermaßen wird die charakteristische Spannung V₁₀ bei 10 % der maximalen Transmission bestimmt. Diese Werte werden bei verschiedenen Temperaturen im Bereich der blauen Phase gemessen.

Am oberen und unteren Ende des Temperaturbereichs der blauen Phase werden relativ hohe charakteristische Betriebsspannungen V₁₀₀ beobachtet. Im Bereich der minimalen Betriebsspannung steigt V₁₀₀ in der Regel nur langsam mit der Temperatur. Dieser Temperaturbereich, begrenzt durch T₁ und T₂ wird als nutzbarer, flacher Temperaturbereich (FB) bezeichnet. Die Breite dieses "Flachbereichs" (FB) beträgt (T₂ - T₁) und wird als Breite des Flachbereichs (BFB) (engl. 'flat range') bezeichnet. Die genauen Werte von T₁ und T₂ werden durch die Schnittpunkte von Tangenten an den flachen Kurvenabschnitt FB und die benachbarten steilen Kurvenabschnitte im V₁₀₀-Temperatur-Diagramm ermittelt.

Im zweiten Teil der Messung werden die Schaltzeiten beim Ein- und Ausschalten ermittelt (τₒₙ, τ_{off}). Die Schaltzeit τₒₙ ist definiert durch die Zeit bis zum Erreichen von 90 % Intensität nach dem Anlegen einer Spannung der Höhe von V₁₀₀ bei der gewählten Temperatur. Die Schaltzeit τ_{off} ist definiert durch die Zeit bis zur Abnahme um 90 % ausgehend von maximaler Intensität bei V₁₀₀ nach dem Erniedrigen der Spannung auf 0 V. Auch die Schaltzeit wird bei verschiedenen Temperaturen im Bereich der blauen Phase ermittelt.

Als weitere Charakterisierung wird bei einer Temperatur innerhalb des FB die Transmission bei kontinuierlich steigender und fallender Betriebsspannung zwischen 0 V und V₁₀₀ gemessen. Den Unterschied zwischen beiden Kurven bezeichnet man als Hysterese. Die Differenz der Transmissionen bei 0,5·V₁₀₀ bzw. die Differenz der Spannungen bei 50 % Transmission sind beispielsweise charakteristische Hysteresewerte und werden als ΔT₅₀ respektive ΔV₅₀ bezeichnet. Ferner unterscheidet man den Kontrast beim ersten Einschalten und beim folgenden Ausschalten als Verhältnis der jeweils maximalen und der minimalen Transmission.

Die Ergebnisse sind in Tabelle 2 zusammengefasst.

**Tabelle 2**

| **Messwerte** | **M1** | **V1** | **V2** | **V3** |
|---|---|---|---|---|
| Übergangspunkt vor der Polymerisation | 32,6 | 44,1 | 44,1 | 49,6 |
| Temperaturbereich der blauen Phase | - | - | - | - |
| V₁₀ (20 °C) | | | | |
| V₁₀₀ (20 °C) | 74,6 V | 67,9 V | 80,5 V | 55,3 |
| ΔV₅₀ (20°C) | 5,7 | 6,5 | 6,9 | 4,2 |
| Kontrast, Einschalten | 403 | 500 | 494 | 45 |
| Kontrast, Ausschalten | 371 | 33 | 68 | 50 |
| Spaltbreite | 10 µm | 10 µm | 10 µm | 10 µm |

Das polymerstabilisierte Medium M1, hergestellt unter Verwendung des erfindungsgemäßen Monomers (5), zeigt eine Verringerung der Hysterese (ΔV₅₀) gegenüber den polymerstabilisierten Medien V1 und V2 sowie einen etwas niedrigeren Kontrast beim Einschalten und eine deutliche Erhöhung des Kontrastes beim Ausschalten gegenüber den polymerstabilisierten Medien V1-V3, hergestellt unter Verwendung des Monomers RM257 aus dem Stand der Technik. Insbesondere liegen im erfindungsgemäßen Medium M1 der Kontrast beim Einschalten und der Kontrast beim Ausschalten nahe beieinander, was eine sehr gute Stabilisierung der blauen Phase bedeutet. Bei der zur Mischung V2 analogen Mischung V3, die weniger chiralen Dotierstoff enthält, ist der Kontrast beim Ein- und Ausschalten ebenfalls sehr ähnlich. Der geringere Kontrast im Vergleich zu M1, V1 und V2 rührt daher, dass es sich bei V3 um eine sichtbare blaue Phase handelt.

Daraus ist ersichtlich, dass sich die erfindungsgemäßen Monomere besonders gut zur Stabilisierung von blauen Phasen, insbesondere bei Medien mit hoher Konzentration an chiralem Dotierstoff, eignen.

## Patentansprüche

1. Verwendung von Verbindungen der Formel I
P^{a}-(Sp^{a})ₛ₁-A²-Q¹-A¹-Q²-A³-(Sp^{b})ₛ₂-P^{b} I
worin die einzelnen Reste folgende Bedeutung besitzen
P^{a}, P^{b} jeweils unabhängig voneinander eine polymerisierbare Gruppe,
Sp^{a}, Sp^{b} jeweils unabhängig voneinander eine Abstandsgruppe,
s1, s2 jeweils unabhängig voneinander 0 oder 1,
Q¹, Q² jeweils unabhängig voneinander -CF₂O- oder -OCF₂-,
A¹, A², A³ jeweils unabhängig voneinander ein Rest ausgewählt aus folgenden Gruppen
a) der Gruppe bestehend aus trans-1,4-Cyclohexylen, 1,4-Cyclohexenylen und 1,4'-Bicyclohexylen, worin auch eine oder mehrere nicht benachbarte CH₂-Gruppen durch -O- und/oder -S- ersetzt sein können und worin auch ein oder mehrere H-Atome durch F ersetzt sein können,
b) der Gruppe bestehend aus 1,4-Phenylen und 1,3-Phenylen, worin auch eine oder zwei CH-Gruppen durch N ersetzt sein können und worin auch ein oder mehrere H-Atome durch L ersetzt sein können
c) der Gruppe bestehend aus der Gruppe bestehend aus Tetrahydropyran-2,5-diyl, 1,3-Dioxan-2,5-diyl, Tetrahydrofuran-2,5-diyl, Cylcobut-1,3-diyl, Piperidin-1,4-diyl, Thiophen-2,5-diyl und Selenophen-2,5-diyl, welche auch ein oder mehrfach durch L substituiert sein können,
d) der Gruppe bestehend aus gesättigten, teilweise ungesättigten oder vollständig ungesättigten, und optional substituierten, polycyclischen Resten mit 5 bis 20 cyclischen C-Atomen, von denen auch eines oder mehrere durch Heteroatome ersetzt sein können, vorzugsweise ausgewählt aus der Gruppe bestehend aus Bicyclo[1.1.1]pentan-1,3-diyl, Bicyclo[2.2.2]octan-1,4-diyl, Spiro[3.3]heptan-2,6-diyl, 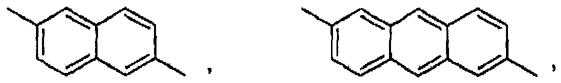 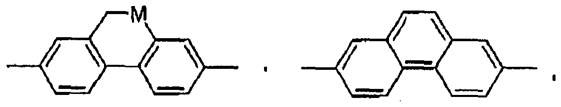 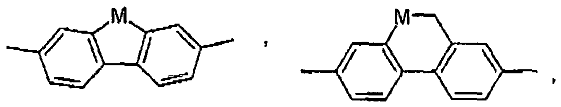 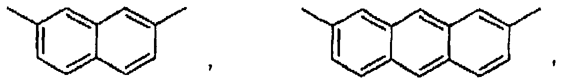 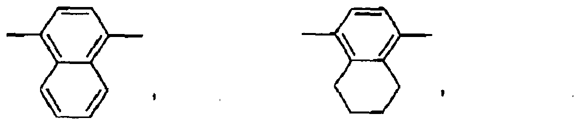 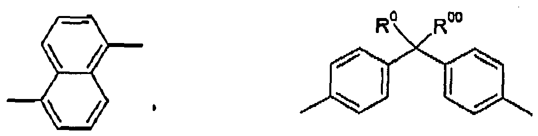 wobei in diesen Resten auch ein oder mehrere H-Atome durch L ersetzt sein können, und/oder eine oder mehrere Doppelbindungen durch Einfachbindungen ersetzt sein können, und/oder ein oder mehrere CH-Gruppen durch N ersetzt sein können,
L bei jedem Auftreten gleich oder verschieden F, Cl, CN, SCN, SF₅ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen,
R⁰, R⁰⁰ jeweils unabhängig voneinander H, F oder geradkettiges oder verzweigtes Alkyl mit 1 bis 12 C-Atomen, worin auch ein oder mehrere H-Atome durch F ersetzt sein können,
M -O-, -S-, -CH₂-, -CHY¹- oder -CY¹Y²-,
Y¹ und Y² jeweils unabhängig voneinander eine der oben für R⁰ angegebenen Bedeutungen, Cl oder CN, und vorzugsweise H, F, Cl, CN, OCF₃ oder CF₃,
oder die Verwendung eines Polymers erhältlich durch Polymerisation einer oder mehrerer Verbindungen der Formel I, in FK-Anzeigen mit blauer Phase.

2. Flüssigkristall-Medium enthaltend eine oder mehrere Verbindungen der Formel
P^{a}-(Sp^{a})ₛ₁-A²-Q¹-A¹-Q²-A³-(Sp^{b})ₛ₂-P^{b} I
worin die einzelnen Reste die in Anspruch 1 angegebenen Bedeutungen besitzen,
oder enthaltend ein Polymer erhältlich durch Polymerisation einer oder mehrerer dieser Verbindungen der Formel I.

3. Flüssigkristall-Medium nach Anspruch 2 enthaltend eine oder mehrere Verbindungen der Formel I.

4. Flüssigkristall-Medium nach Anspruch 2 enthaltend ein Polymer erhältlich durch Polymerisation einer oder mehrerer Verbindungen der Formel I.

5. Flüssigkristall-Medium nach Anspruch 3 oder 4, **dadurch gekennzeichnet, dass** es folgende Komponenten enthält
- eine polymerisierbare Komponente A, enthaltend eine oder mehrere Verbindungen der Formel
P^{a}-(Sp^{a})ₛ₁-A²-Q¹-A¹-Q²-A³-(Sp^{b})ₛ₂-P^{b} I
worin die einzelnen Reste die in Anspruch 1 angegebenen Bedeutungen besitzen oder ein daraus erhältliches Polymer, und
- eine flüssigkristalline Komponente B, enthaltend eine oder mehrere Verbindungen der Formel II 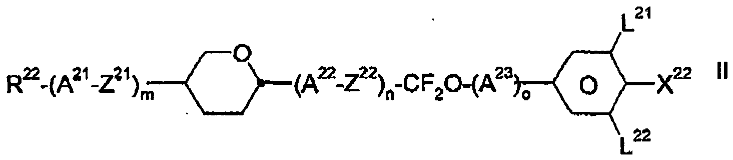 worin die einzelnen Reste folgende Bedeutung besitzen
R²² H, F, Cl, CN, NCS, SF₅, SO₂CF₃ oder geradkettiges oder verzweigtes Alkyl mit 1 bis 20 C-Atomen, welches unsubstituiert, oder durch F, Cl oder CN ein- oder mehrfach substituiert ist, und worin auch eine oder mehrere nicht benachbarte CH₂-Gruppen auch jeweils unabhängig voneinander durch -O-, -S-, -NH-, -NR⁰¹-, -SiR⁰¹R⁰²-, -CO-, -COO-, -OCO-, -OCO-O-, -S-CO-, -CO-S-, -CY⁰¹=CY⁰¹- oder -C≡C- so ersetzt sein können, dass O- und/oder S-Atome nicht direkt miteinander verknüpft sind,
Y⁰¹, Y⁰² jeweils unabhängig voneinander F, Cl oder CN, einer der Reste Y⁰¹ und Y⁰² auch H,
R⁰¹, R⁰² jeweils unabhängig voneinander H oder Alkyl mit 1 bis 12 C-Atomen,
A²¹, A²², A²³ jeweils unabhängig voneinander und bei jedem Auftreten gleich oder verschieden 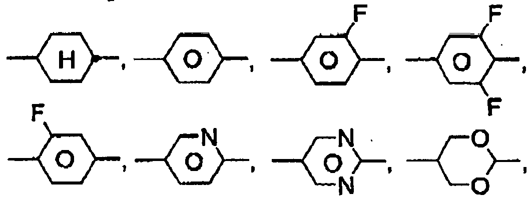
Z²¹, Z²² jeweils unabhängig voneinander, und bei jedem Auftreten gleich oder verschieden, eine Einfachbindung, -(CH₂)₄-, -CH₂CH₂-, -CF₂-CF₂-, -CF₂-CH₂-, -CH₂-CF₂-, -CH=CH-, - CF=CF-, -CF=CH-, -(CH₂)₃O-, -O(CH₂)₃-, -CH=CF-, -C≡C-, -CH₂O-, OCH₂-, -CF₂O-, -OCF₂-, -CO-O- oder -O-CO-,
X²² F, Cl, -CN, -NCS, -SF₅, -SO₂CF₃, oder Alkyl, Alkenyl, Alkenyloxy, Alkylalkoxy oder Alkoxy mit 1 bis 3 C-Atomen, welches durch F, Cl oder CN ein- oder mehrfach substituiert ist,
L²¹, L²² jeweils unabhängig voneinander H oder F,
m 0, 1 oder 2,
n 0, 1, 2 oder 3,
o 0, 1 oder 2, wobei
m + n + o 0, 1, 2 oder 3, vorzugsweise 0, 1 oder 2 bedeutet,
- und eine Komponente D, enthaltend eine oder mehrere optisch aktive und/oder chirale Verbindungen.

6. Flüssigkristall-Medium nach Anspruch 5, **dadurch gekennzeichnet, dass** es zusätzlich eine oder mehrere Verbindungen ausgewählt aus den Formeln Z, Q1 und Q2 enthält 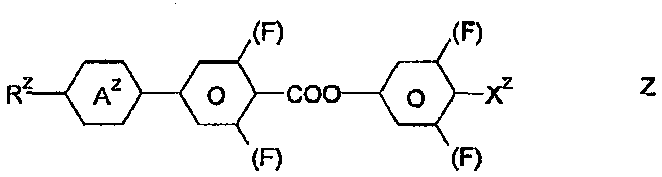 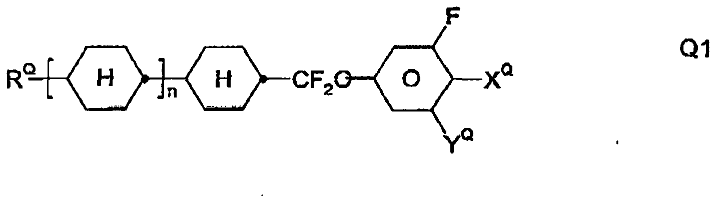 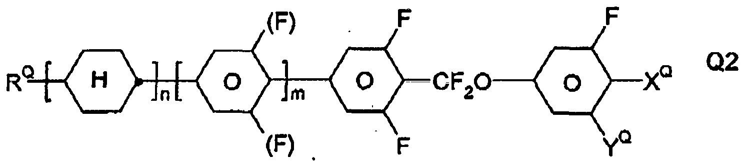 worin R² eine der für R²² in Formel II angegebenen Bedeutungen hat, 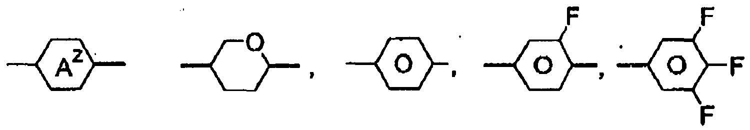
X^{Z} F, Cl, CN, NCS, OCF₃, CF₃ or SF₅ bedeutet,
R^{Q} eine der für R²² in Formel II angegebenen Bedeutungen hat, X^{Q} eine der für X^{E} in Formel E angegebenen Bedeutungen hat,
(F) F oder H bedeutet, und
n und m jeweils unabhängig voneinander 0 oder 1 bedeuten.

7. Verfahren zur Herstellung eines FK-Medlums nach einem oder mehreren der Ansprüche 2 bis 6, Indem man eine oder mehrere flüssigkristalline Verbindungen mit einer oder mehreren Verbindungen der Formel I nach Anspruch 1 mischt.

8. FK-Anzeige, enthaltend eine oder mehrere Verbindungen der Formel I
P^{a}-(Sp^{a})ₛ₁-A²-Q¹-A¹-Q²-A³-(Sp^{b})ₛ₂-P^{b} I
worin die einzelnen Reste die in Anspruch 1 angegebenen Bedeutungen besitzen oder ein daraus erhältliches Polymer oder enthaltend ein FK-Medium nach einem oder mehreren der Ansprüche 2 bis 6.

9. FK-Anzeige nach Anspruch 8, **dadurch gekennzeichnet, dass** sie eine Anzeige mit blauer Phase ist.

10. FK-Anzeige nach Anspruch 8 oder 9, enthaltend eine FK-Zelle mit zwei Substraten und zwei Elektroden, wobei mindestens ein Substrat lichtdurchlässig ist und mindestens ein Substrat eine oder zwei Elektroden aufweist, sowie einer zwischen den Substraten befindlichen Schicht eines FK-Mediums enthaltend eine polymerisierte Komponente und eine niedermolekulare Komponente, wobei die polymerisierte Komponente erhältlich ist durch Polymerisation einer oder mehrerer polymerisierbarer Verbindungen zwischen den Substraten der FK-Zelle im FK-Medium, **dadurch gekennzeichnet, dass** mindestens eine der polymerisierbaren Verbindungen eine Verbindung der Formel I
P^{a}-(Sp^{a})ₛ₁-A²-Q¹-A¹-Q²-A³-(Sp^{b})ₛ₂-P^{b} I
worin die einzelnen Reste die in Anspruch 1 angegebenen Bedeutungen besitzen, ist.

11. Verfahren zur Herstellung einer FK-Anzelge nach Anspruch 10, indem man ein FK-Medium nach einem oder mehreren der Ansprüche 2, 3, 6 und 6 in eine FK-Zelle mit zwei Substraten und zwei Elektroden, wie in Anspruch 10 beschrieben, füllt, und die polymerisierbaren Verbindungen polymerisiert.

12. Verbindungen der Formel I
P^{a}-(Sp^{a})ₛ₁-A²-Q¹-A¹-Q²-A³-(Sp^{b})ₛ₂-P^{b} I
worin die Parameter die in Anspruch 1 angegebenen Bedeutungen besitzen.

13. Verbindungen der Formel I nach Anspruch 12,
worin einer oder mehrere der Reste A¹, A² und A³ ausgewählt sind aus
der Gruppe b1) bestehend aus 1,3-Phenylen, worin auch eine oder zwei CH-Gruppen durch N ersetzt sein können und worin auch ein oder mehrere H-Atome durch L ersetzt sein können, oder
der Gruppe d) bestehend aus gesättigten, teilweise gesättigten oder vollständig ungesättigten, und optional substituierten, polycyclischen Resten mit 5 bis 20 cyclischen C-Atomen, von denen auch eines oder mehrere durch Heteroatome ersetzt sein können.

14. Verbindungen der Formel I nach Anspruch 12 oder 13,
worin einer oder mehrere der Reste A¹, A² und A³ ausgewählt sind aus der Gruppe bestehend aus 1,3-Phenylen, Bicyclo[1.1.1]pentan-1,3-diyl, Bicyclo[2.2.2]octan-1,4-diyl, Spiro[3.3]heptan-2,6-diyl, 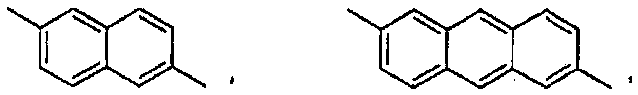 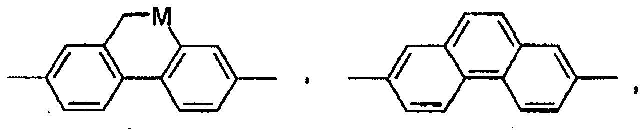 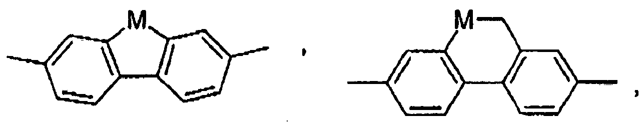 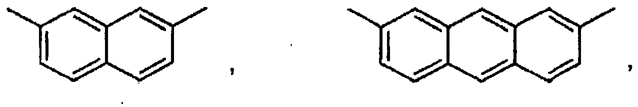 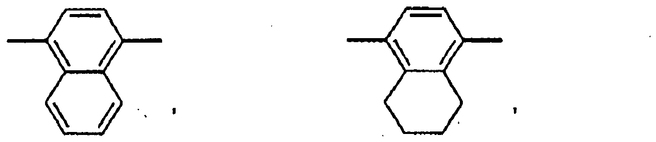 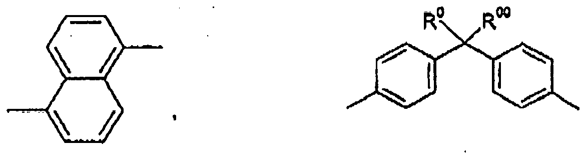 wobei in diesen Resten auch ein oder mehrere H-Atome durch L ersetzt sein können, und/oder eine oder mehrere Doppelbindungen durch Einfachbindungen ersetzt sein können, und/oder ein oder mehrere CH-Gruppen durch N ersetzt sein können, und L, R⁰, R⁰⁰,M, Y¹ und Y² die in Anspruch 1 angegebene Bedeutung besitzen,

15. Verbindungen der Formel IA
G-O-(Sp^{a})ₛ₁-A²-Q¹-A¹-Q²-A³-(Sp^{b})ₛ₂-O-G' IA
worin Q¹, Q², A¹, A², A³, Sp^{a}, Sp^{b}, s1 und s2 die in Anspruch 1 angegebene Bedeutungen besitzen, und G und G' jeweils unabhängig voneinander ein H-Atom oder eine Schutzgruppe bedeuten.

16. FK-Medium oder FK-Anzeige enthaltend eine oder mehrere Verbindungen der Formel I nach einem oder mehreren der Ansprüche 12 bis 15.

## Claims

1. Use of compounds of the formula I
P^{a}-(Sp^{a})ₛ₁-A²-Q¹-A¹-Q²-A³-(Sp^{b})ₛ₂-P^{b} I
in which the individual radicals have the following meanings:
P^{a}, P^{b} each, independently of one another, denote a polymerisable group,
Sp^{a}, Sp^{b} each, independently of one another, denote a spacer group,
s1, s2 each, independently of one another, denote 0 or 1,
Q¹, Q² each, independently of one another, denote -CF₂O- or -OCF₂-,
A¹, A², A³ each, independently of one another, denote a radical selected from the following groups:
a) the group consisting of trans-1,4-cyclohexylene, 1,4-cyclohexenylene and 1,4'-bicyclohexylene, in which, in addition, one or more non-adjacent CH₂ groups may be replaced by -O- and/or -S- and in which, in addition, one or more H atoms may be replaced by F,
b) the group consisting of 1,4-phenylene and 1,3-phenylene, in which, in addition, one or two CH groups may be replaced by N and in which, in addition, one or more H atoms may be replaced by L,
c) the group consisting of tetrahydropyran-2,5-diyl, 1,3-dioxane-2,5-diyl, tetrahydrofuran-2,5-diyl, cyclobutane-1,3-diyl, piperidine-1,4-diyl, thiophene-2,5-diyl and selenophene-2,5-diyl, each of which may, in addition, be mono- or polysubstituted by L,
d) the group consisting of saturated, partially unsaturated or fully unsaturated, and optionally substituted, polycyclic radicals having 5 to 20 cyclic C atoms, one or more of which may also be replaced by heteroatoms, preferably selected from the group consisting of bicyclo[1.1.1]pentane-1,3-diyl, bicyclo[2.2.2]-octane-1,4-diyl, spiro[3.3]heptane-2,6-diyl, 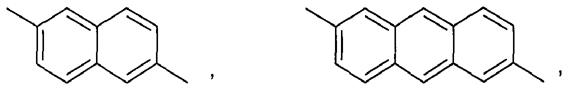 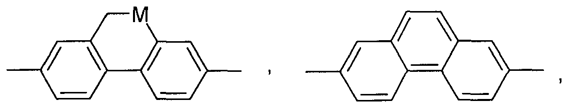 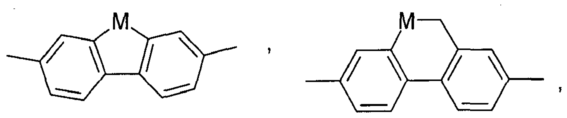 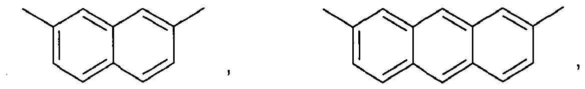 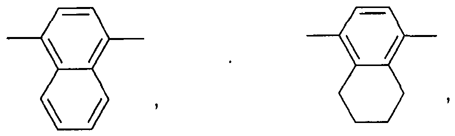 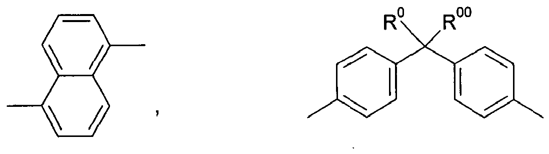 where, in addition, one or more H atoms in these radicals may be replaced by L, and/or one or more double bonds may be replaced by single bonds, and/or one or more CH groups may be replaced by N,
L on each occurrence, identically or differently, denotes F, Cl, CN, SCN, SF₅ or straight-chain or branched, in each case optionally fluorinated alkyl, alkoxy, alkylcarbonyl, alkoxycarbonyl, alkylcarbonyloxy or alkoxycarbonyloxy having 1 to 12 C atoms,
R⁰, R⁰⁰ each, independently of one another, denote H, F or straight-chain or branched alkyl having 1 to 12 C atoms, in which, in addition, one or more H atoms may be replaced by F,
M denotes -O-, -S-, -CH₂-, -CHY¹- or -CY¹Y²-,
Y¹ and Y² each, independently of one another, have one of the meanings indicated above for R⁰, or denote Cl or CN, and preferably denote H, F, Cl, CN, OCF₃ or CF₃,
or the use of a polymer obtainable by polymerisation of one or more compounds of the formula I, in LC displays having a blue phase.

2. Liquid-crystal medium comprising one or more compounds of the formula I
P^{a}-(Sp^{a})ₛ₁-A²-Q¹-A¹-Q²-A³-(Sp^{b})ₛ₂-P^{b} I
in which the individual radicals have the meanings indicated in Claim 1,
or comprising a polymer obtainable by polymerisation of one or more of these compounds of the formula I.

3. Liquid-crystal medium according to Claim 2, comprising one or more compounds of the formula I.

4. Liquid-crystal medium according to Claim 2, comprising a polymer obtainable by polymerisation of one or more compounds of the formula I.

5. Liquid-crystal medium according to Claim 3 or 4, **characterised in that** it comprises the following components:
- a polymerisable component A comprising one or more compounds of the formula I
P^{a}-(Sp^{a})ₛ₁-A²-Q¹-A¹-Q²-A³-(Sp^{b})ₛ₂-P^{b} I
in which the individual radicals have the meanings indicated in Claim 1, or a polymer obtainable therefrom, and
- a liquid-crystalline component B comprising one or more compounds of the formula II 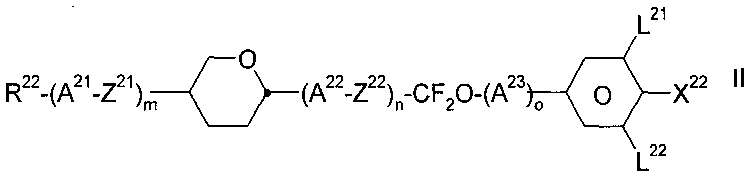 in which the individual radicals have the following meanings:
R²² denotes H, F, Cl, CN, NCS, SF₅, SO₂CF₃ or straight-chain or branched alkyl having 1 to 20 C atoms, which is unsubstituted or mono- or polysubstituted by F, Cl or CN, and in which, in addition, one or more non-adjacent CH₂ groups may also each be replaced, independently of one another, by -O-, -S-, -NH-, -NR⁰¹-, -SiR⁰¹R⁰²-, -CO-, -COO-, -OCO-, -OCO-O-, -S-CO-, -CO-S-, -CY⁰¹=CY⁰¹- or -C≡C- in such a way that O and/or S atoms are not linked directly to one another,
Y⁰¹, Y⁰² each, independently of one another, denote F, Cl or CN, one of the radicals Y⁰¹ and Y⁰² also denotes H,
R⁰¹, R⁰² each, independently of one another, denote H or alkyl having 1 to 12 C atoms,
A²¹, A²², A²³ each, independently of one another and on each occurrence identically or differently, denote 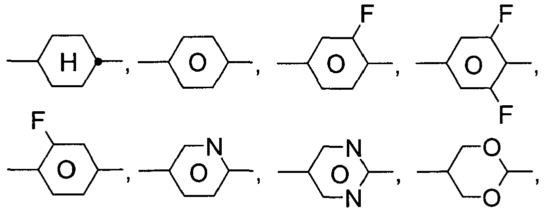
Z²¹, Z²² each, independently of one another and on each occurrence identically or differently, denote a single bond, -(CH₂)₄-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- or -O-CO-,
X²² denotes F, Cl, -CN, -NCS, -SF₅, -SO₂CF₃, or alkyl, alkenyl, alkenyloxy, alkylalkoxy or alkoxy having 1 to 3 C atoms, each of which which is mono- or polysubstituted by F, Cl or CN,
L²¹, L²² each, independently of one another, denote H or F,
m denotes 0, 1 or 2,
n denotes 0, 1, 2 or 3,
o denotes 0, 1 or 2, where
m + n + o denotes 0, 1, 2 or 3, preferably 0, 1 or 2,
- and a component D comprising one or more optically active and/or chiral compounds.

6. Liquid-crystal medium according to Claim 5, **characterised in that** it additionally comprises one or more compounds selected from the formulae Z, Q1 and Q2 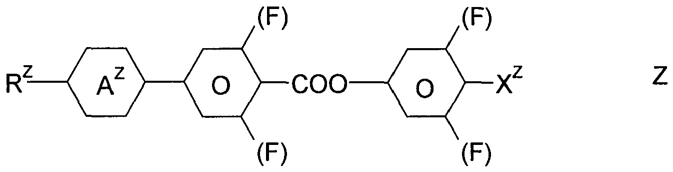 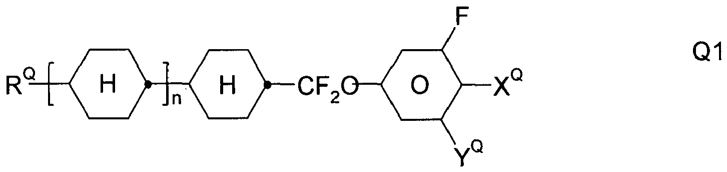 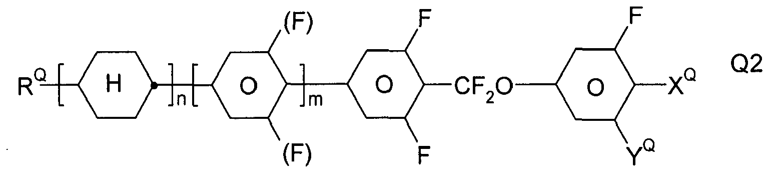 in which R^{Z} has one of the meanings indicated for R²² in formula II, 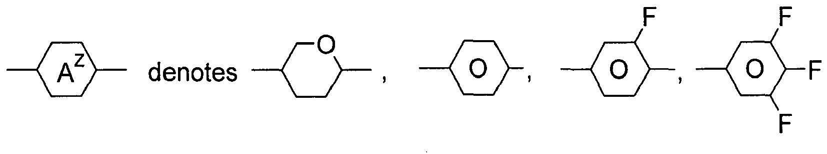
X^{Z} denotes F, Cl, CN, NCS, OCF₃, CF₃ or SF₅,
R^{Q} has one of the meanings indicated for R²² in formula II,
X^{Q} has one of the meanings indicated for X^{E} in formula E,
(F) denotes F or H, and
n and m each, independently of one another, denote 0 or 1.

7. Process for the preparation of an LC medium according to one or more of Claims 2 to 6, in which one or more liquid-crystalline compounds are mixed with one or more compounds of the formula I according to Claim 1.

8. LC display containing one or more compounds of the formula I
P^{a}-(Sp^{a})ₛ₁-A²-Q¹-A¹-Q²-A³-(Sp^{b})ₛ₂-P^{b} I
in which the individual radicals have the meanings indicated in Claim 1, or a polymer obtainable therefrom, or containing an LC medium according to one or more of Claims 2 to 6.

9. LC display according to Claim 8, **characterised in that** it is a display having a blue phase.

10. LC display according to Claim 8 or 9, comprising an LC cell having two substrates and two electrodes, where at least one substrate is transparent to light and at least one substrate has one or two electrodes, and a layer, located between the substrates, of an LC medium comprising a polymerised component and a low-molecular-weight component, where the polymerised component is obtainable by polymerisation of one or more polymerisable compounds between the substrates of the LC cell in the LC medium, **characterised in that** at least one of the polymerisable compounds is a compound of the formula I
P^{a}-(Sp^{a})ₛ₁-A²-Q¹-A¹-Q²-A³-(Sp^{b})ₛ₂-P^{b} I
in which the individual radicals have the meanings indicated in Claim 1.

11. Process for the production of an LC display according to Claim 10, in which an LC medium according to one or more of Claims 2, 3, 5 and 6 is introduced into an LC cell having two substrates and two electrodes, as described in Claim 10, and the polymerisable compounds are polymerised.

12. Compounds of the formula I
P^{a}-(Sp^{a})ₛ₁-A²-Q¹-A¹-Q²-A³-(Sp^{b})ₛ₂-P^{b} I
in which the parameters have the meanings indicated in Claim 1.

13. Compounds of the formula I according to Claim 12,
in which one or more of the radicals A¹, A² and A³ are selected from the group b1) consisting of 1,3-phenylene, in which, in addition, one or two CH groups may be replaced by N and in which, in addition, one or more H atoms may be replaced by L, or
the group d) consisting of saturated, partially unsaturated or fully unsaturated, and optionally substituted, polycyclic radicals having 5 to 20 cyclic C atoms, one or more of which may also be replaced by heteroatoms.

14. Compounds of the formula I according to Claim 12 or 13,
in which one or more of the radicals A¹, A² and A³ are selected from the group consisting of 1,3-phenylene, bicyclo[1.1.1]pentane-1,3-diyl, bicyclo[2.2.2]octane-1,4-diyl, spiro[3.3]heptane-2,6-diyl, 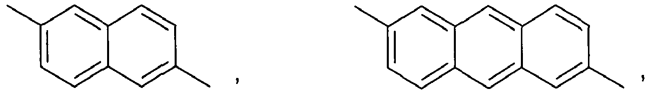 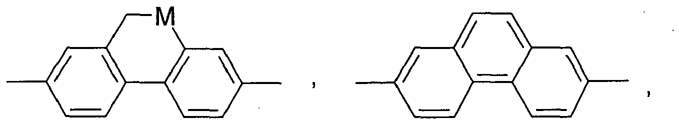 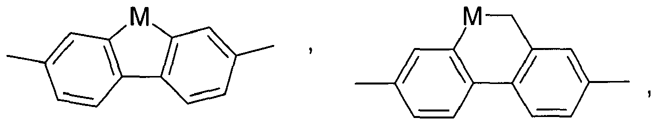 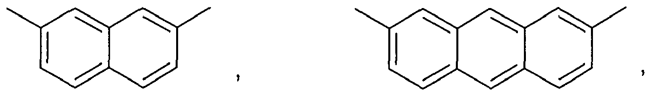 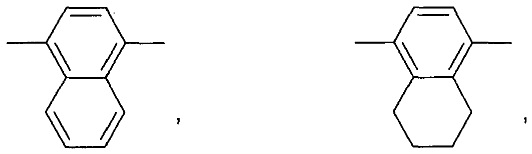 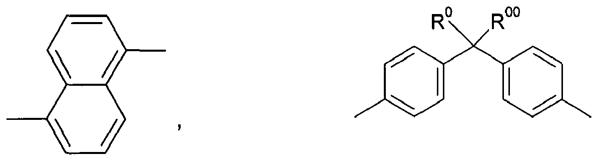 where one or more H atoms in these radicals may also be replaced by L, and/or one or more double bonds may be replaced by single bonds, and/or one or more CH groups may be replaced by N, and L, R⁰, R⁰⁰, M, Y¹ and Y² have the meaning indicated in Claim 1.

15. Compounds of the formula IA
G-O-(Sp^{a})ₛ₁-A²-Q¹-A¹-Q²-A³-(Sp^{b})ₛ₂-O-G' IA
in which Q¹, Q², A¹, A², A³, Sp^{a}, Sp^{b}, s1 and s2 have the meanings indicated in Claim 1, and G and G' each, independently of one another, denote an H atom or a protecting group.

16. LC medium or LC display comprising one or more compounds of the formula I according to one or more of Claims 12 to 15.

## Revendications

1. Utilisation de composés de la formule I
P^{a}-(Sp^{a})ₛ₁-A²-Q¹-A¹-Q²-A³-(Sp^{b})ₛ₂-P^{b} I
dans laquelle les radicaux individuels présentent les significations qui suivent :
P^{a}, P^{b} représentent, chacun indépendamment de l'autre, un groupe polymérisable,
Sp^{a}, Sp^{b} représentent, chacun indépendamment de l'autre, un groupe d'espaceur,
s1, s2 représentent, chacun indépendamment de l'autre, 0 ou 1,
Q¹, Q² représentent, chacun indépendamment de l'autre, -CF₂O-ou -OCF₂-,
A¹, A², A³ représentent, chacun indépendamment des autres, un radical choisi parmi les groupes qui suivent :
a) le groupe constitué par trans-1,4-cyclohexylène, 1,4-cyclohexénylène et 1,4'-bicyclohexylène, où, en outre, un ou plusieurs groupes CH₂ non adjacents peut/peuvent être remplacé(s) par -O- et/ou -S- et où, en outre, un ou plusieurs atomes de H peut/ peuvent être remplacé(s) par F,
b) le groupe constitué par 1,4-phénylène et 1,3-phénylène, où, en outre, un ou deux groupes CH peut/ peuvent être remplacé(s) par N et où, en outre, un ou plusieurs atomes de H peut/peuvent être remplacé(s) par L,
c) le groupe constitué par tétrahydropyran-2,5-diyle, 1,3-dioxane-2,5-diyle, tétrahydrofuran-2,5-diyle, cyclobut-1,3-diyle, pipéridine-1,4-diyle, thiophène-2,5-diyle et sélénophène-2,5-diyle, dont chacun peut, en outre, être mono- ou polysubstitué par L,
d) le groupe constitué par des radicaux polycycliques saturés, partiellement non saturés ou totalement non saturés et en option substitués, comportant de 5 à 20 atomes de C cycliques, dont un ou plusieurs peut/ peuvent également être remplacé(s) par des hétéroatomes, de préférence choisis parmi le groupe constitué par bicyclo[1.1.1]pentane-1,3-diyle, bicyclo-[2.2.2]octane-1,4-diyle, spiro[3.3]heptane-2,6-diyle, 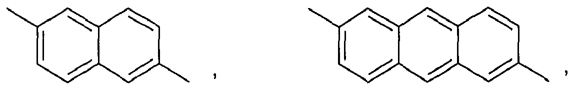 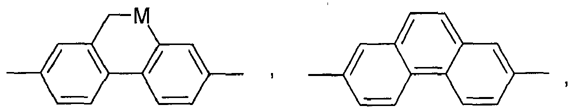 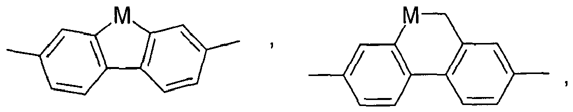 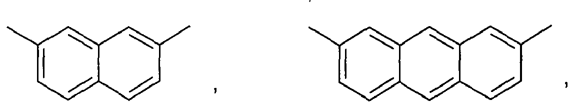 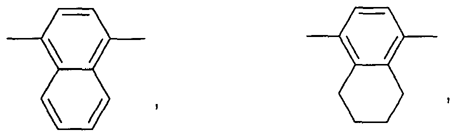 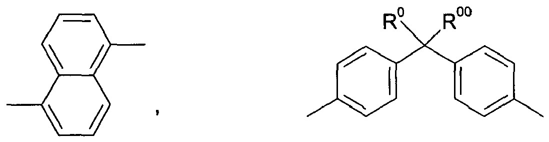 où, en outre, un ou plusieurs atomes de H dans ces radicaux peut/peuvent être remplacé(s) par L, et/ou une ou plusieurs liaisons doubles peut/peuvent être remplacée(s) par des liaisons simples, et/ou un ou plusieurs groupes CH peut/peuvent être remplacé(s) par N,
L représente, pour chaque occurrence, de manière identique ou différente, F, CI, CN, SCN, SF₅ ou alkyle, alcoxy, alkylcarbonyle, alcoxycarbonyle, alkylcarbonyloxy ou alcoxycarbonyloxy en chaîne droite ou ramifié, dans chaque cas en option fluoré, comportant de 1 à 12 atomes de C,
R⁰, R⁰⁰ représentent, chacun indépendamment de l'autre, H, F ou alkyle en chaîne droite ou ramifié comportant de 1 à 12 atomes de C, où, en outre, un ou plusieurs atomes de H peut/peuvent être remplacé(s) par F,
M représente -O-, -S-, -CH₂-, -CHY¹- ou -CY¹Y²-,
Y¹ et Y² présentent, chacun indépendamment de l'autre, l'une des significations indiquées ci avant pour R⁰, ou représentent Cl ou CN, et de préférence, représentent H, F, CI, CN, OCF₃ ou CF₃,
ou utilisation d'un polymère pouvant être obtenu par polymérisation d'un ou de plusieurs composés de la formule I, dans des affichages LC présentant une phase bleue ou dans des affichages LC du type PS ou PSA.

2. Milieu cristallin liquide comprenant un ou plusieurs composés de la formule I
P^{a}-(Sp^{a})ₛ₁-A²-Q¹-A¹-Q²-A³-(Sp^{b})ₛ₂-P^{b} I
dans laquelle les radicaux individuels présentent les significations indiquées selon la revendication 1,
ou comprenant un polymère pouvant être obtenu par polymérisation d'un ou de plusieurs de ces composés de la formule I.

3. Milieu cristallin liquide selon la revendication 2, comprenant un ou plusieurs composés de la formule I.

4. Milieu cristallin liquide selon la revendication 2, comprenant un polymère pouvant être obtenu par polymérisation d'un ou de plusieurs composés de la formule I.

5. Milieu cristallin liquide selon la revendication 3 ou 4, **caractérisé en ce qu'**il comprend les composants qui suivent :
- un composant polymérisable A comprenant un ou plusieurs composés de la formule I
P^{a}-(Sp^{a})ₛ₁-A²-Q¹-A¹-Q²-A³-(Sp^{b})ₛ₂-P^{b} I
dans laquelle les radicaux individuels présentent les significations indiquées selon la revendication 1, ou un polymère pouvant être obtenu à partir de ceux-ci, et
- un composant cristallin liquide B comprenant un ou plusieurs composés de la formule II 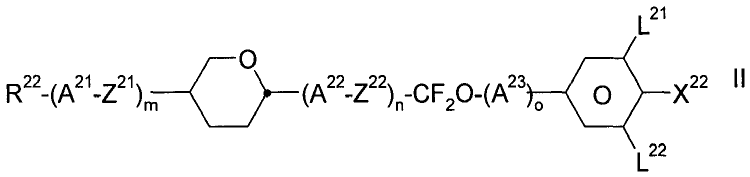 où les radicaux individuels présentent la signification qui suit :
R²² représente H, F, CI, CN, NCS, SF₅, SO₂CF₃ ou alkyle en chaîne droite ou ramifié comportant de 1 à 20 atomes de C, lequel est non substitué ou mono- ou polysubstitué par F, Cl ou CN, et où, en outre, un ou plusieurs groupes CH₂ non adjacents peuvent chacun être remplacés, indépendamment les uns des autres, par -O-, -S-, -NH-, -NR⁰¹-, -SiR⁰¹R⁰²-, -CO-, -COO-, -OCO-, -OCO-O-, -S-CO-, -CO-S-, -CY⁰¹=CY⁰¹- ou -C≡C- de telle sorte que des atomes de O et/ou de S ne soient pas liés directement les uns aux autres,
Y⁰¹, Y⁰² représentent, chacun indépendamment de l'autre, F, Cl ou CN, l'un des radicaux Y⁰¹ et Y⁰² représentant également H,
R⁰¹, R⁰² représentent, chacun indépendamment de l'autre, H ou alkyle comportant de 1 à 12 atomes de C,
A²¹, A²², A²³ représentent, chacun indépendamment des autres et pour chaque occurrence de manière identique ou différente, 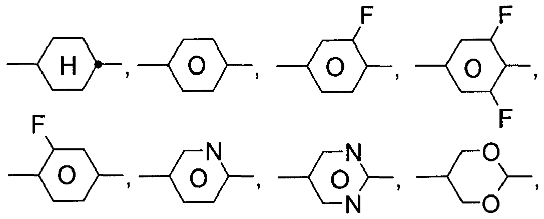
Z²¹, Z²² représentent, chacun indépendamment de l'autre et pour chaque occurrence de manière identique ou différente, une liaison simple, -(CH₂)₄-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- ou -O-CO-,
X²² représente F, CI, -CN, -NCS, -SF₅, -SO₂CF₃, ou alkyle, alkényle, alkényloxy, alkylalcoxy ou alcoxy comportant de 1 à 3 atomes de C, lequel est mono- ou polysubstitué par F, Cl ou CN,
L²¹, L²² représentent, chacun indépendamment de l'autre, H ou F,
m représente 0, 1 ou 2,
n représente 0, 1, 2 ou 3,
o représente 0, 1 ou 2, où
m + n + o représente 0, 1, 2 ou 3, de préférence 0, 1 ou 2,
- et un composant D comprenant un ou plusieurs composés optiquement actifs et/ou chiraux.

6. Milieu cristallin liquide selon la revendication 5, **caractérisé en ce qu'**il comprend additionnellement un ou plusieurs composés choisis parmi les formules Z, Q1 et Q2, 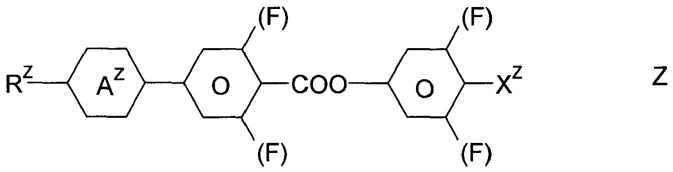 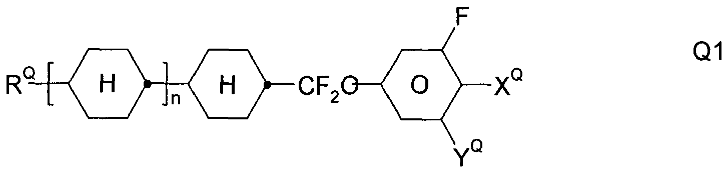 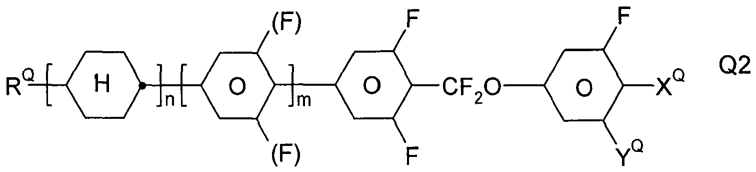 dans lesquelles R^{z} présente l'une des significations indiquées pour R²² dans la formule II, 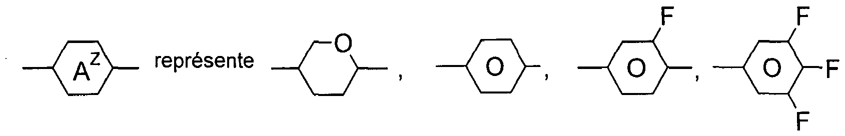
X^{z} représente F, Cl, CN, NCS, OCF₃, CF₃ ou SF₅,
R^{Q} présente l'une des significations indiquées pour R²² dans la formule II,
X^{Q} présente l'une des significations indiquées pour X^{E} dans la formule E,
(F) représente F ou H, et
n et m représentent, chacun indépendamment de l'autre, 0 ou 1.

7. Procédé pour la préparation d'un milieu LC selon une ou plusieurs des revendications 2 à 6, dans lequel un ou plusieurs composés cristallins liquides sont mélangés avec un ou plusieurs composés de la formule I selon la revendication 1.

8. Affichage LC contenant un ou plusieurs composés de la formule I
P^{a}-(Sp^{a})ₛ₁-A²-Q¹-A¹-Q²-A³-(Sp^{b})ₛ₂-P^{b} I
dans laquelle les radicaux individuels présentent les significations indiquées selon la revendication 1, ou un polymère pouvant être obtenu à partir de ceux-ci, ou contenant un milieu LC selon une ou plusieurs des revendications 2 à 6.

9. Affichage LC selon la revendication 8, **caractérisé en ce qu'**il s'agit d'un affichage présentant une phase bleue.

10. Affichage LC selon la revendication 8 ou 9, comprenant une cellule LC comportant deux substrats et deux électrodes, où au moins un substrat est transparent vis-à-vis de la lumière et au moins un substrat comporte une ou deux électrodes, et une couche, localisée entre les substrats, d'un milieu LC comprenant un composant polymérisé et un composant de poids moléculaire faible, où le composant polymérisé peut être obtenu par polymérisation d'un ou de plusieurs composés polymérisables entre les substrats de la cellule LC dans le milieu LC, **caractérisé en ce qu'**au moins l'un des composés polymérisables est un composé de la formule I
P^{a}-(Sp^{a})ₛ₁-A²-Q¹-A¹-Q²-A³-(Sp^{b})ₛ₂-P^{b} I
dans laquelle les radicaux individuels présentent les significations indiquées selon la revendication 1.

11. Procédé pour la production d'un affichage LC selon la revendication 10, dans lequel un milieu LC selon une ou plusieurs des revendications 2, 3, 5 et 6 est introduit à l'intérieur d'une cellule LC comportant deux substrats et deux électrodes, comme décrit selon la revendication 10, et les composés polymérisables sont polymérisés.

12. Composés de la formule I
P^{a}-(Sp^{a})ₛ₁-A²-Q¹-A¹-Q²-A³-(Sp^{b})ₛ₂-P^{b} I
dans laquelle les radicaux individuels présentent les significations indiquées selon la revendication 1.

13. Composés de la formule I selon la revendication 12,
dans laquelle un ou plusieurs des radicaux A¹, A² et A³ sont choisis parmi
le groupe b1) constitué par 1,3-phénylène, où, en outre, un ou deux groupes CH peuvent chacun être remplacés par N et où, en outre, un ou plusieurs atomes de H peut/peuvent être remplacé(s) par L, ou le groupe d) constitué par des radicaux polycycliques saturés, partiellement non saturés ou totalement non saturés, et en option substitués, comportant de 5 à 20 atomes de C cycliques, dont un ou plusieurs peut/peuvent également être remplacé(s) par des hétéroatomes.

14. Composés de la formule I selon la revendication 12 ou 13,
dans laquelle un ou plusieurs des radicaux A¹, A² et A³ est/sont choisi(s) parmi le groupe constitué par 1,3-phénylène, bicyclo-[1.1.1]pentane-1,3-diyle, bicyclo[2.2.2]octane-1,4-diyle, spiro[3.3]-heptane-2,6-diyle, 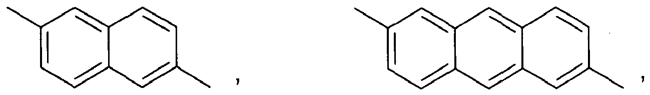 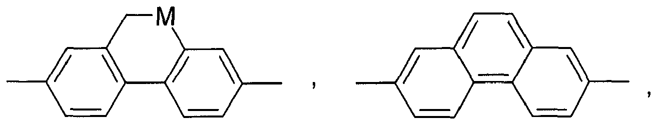 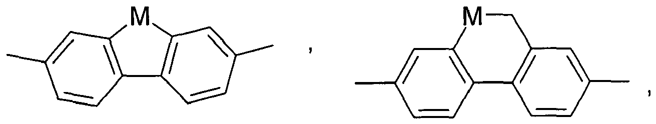 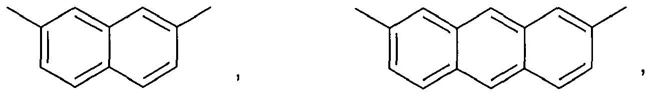 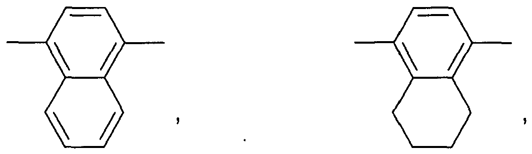 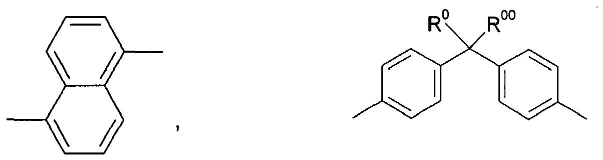 où un ou plusieurs atomes de H dans ces radicaux peut/peuvent également être remplacé(s) par L, et/ou une ou plusieurs liaisons doubles peut/peuvent être remplacée par des liaisons simples, et/ou un ou plusieurs groupes CH peut/peuvent être remplacé(s) par N, et L, R⁰, R⁰⁰, M, Y¹ et Y² présentent les significations indiquées selon la revendication 1.

15. Composés de la formule IA
G-O-(Sp^{a})ₛ₁-A²-Q¹-A¹-Q²-A³-(Sp^{b})ₛ₂-O-G' IA
dans laquelle Q¹, Q², A¹, A², A³, Sp^{a}, Sp^{b}, s1 et s2 présentent les significations indiquées selon la revendication 1, et G et G' représentent, chacun indépendamment de l'autre, un atome de H ou un groupe de protection.

16. Milieu LC ou affichage LC comprenant un ou plusieurs composés de la formule I selon les revendications 12 à 15.
